# EUROPEAN PATENT SPECIFICATION

(11) **EP 2 334 821 B1**
(45) Date of publication and mention of the grant of the patent: **25.02.2015**
(21) Application number: 09782714.1
(22) Date of filing: 07.09.2009
(51) Int. Cl.: C12Q 1/68

(54) **MEANS AND METHODS FOR DIAGNOSING PREDISPOSITION FOR TREATMENT EMERGENT SUICIDAL IDEATION (TESI)**
MITTEL UND VERFAHREN ZUR DIAGNOSE DER PRÄDISPOSITION ZUR BEHANDLUNG VON AUFTAUCHENDEN SUIZIDGEDANKEN
Supports et procédés pour le diagnostic d'une prédisposition pour le traitement d'idéation suicide émergeant (TESI)

(30) Priority: 18.09.2008 EP 08016477; 12.03.2009 EP 09003639
(43) Date of publication of application: 22.06.2011
(73) Proprietor: Max-Planck-Gesellschaft zur Förderung der Wissenschaften e.V., 80539 München (DE)
(72) Inventor: MENKE, Andreas, 80336 München (DE); BINDER, Elisabeth, B., 80336 München (DE); HOLSBOER, Florian, 80805 München (DE)
(74) Representative: Vossius & Partner
(86) International application number: PCT/EP2009/061575
(87) International publication number: WO 2010/031712

(56) References cited:
- WO-A-2008/052167
- US-A1- 2008 233 657
- PERLIS R H ETAL: "Association between treatment-emergent suicidal ideation with citalopram and polymorphisms near cyclic adenosine monophosphate response element binding protein in the STAR*D study" ARCHIVES OF GENERAL PSYCHIATRY, AMERICAN MEDICAL ASSOCIATION, vol. 64, no. 6, 1 June 2007 (2007-06-01), pages 689-697, XP008093988 ISSN: 0003-990X
- LAJE GONZALO ET AL: "Genetic markers of suicidal ideation emerging during citalopram treatment of major depression" AMERICAN JOURNAL OF PSYCHIATRY, vol. 164, no. 10, October 2007 (2007-10), pages 1530-1538, XP002512529 ISSN: 0002-953X
- DATABASE DBSNP Submitted SNP of reference SNP rs1630535 18 July 2005 (2005-07-18), XP002512531 retrieved from NCBI Database accession no. ss43689680
- DATABASE dBSNP 20 June 2000 (2000-06-20), "Submitted SNP of reference SNP rs 279553" XP002560219 retrieved from NCBI Database accession no. ss362775
- MENKE ANDREAS ET AL: "Genetic markers within glutamate receptors associated with antidepressant treatment-emergent suicidal ideation" AMERICAN JOURNAL OF PSYCHIATRY, vol. 165, no. 7, July 2008 (2008-07), pages 917-918, XP002512530 ISSN: 0002-953X

## Description

This invention relates to a method of diagnosing a predisposition for or the occurrence of treatment emergent suicidal ideation in an individual, the method comprising determining the presence or absence of the SNPs defined by SEQ ID NOs: 8, 18, 19, 47, 50, 52, 56, 64, 73, 86, 87, 92, 94, 95, 97 and 99, wherein the polymorphic position defining the respective SNP is position 201 of the respective SEQ ID NO.

Major depression is a common psychiatric disease carrying a substantial loss of productivity and life quality and is associated with a significant morbidity and mortality with a suicide rate of about 15%^{1,2}. While antidepressants are the most effective treatment for depressive patients, there has been controversy if antidepressants, in particular serotonin reuptake inhibitors (SSRI) are implicated in the emergence or worsening of suicidal ideation ³⁻⁵. Although treatment with antidepressants is associated with a significant reduction in suicides 6-9 and was proven to have a suicide preventive effect ¹⁰, there is some evidence that a subgroup of patients (about 5 to 15%) develop treatment emergent suicidal ideation (TESI) in the first weeks following treatment initiation and dose adjustments ^{6, 11, 12}. In 2003, the Regulatory Agency of the British Department of Health warned physicians to avoid treatment with SSRI for depression in children and adolescents after studies showed an increase of agitation, hostility and suicidal behavior in this group ^{13, 14}. This led to a public health advisory by the U.S. Food and Drug Administration (FDA) about the risk of suicidality in pediatric patients taking SSRIs for depression ¹⁵. In 2005 the agency issued a black box warning and medication guide for a series of antidepressants (not only SSRI_{S}) indicating that pediatric and adult patients may be at risk for this side effect. Recent studies showed a significant decrease in diagnosis and psychopharmacologic treatment of depressive episodes in children, adolescents but also adults following these warnings ^{16, 17}. This was paralleled by an increase of suicide rates in the USA and the Netherlands between 2003 and 2005 ¹⁸ for the first time in a decade. Identification of a subgroup of patients at risk for treatment emergent suicidal behavior could therefore be critical to stop depriving patients of beneficial treatment options and to provide the risk group with closer monitoring.

While large family and twin studies estimate the heritability of suicidal behavior to be in the range of 30 to 55 % ^{19.20}, no such formal evidence exists for TESI.

Markers for predicting the risk of developing TESI have previously been described; see for example US 2008/0102467. However, the markers described in this patent application deliver a significant number of false positives and false negatives when using them for predictive purposes and have not yet been replicated in an independent sample.

The technical problem underlying the present invention is the provision of alternative or improved means and methods for determining a predisposition for treatment emergent suicidal ideation in a patient.

Accordingly, this invention relates to a method of diagnosing a predisposition for or the occurrence of treatment emergent suicidal ideation in an individual, the method comprising determining the presence or absence of the SNPs defined by SEQ ID NOs: 8, 18, 19, 47, 50, 52, 56, 64, 73, 86, 87, 92, 94, 95, 97 and 99, wherein the polymorphic position defining the respective SNP is position 201 of the respective SEQ ID NO.

As discussed in more detail below, these 16 SNPs are the result from a double-filter procedure which has not been described in the art relating to the field of markers for treatment emergent suicidal ideation. More specifically, the 16 SNPs according to the main embodiment share the feature of being validated on a patient data set different from the training or discovery data set. To the inventors' best knowledge, such a set of validated SNPs for predicting or diagnosing TESI is not available in the art. The set of 16 SNPs is listed in Table 2 below.

The term "treatment emergent suicidal ideation (TESI)" is known in the art and designates the occurrence or enhanced occurrence of suicidal ideation in response to treatment, more specifically in response to treatment with anti-depressive drugs. To explain further, suicidal ideation may be a symptom of depressive disorders prior to beginning any therapy. A subgroup of patients (about 5 to 15%), however, develop and adverse response to treatment, the treatment being directed to an alleviation of the symptoms of depressive disorders, in that they develop or develop in enhanced form suicidal ideation *upon the administration* of anti-depressant drugs. While sufficient psychopharmacological treatment may eventually cure or at least alleviate this side effect, it is necessary to closely monitor those patients which have a predisposition to develop TESI. Moreover, the additional administration of anxiolytica and/or sedative medication is to be considered in such patients.

The term "predisposition" in relation to a disease has its established meaning in the art and is used accordingly here. To explain further, the term "predisposition" relates to the susceptibility to a disease that can be triggered under certain conditions. A person exhibiting a predisposition to a certain disease is not necessarily destined to develop the disease, but exhibits a risk to do so, wherein the risk exceeds the population average. As regards predisposition for TESI, the conditions which may serve as a trigger comprise, as explained above, the administration of anti-depressant drugs.

In addition to providing a method of diagnosing a predisposition for TESI, the main embodiment, in an alternative, provides a method of diagnosing the occurrence of TESI. While the former embodiment relates to patients which have not or have not yet developed TESI, the latter embodiment relates to patients which already suffer from TESI. The method of diagnosing the occurrence of TESI may be applied to the latter group of patients either as the only method of diagnosing TESI, or in conjunction with established methods of diagnosing TESI. When applying the method of diagnosing TESI according to the invention in conjunction with known methods of diagnosing TESI, the performance of the overall diagnostic method is improved. In particular, the number of falls positives and falls negatives is reduced.

The term "SNP" is well-known in the art and is shorthand for "single nucleotide polymorphism". A SNP is a DNA sequence variation which is confined to a single position. Usually, polymorphisms are distinguished from mutations based on their prevalence. Sometimes a threshold of 1% prevalence in a population of individuals is considered for separating polymorphisms (more frequent) from mutations (less frequent). When present in an exon, and dependent on the occurrence of alternative splicing, the SNP may also be present in the mature mRNA. In the latter case, and depending on the degeneracy of the genetic code, a SNP may also be visible at the protein level. SNPs may furthermore be divided into SNPs occurring within a known locus, in the proximity of a known locus, and SNPs that are 5' further away than 2kb from the most 5' feature of a gene and 3' further away than 500 bases from most 3' feature of a gene. Where applicable, loci where a SNP is located in and/or loci in the proximity of a SNP are indicated in the tables provided further below. Generally speaking, a SNP may or may not be associated with a certain phenotype, disease, or predisposition for a disease. The present inventors provide a plurality of SNPs, the association of which with TESI is disclosed herein for the first time.

SNPs are being annotated, collected and maintained in various databases including the SNP database of the National Centre for Biotechnology Information of the National Institutes of Health (NCBI), US. The SNPs according to the invention are referred to below by using "rs" identifiers as used in the SNP database of the NCBI, also known as "dbSNP". More specifically, the rs identfiers refer to the dbSNP build 125, NCBI build 36.1/HG18. The SNP database of the NCBI is described in, for example, Sherry et al. (2001)²¹.

The SNPs according to the present invention are defined by the sequences comprised in the sequence listing. Generally speaking, a polymorphism may occur in at least two allelic forms, at least one of which may be referred to as the risk allele in those cases where an allelic form is associated with a disease or a risk to develop a disease. The sequences enclosed herewith and defining the SNPs of SEQ ID NOs: 1 to 100 throughout present the risk allele. In other words, presence of the respective sequence in a sample obtained from a patient is indicative of a risk to develop treatment emergent suicidal ideation. The respective allele which is not associated with a risk to develop TESI is indicated in the feature part of the respective entry of the sequence listing as "variation". The relevant polymorphic position defining the SNP is the position indicated in the corresponding entries of Table 7. This position is located in the middle of the sequence of the respective entry of the sequence listing. This is position is position number 201; see also Table 7. In those cases where the sequence listing lists more than one variable position for a given entry, it is understood that the polymorphic position at position number 201 is intended for the purpose of the present invention.

While the respective entire sequence as presented in the sequence listing may be used to define the SNP according to the invention, it is also envisaged to use a subsequence of the sequence of the respective SEQ ID NO., said subsequence being, for example, 15, 17, 19, 21, 23 or 25 nucleotides in length and the variable position being located, for example, in the middle of the respective subsequence. Also subsequences consisting of an even number of nucleotides may be considered such as subsequences consisting of 16, 18, 20, 22 and 24 nucleotides, wherein the variable position may be, for example, one of the two central positions of the respective subsequence. The nucleobase characterizing the risk allele is furthermore presented in Tables 2 to 4. We note that the SNP as such is confined to the variable position itself. The length of the flanking sequence used for defining the SNP does not have any limiting effect, neither on the definition of the SNP nor on any specific method for determining presence or absence of the SNP, such methods being further detailed below. Preferably, the length of the flanking sequences is chosen such that a unique position in the genome is defined. This is why the SNP databases give preference to long flanking sequences. On the other hand it would equally be possible to define a SNP by (i) specifying the bases which may occur at the variable position and (ii) providing an "absolute" position on the given chromosome, for a given release of the sequence of the respective chromosome or genome.

The set of 100 SNPs defined by SEQ ID NOs: 1 to 100 has been obtained by determining SNPs associated with TESI in a first group of patients (also referred to as "discovery sample" herein). All 100 SNPs are characterized by statistically significant correlation with TESI and capability to predict TESI within this first group of patients. In a second step, a second group of patients has been considered. This second group represents an independent sample and is also referred to as "replication sample" herein. Each SNP of the set of 16 SNPs according to the main embodiment is characterized in that it correlates with TESI also in this second group in a statistically significant manner and is furthermore capable of predicting TESI in this second group of individuals.

To explain further, a discriminant analysis classifying patients using these 16 SNPs significantly associated with TESI in both a discovery and a replication sample revealed a 92% probability to classify TESI vs. non-TESI cases correctly in the discovery sample and a 85% probability in the replication sample. Specificity was 100% in discovery sample and 95% in replication sample, sensitivity was 72% in discovery sample and 50% in replication sample; see also Table 2 below. In Table 2, the performance of individual SNPs is also indicated.

The terms "discovery sample" and "replication sample" are commonly used in the art. The discovery sample is used for determining associations between polymorphisms and a phenotype such as TESI. The replication sample is an independent sample, i.e., a nonoverlapping group of patients. It is used for validating the associations determined using the discovery sample. Examples of a discovery sample and a replication sample are disclosed in the Example.

The selected SNPs consist of or comprise the SNP defined by SEQ ID NO: 8. The SNP defined by SEQ ID NO: 8 is the SNP with the lowest p-value for the discovery group among the group of 16 SNPs according to the main embodiment.

In a preferred embodiment, in addition to one, more or all SNPs selected from the SNPs defined by SEQ ID NOs: 8, 18, 19, 47, 50, 52, 56, 64, 73, 86, 87, 92, 94, 95, 97 and 99, one, more or all SNPs selected from the SNPs defined by SEQ ID NOs: 1 to 7, 9 to 17, 20 to 46, 48, 49, 51, 53 to 55, 57 to 63, 65 to 72, 74 to 85, 88 to 91, 93, 96, 98 and 100 are used. According to this embodiment, one, more or all validated SNPs may be used in combination with one, more or all SNPs as defined by the remaining 84 SNPs as comprised in the set of 100 SNPs as shown in Table 1.

In another preferred embodiment, instead of or in addition to a SNP as defined in any one of the embodiments above, a corresponding tagging SNP is used, wherein said tagging SNP is provided in Table 4. As discussed in more detail below, tagging SNPs are those SNPs which highly or fully correlate with a SNP as defined by any one of SEQ ID NOs: 1 to 100. The invention can be practiced by using one or more tagging SNPs instead of (or in addition to) one or more SNPs as defined herein above.

Described is also a method of diagnosing a predisposition for or the occurrence of treatment emergent suicidal ideation in an individual, the method comprising determining the presence or absence of one or more SNPs selected from the SNPs defined by SEQ ID NOs: 1 to 100 in a sample obtained from a patient, wherein presence of the respective SNP(s) is/are indicative of said predisposition for or said occurrence of treatment emergent suicidal ideation, wherein said one SNP is or said more SNPs comprise the SNP defined by SEQ ID NO: 8.

Table 1 below presents the 100 SNPs according to the invention in the same order as they are presented in the enclosed sequence listing. More specifically, the SNPs are ordered according to the p-value. The p-value in this case is the likelihood that the observed association with TESI occurs by chance. The first column of Table 1 provides the database identifier of the respective SNP in dbSNP build 125. The corresponding sequences as presented in the enclosed sequence listing may also be retrieved from database NCBI build 36.1/HG18. The fifth column of the table presents the nucleobase present at the variable position in the risk allele. In addition to the sequence listing, also Table 7 presents the sequences of the SNPs of SEQ ID NOs:1 to 100.

**Table 1**

| **SNP** | **CHR** | **MAP** | **Gene** | **Risk allele** | **p-value** |
|---|---|---|---|---|---|
| rs1630535 | 15 | 58297467 | | A | 1.3x-7 |
| rs4724701 | 7 | 5495408 | #FBXL18 | A | 2x10-6 |
| rs1265235 | 6 | 4445680 | | A | 1.06x-5 |
| rs2414660 | 15 | 58262633 | | T | 1.37x-5 |
| rs1258962 3 | 14 | 47934734 | | A | 0.00002 |
| rs583338 | 18 | 39126230 | G | G | 0.00003 |
| rs279542 | 3 | 9910772 | #IL17RE::2 | G | 0.00003 |
| rs279553 | 3 | 9909604 | #IL17RE::2 | G | 0.00003 |
| rs2774089 | 13 | 111885503 | | G | 0.00004 |
| rs1421780 | 5 | 174335276 | | A | 0.00005 |
| rs4907674 | 13 | 111900673 | | G | 0.00005 |
| rs7676106 | 4 | 190343908 | | C | 0.00006 |
| rs9949324 | 18 | 74181100 | A | A | 0.00007 |
| rs4518023 | 20 | 39454182 | A | A | 0.00008 |
| rs7704939 | 5 | 78109953 | #ARSB | A | 0.00008 |
| rs4544214 | 15 | 30719195 | #ARHGAP11A::2 | T | 0.00009 |
| rs6072343 | 20 | 39401601 | #LPIN3 | A | 0.00009 |
| rs965118 | 7 | 150809355 | #RHEB | A | 0.0001 |
| rs1109089 | 7 | 150800951 | #RHEB | G | 0.00011 |
| rs2980872 | 8 | 126486101 | | C | 0.00011 |
| rs2025949 | 13 | 92742277 | #GPC6 | G | 0.00012 |
| rs1214226 6 | 1 | 87070436 | | T | 0.00012 |
| rs9877859 | 3 | 11292337 8 | #PLCXD2 | C | 0.00012 |
| rs2070439 | 21 | 34933082 | 34933082 | T | 0.00012 |
| rs1261756 6 | 2 | 167435454 | | A | 0.00013 |
| rs1958421 | 14 | 83249995 | | G | 0.00013 |
| rs9589698 | 13 | 92722965 | #GPC6 | C | 0.00013 |
| rs7645289 | 3 | 18047811 | | G | 0.00014 |
| rs2664537 | 20 | 39247142 | #ZHX3#PLCG1 | A | 0.00015 |
| rs2228246 | 20 | 39225477 | #PLCG1 | G | 0.00015 |
| rs2088138 | 17 | 44359721 | #SNF8#UBE2Z | T | 0.00015 |
| rs2839178 | 21 | 46503201 | #MCM3AP | A | 0.00016 |
| rs6500497 | 16 | 87338087 | #FLJ40448 | T | 0.00016 |
| rs1125890 3 | 10 | 14236785 | #FRMD4A | T | 0.00019 |
| rs2244057 | 14 | 61162625 | | C | 0.00019 |
| rs1459841 | 5 | 95936220 | | G | 0.0002 |
| rs7901463 | 10 | 59842383 | | C | 0.0002 |
| rs584762 | 11 | 31827333 | | C | 0.0002 |
| rs761453 | 11 | 31834575 | | A | 0.0002 |
| rs6035712 | 20 | 20773775 | | T | 0.0002 |
| rs554710 | 9 | 135171668 | | T | 0.00021 |
| rs6868846 | 5 | 59164510 | | A | 0.00021 |
| rs1013120 | 17 | 13485943 | | C | 0.00022 |
| rs649867 | 18 | 75501034 | | G | 0.00023 |
| rs6072317 | 20 | 39314930 | #ZHX3 | T | 0.00023 |
| rs4795069 | 17 | 30633359 | | A | 0.00026 |
| rs6948196 | 7 | 150844144 | #RHEB | T | 0.00029 |
| rs1340358 4 | 2 | 179550306 | | T | 0.00029 |
| rs7685314 | 4 | 190363247 | | A | 0.0003 |
| rs2662090 | 3 | 9066945 | #SRGAP3 | C | 0.0003 |
| rs6500498 | 16 | 87338145 | #FLJ40448 | G | 0.0003 |
| rs2074997 | 7 | 15080521 8 | #RHEB | G | 0.0003 |
| rs1204798 | 6 | 116650539 | #NT5DC1 | A | 0.00031 |
| rs1214364 7 | 1 | 228874868 | #COG2 | T | 0.00032 |
| rs3801033 | 7 | 6700627 | #ZNF12 | T | 0.00032 |
| rs1099704 4 | 10 | 67727448 | #CTNNA3 | A | 0.00035 |
| rs7350731 | 14 | 25179946 | | A | 0.00038 |
| rs9323737 | 14 | 83252414 | | G | 0.00038 |
| rs4881394 | 10 | 5103710 | | C | 0.0004 |
| rs4799159 | 18 | 74381450 | | A | 0.0004 |
| rs2270101 | 7 | 22902528 | | C | 0.00041 |
| rs356999 | 2 | 60665087 | | T | 0.00041 |
| rs618670 | 18 | 74187714 | | A | 0.00042 |
| rs7788668 | 7 | 22947162 | #DRCTNNB1A | A | 0.00045 |
| rs9332172 | 10 | 96721777 | #CYP2C9 | G | 0.00046 |
| rs902923 | 9 | 93373419 | | G | 0.00046 |
| rs4810622 | 20 | 45310632 | #PRKCBP1 | T | 0.00047 |
| rs6873640 | 5 | 123937398 | | C | 0.00047 |
| rs9302822 | 16 | 6482925 | #A2BP1 | A | 0.00047 |
| rs3753151 | 7 | 150815917 | #RHEB | C | 0.00051 |
| rs1049083 2 | 3 | 74427045 | #CNTN3 | G | 0.00052 |
| rs1545384 | 3 | 74434950 | #CNTN3 | A | 0.00052 |
| rs1884641 | 20 | 5987908 | #C20orf75 | G | 0.00052 |
| rs1958438 | 14 | 83230211 | | T | 0.00053 |
| rs4917639 | 10 | 96715524 | #CYP2C9 | C | 0.00054 |
| rs2279103 | 18 | 75574114 | #CTDP1 | T | 0.00057 |
| rs7307064 | 12 | 21525660 | #RECQL::2 | C | 0.00059 |
| rs6793017 | 3 | 2554126 | #CNTN4 | A | 0.00066 |
| rs523386 | 18 | 75628182 | | C | 0.00072 |
| rs1984151 | 6 | 94585819 | T | T | 0.00076 |
| rs7719325 | 5 | 123500474 | | G | 0.00076 |
| rs1454333 | 4 | 92062152 | | A | 0.00078 |
| rs7146332 | 14 | 40847650 | | A | 0.00079 |
| rs1125001 7 | 8 | 10357428 | | C | 0.00079 |
| rs1717509 6 | 18 | 60871165 | | C | 0.00084 |
| rs2299965 | 7 | 15082359 4 | #RHEB | C | 0.00085 |
| rs2299967 | 7 | 15083614 3 | #RHEB | T | 0.00085 |
| rs720733 | 4 | 13821679 6 | | A | 0.00085 |
| rs635546 | 18 | 5927731 | | C | 0.00086 |
| rs7550277 | 1 | 239520957 | #RGS7 | C | 0.00086 |
| rs301191 | 3 | 177621532 | | A | 0.00089 |
| rs301193 | 3 | 177622218 | | C | 0.00089 |
| rs1258339 5 | 13 | 34117777 | | G | 0.00095 |
| rs1037448 | 11 | 60891107 | #CYBASC3 | T | 0.00096 |
| rs4939517 | 11 | 60883433 | #CYBASC3 | C | 0.00096 |
| rs4388301 | 6 | 138324067 | | C | 0.00096 |
| rs8095186 | 18 | 38167177 | | G | 0.00098 |
| rs1891877 | 10 | 23018034 | #PIP5K2A | C | 0.00098 |
| rs2348427 | 4 | 11163384 7 | #ENPEP | T | 0.00098 |
| rs9480684 | 6 | 10711891 7 | #RTN41P1 | A | 0.00099 |

***Table 1:* Results from association analysis**
Best empiric associations calculated with Fisher Product Method over both allelic and genotypic tests with p < 0.001 and being significantly associated in both non-TESI comparison groups. The non-TESI comparison groups were 1) all individuals without increase in suicidality independent of baseline suicidality (no increase of the HAM-D item 3 overtime; n = 329) and 2) a sub-group of 1), patients rating zero on item 3 throughout treatment (item 3 of the HAM-D = zero at all visits; n = 79; see also example 1, *Psychopathology and phenotype definition*). SNP: reference sequence identification number according to dbSNP; CHR: chromosome number; MAP: physical location of SNP on genome build HG18.

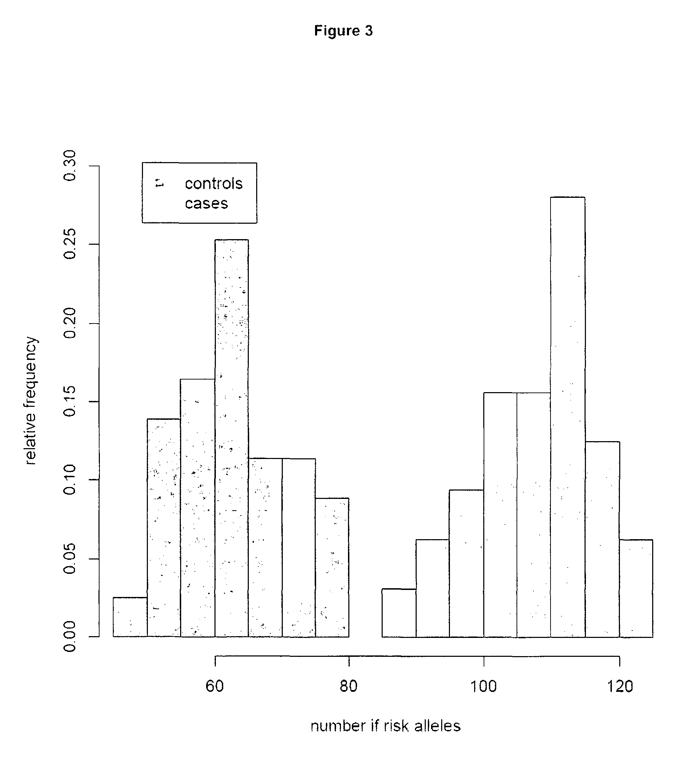

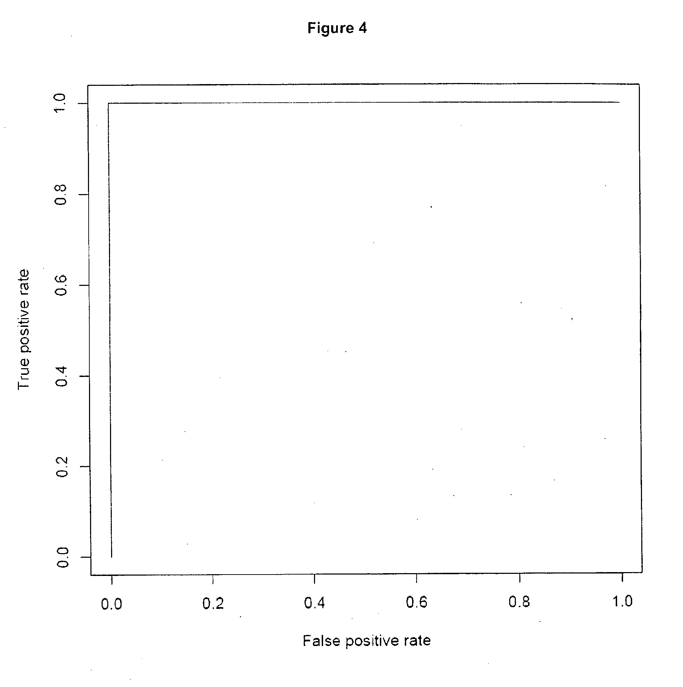

The SNPs according to the invention are characterized by a particularly high predictive value. As shown in the Example as well as in Figures 3 and 4 enclosed herewith, when using all 100 SNPs according to the invention, a perfect discrimination between risk patients and patients which do not exhibit a risk of developing TESI is achieved in the discovery sample.

The SNPs according to the invention are furthermore indicative of possible pathophysiological pathways relevant for TESI. Accordingly, they are indicative of candidate targets for therapeutic intervention. Genes encoding said candidate targets are indicated in Tables 1 to 4. It is of note that none of the genes harbouring the 16 SNPs have been associated with suicidality yet, however, regions around the genes RHEB, TMEM138 and CYBASC3 have been implicated with bipolar disorder in linkage studies. Of note is that 6 of 11 RHEB SNPs are associated with TESI, even if they are in high linkage disequilibrium. Most of the genes are involved in cell growth or organization (RHEB, ENPEP, TNNA3 and JAGN1); SRGAP3 is associated with neuronal signalling.

**Table 2**

| | | | | | | **Discovery** | **Sample** | | | **Replication** | **Sample** | | |
|---|---|---|---|---|---|---|---|---|---|---|---|---|---|
| **SNP** | **CHR** | **MAP** | **Gene (Closest gene and distance)** | **Location** | **Risk allele** | **P value FM Gr B** | **P value Group A** | **P value Group B** | **OR Gr B** | **P value FM Gr B** | **P value Group A** | **P value Group B** | **OR Gr B** |
| rs1037448 | 11 | 60891107 | TMEM138 | INTRONIC | T | 0.00096 | 1.48x-5 | 0.00093 | 3.3 (1.6-6.8) | 0.035 | 0.012 | 0.035 | 1.8 (1.0-3.4) |
| rs10997044 | 10 | 67727448 | CTNNA3 | INTRONIC | A | 0.00035 | 0.001 | 0.00101 | 3.1 (1.6-6.1) | 0.141 | 0.046 | 0.113 | 1.7 (1.0-3.3) |
| rs1109089* | 7 | 150800951 | RHEB | INTRONIC | G | 0.00011 | 1.67x-5 | 0.00011 | 3.5 (1.8-6.6) | 0.041 | 0.004 | 0.03 | 1.7 (1.0-2.9) |
| rs1884641 | 20 | 5987908 | (C20orf75 5233) | INTERGENIC | G | 0.00052 | 0.01045 | 0.00618 | 2.5 (1.3-4.8) | 0.018 | 0.009 | 0.014 | 1.8 (1.0-3.0) |
| rs2074997* | 7 | 150805218 | RHEB | INTRONIC | G | 0.0003 | 3.49x-5 | 0.00024 | 3.2 (1.7-6.0) | 0.041 | 0.003 | 0.032 | 1.6 (1.0-2.7) |
| rs2299965* | 7 | 150823594 | RHEB | INTRONIC | C | 0.00085 | 0.00013 | 0.00073 | 3.0 (1.6-5.5) | 0.024 | 0.005 | 0.018 | 2.0 (1.1-3.5) |
| rs2299967* | 7 | 150836143 | RHEB | INTRONIC | T | 0.00085 | 0.00013 | 0.00073 | 3.0 (1.6-5.5) | 0.019 | 0.003 | 0.018 | 2.2 (1.2-4.0) |
| rs2348427 | 4 | 111633847 | ENPEP | INTRONIC | T | 0.00098 | 0.00659 | 0.00177 | 2.5 (1.4-4.6) | 0.043 | 0.054 | 0.027 | 2.0 (1.2-3.3) |
| rs2662090 | 3 | 9066945 | SRGAP3 | INTRON IC | C | 0.0003 | 0.00051 | 0.00026 | 3.2 (1.7-6.1) | 0.041 | 0.105 | 0.037 | 1.8 (1.1-3.0) |
| rs279553 | 3 | 9909604 | JAGN1 | SYNONYMOUS_ CODING | G | 0.00003 | 0.00077 | 8x-5 | 4.1 (1.8-9.1) | 0.039 | 0.068 | 0.032 | 1.9 (1.0-3.4) |
| rs301193 | 3 | 177622218 | (N/A) | INTERGENIC | C | 0.00089 | 4.45x-5 | 0.00127 | 3.1 (1.6-6.1) | 0.005 | 0.004 | 0.004 | 2.3 (1.3-4.3) |
| rs4939517 | 11 | 60883433 | CYBASC3 | INTRONIC | C | 0.00096 | 1.96x-5 | 0.00093 | 3.3 (1.6-6.8) | 0.079 | 0.01 | 0.056 | 1.7 (1.0-3.3) |
| rs6948196* | 7 | 150844144 | RHEB | INTRONIC | T | 0.00029 | 3.13x-5 | 0.00023 | 3.2 (1.7-6.0) | 0.027 | 0.004 | 0.022 | 2.1 (1.1-3.7) |
| rs7788668 | 7 | 22947162 | (FAM126A -2622) | INTERGENIC | A | 0.00045 | 0.00294 | 0.00096 | 2.8 (1.5-5.1) | 0.052 | 0.04 | 0.042 | 1.7 (1.0-2.8) |
| rs8095186 | 18 | 38167177 | (PIK3C3 251736) | INTERGENIC | G | 0.00098 | 0.00057 | 0.00148 | 4.1 (1.8-9.2) | 0.04 | 0.031 | 0.034 | 2.0 (1.0-3.9) |
| rs965118* | 7 | 150809355 | RHEB | INTRONIC | A | 0.0001 | 7.1 x-5 | 8x-5 | 3.6 (1.9-6.7) | 0.055 | 0.005 | 0.044 | 1.7 (1.0-2.9) |

***Table 2:* Best empiric associations calculated with allelic tests and being significantly associated in both discovery and replication sample.**
TESI positive: discovery n=32; replication n=42. The non-TESI comparison groups were A) all individuals without increase in suicidality independent of baseline suicidality (no increase of the HAM-D item 3 over time; discovery n=329; replication n=434) and B) a sub-group of A), patients rating zero on item 3 throughout treatment (item 3 of the HAM-D = zero at all visits; discovery n=79; replication n=149; see also example 1, subsection entitled *"Psychopathology and phenotype definition"*). SNP: reference sequence identification number according to dbSNP; CHR: chromosome number; MAP: physical location of SNP on genome build HG18. Allelic p-values are shown. FM= Fisher Product Method over both allelic and genotypic tests. * SNPs with LD>0.8. OR=odds ratios based on comparison between cases and control group B.

Described is furthermore a method of diagnosing a predisposition for or the occurrence of treatment emergent suicidal ideation in an individual, the method comprising determining the presence or absence of one or more SNPs selected from the SNPs defined by SEQ ID NOs: 1 to 100 in a sample obtained from a patient, wherein presence of the respective SNP(s) is/are indicative of said predisposition for or said occurrence of treatment emergent suicidal ideation.

Exactly 2, 3, 4, 5, 10, 15, 16 or 18 SNPs may be used.

Said one SNP or said more SNPs may comprise the SNP defined by SEQ ID NO: 1. This is the SNP being mentioned first in Table 1. When selecting subsets of SNPs, preference is given to subsets comprising SNPs with low p-values. P-values are indicated in Table 1.

Said SNPs consist of or comprise the SNPs defined by (a) SEQ ID NOs: 1, 3, 66 and 78; or (b) SEQ ID NOs: 1, 3, 6, 9, 21, 23, 29, 32, 38, 53, 55, 59, 61, 62, 66, 76, 78 and 94.

The set of four SNPs according to (a) has been shown to correctly predict a risk of developing TESI in 90% of the patients analyzed.

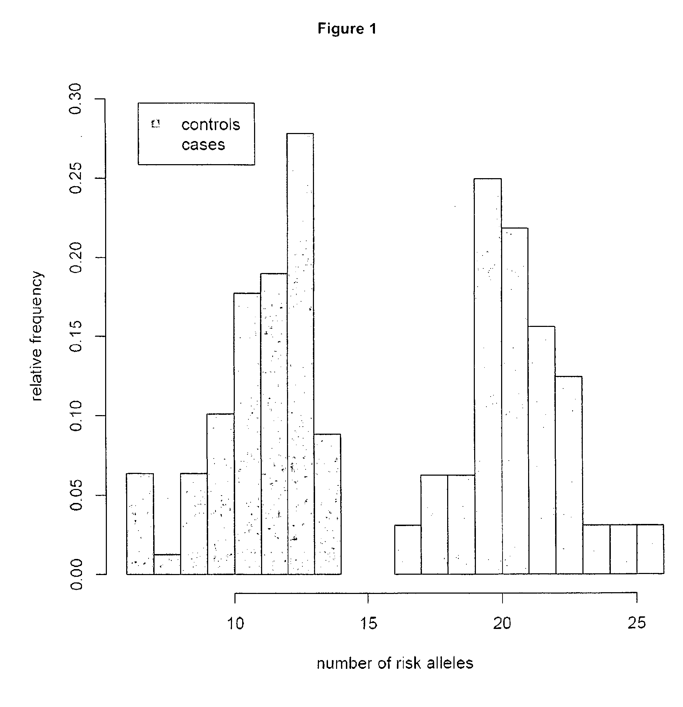

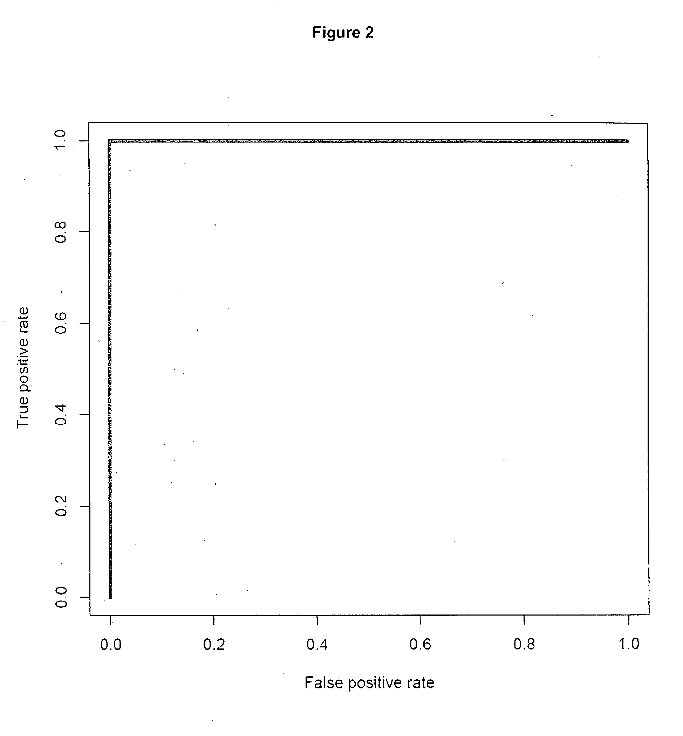

The set of 18 SNPs according to (b) provides correct predictions for 100% of the patients analyzed (see Figures 1 and 2 enclosed herewith). Table 3 provides a listing of the 18 SNPs characterized by SEQ ID NOs: 1, 3, 6, 9, 21, 23, 29, 32, 38, 53, 55, 59, 61, 62, 66, 76, 78 and 94. It is noteworthy that also this subset of 18 SNPs achieves the same discriminatory power as does the full set of 100 SNPs; see also Figures 1 and 2. When using the subset of 18 SNPs according to the invention or the respective subsequences thereof as defined herein above, individuals with 16 or more risk alleles are classified as TESI positive. When using 100 SNPs as defined by SEQ ID NOs. 1 to 100 or the respective subsequences thereof as defined herein above, individuals with 85 or more risk alleles are classified as TESI positive. Even single SNPs, in particular those with low P-values, allow excellent prediction.

**Table 3**

| **SNP** | **CHR** | **MAP** | **Gene** | **Closest gene** | **Distance to cl. gene** | **Risk allele** | **P value** |
|---|---|---|---|---|---|---|---|
| rs1630535 | 15 | 58297467 | | ANXA2P3 | -129157 | A | 1.3x-7 |
| rs1265235 | 6 | 4445680 | | Q5G014 | -110164 | A | 1.06x-5 |
| rs583338 | 18 | 39126230 | | SYT4 | 14801 | G | 0.00003 |
| rs2774089 | 13 | 111885503 | | C13orf28 | -193130 | G | 0.00004 |
| rs2025949 | 13 | 92742277 | GPC6 | | | G | 0.00012 |
| rs9877859 | 3 | 112923378 | PLCXD2 | | | C | 0.00012 |
| rs2664537 | 20 | 39247142 | ZHX3 | | | A | 0.00015 |
| rs2839178 | 21 | 46503201 | MCM3AP | | A | A | 0.00016 |
| rs584762 | 11 | 31827333 | | PAX6 | 31249 | C | 0.0002 |
| rs1204798 | 6 | 116650539 | NT5DC1 | | | A | 0.00031 |
| rs3801033 | 7 | 6700627 | ZNF12 | | | T | 0.00032 |
| rs4881394 | 10 | 5103710 | | AKR1C3 | -22872 | C | 0.0004 |
| rs2270101 | 7 | 22902528 | | FAM126A | -47256 | C | 0.00041 |
| rs356999 | 2 | 60665087 | | BCL11A | 30951 | T | 0.00041 |
| rs902923 | 9 | 93373419 | ROR2 | | | G | 0.00046 |
| rs2279103 | 18 | 75574114 | CTDP1 | | | T | 0.00057 |
| rs6793017 | 3 | 2554126 | CNTN4 | | | A | 0.00066 |
| rs1037448 | 11 | 60891107 | TMEM138 | | | T | 0.00096 |

***Table 3:* Results from association analysis, subset with 18 SNPs**
Best empiric associations calculated with Fisher Product Method over both allelic and genotypic tests with p < 0.001 and being significantly associated in both non-TESI comparison groups. SNP: reference sequence identification number according to dbSNP; CHR: chromosome number; MAP: physical location of SNP on genome build HG18.

Subsets of SNPs, i.e. in the present case subsets of less than 100 SNPs, exhibiting optimal predictive power on a given data set may be determined with tools known in the art when provided with the set of 100 SNPs according to the invention. Suitable tools are described in the examples enclosed herewith.

As regards the means and methods for determining presence or absence of a given SNP, numerous suitable methods are known in the art. Any of these methods, either alone or in combination, may be used for determining presence or absence of one or more SNPs according to the invention in a sample. In a preferred embodiment, said determining is effected by allele specific hybridization, allele specific oligonucleotide ligation, primer extension, minisequencing, mass spectroscopy, heteroduplex analysis, single strand conformational polymorphism, denaturing gradient gel electrophoresis, microarray analysis, temperature gradient gel electrophoresis or combinations thereof.

To explain further, in allele specific hybridization a probe is used which is preferably strictly complementary to a region of the target nucleic acid comprising the SNP in question. Hybridization conditions are chosen which allow to distinguish between full complementarity and a single mismatch.

Allele specific oligonucleotide ligation is another method for detecting alleles that differ by a single base. A pair of oligonucleotide probes that hybridize to adjacent segments of the target nucleic acid are used. The oligomer on the 5' side of the pair is an allele specific oligonucleotide in that it is strictly complementary to one specific allele of the target nucleic acid sequence, in the present case to the risk allele. The last base at the 3' end of this oligonucleotide corresponds to the SNP. The oligomer on the 3' side of the pair is the same for both different alleles. In case the risk allele is present, both oligonucleotides hybridize completely and are amenable to ligation with DNA ligase. In case the risk allele is not present, hybridization is not complete and no ligation is possible. Detecting the ligation product corresponds to detecting the risk allele. A variety of suitable detection schemes for the ligation product are known in the art.

In a primer extension assay according to the invention, nucleic acid comprised in the patient sample is hybridized to a primer complementary to the region adjacent to the SNP site. Dideoxyribonucleotides (ddNTPS) and DNA polymerase are added to the mixture and the primer is extended by a single nucleotide. The single nucleotide added is dependent on the allele of the amplified DNA. Primer extension biochemistry can be coupled with a variety of detection schemes, comprising fluorescence, fluorescence polarization (FP), luminescence and mass spectrometry (MS). Primer extension is sometimes also referred to as minisequencing.

Preferably, the hybridization of said primer to said nucleic acid is specific. Means of ensuring specificity of hybridization according to the present invention are known in the art and include stringent hybridization conditions. The term "stringent hybridization conditions", as used in the description of the present invention, is well known to the skilled artisan. Appropriate stringent hybridization conditions for each sequence may be established by a person skilled in the art on well-known parameters such as temperature, composition of the nucleic acid molecules, salt conditions etc.; see, for example, Sambrook et al., "Molecular Cloning, A Laboratory Manual"; CSH Press, Cold Spring Harbor, 1989 or Higgins and Hames (eds.), "Nucleic acid hybridization, a practical approach", IRL Press, Oxford 1985, see in particular the chapter "Hybridization Strategy" by Britten & Davidson, 3 to 15. Stringent hybridization conditions are, for example, conditions comprising overnight incubation at 42° C in a solution comprising: 50% formamide, 5x SSC (750 mM NaCl, 75 mM trisodium citrate), 50 mM sodium phosphate (pH 7.6), 5x Denhardt's solution, 10% dextran sulfate, and 20 micrograms/ml denatured, sheared salmon sperm DNA, followed by washing the filters in 0.1 x SSC at about 65°. Other stringent hybridization conditions are for example 0.2 x SSC (0.03 M NaCl, 0.003M Natriumcitrat, pH 7) at 65°C.

Microarrays inter alia provide a miniaturized implementation of a plurality of allele specific hybridization assays in arrayed format. Microarrays for SNP detection are known in the art and available from a variety of manufacturers including Affymetrix. These microarrays include oligonucleotide microarrays.

In a further preferred embodiment, said treatment emergent suicidal ideation occurs or may occur in a patient being administered selective serotonin re-uptake inhibitors, selective noradrenalin re-uptake inhibitors, dual serotonin and noradrenalin re-uptake inhibitors and/or tricyclic antidepressants, and optionally being administered neuroleptics, mood stabilizers and/or benzodiazepines. As explained in the background section herein above, antidepressant drugs in general, and in particular selective serotonin reuptake inhibitors may be causative of TESI.

In a further preferred embodiment, said individual is a child or adolescent. As stated in the background section herein above, children and adolescents are considered to be particularly effected by drug induced TESI.

In a further preferred embodiment, said determining comprises isolating a nucleic acid from said sample.

Means and methods for isolating nucleic acids from a sample taken from an individual are well-known in the art²². Preferred samples according to the invention are samples comprising or consisting of body fluids. Generally speaking, body fluids are liquid components of living organisms. Preferred body fluids include blood, serum, saliva, semen, vaginal secretions as well amniotic, cerebrospinal, synovial, pleural, peritoneal and pericardial fluids. The term "nucleic acid" as used herein includes DNA such as cDNA or genomic DNA, and RNA. It is understood that the term "RNA" as used herein comprises all forms of RNA including mRNA, non-coding RNA, tRNA and rRNA. The term "non-coding RNA" in turn includes siRNA (small interfering RNA), miRNA (micro RNA), rasiRNA (repeat associated RNA), snoRNA (small nucleolar RNA) and snRNA (small nuclear RNA). A preferred RNA is mRNA.

Described is also a kit comprising (a) one or more agents suitable for determining presence or absence of two or more SNPs, said SNPs being defined by SEQ ID NOs: 1 to 100; and (b) optionally a manual with instructions for performing the method of the invention. Agents suitable for determining presence or absence of SNPs are well-known in the art^{23, 24}.

In the kit, said agents may be selected from primers and probes for determining presence or absence of one or more SNPs of SEQ ID NOs: 1 to 100. Probes and primers may be used in methods such as allele specific hybridization, allele specific oligonucleotide ligation and primer extension as mentioned herein above.

Said more SNPs may be at least 2, 3, 4, 5, 10, 15, 16, 18, 20, 30, 40, 50, 60, 70, 80, 90 or 100 SNPs.

Specific sets of SNPs are those defined herein above. For example, a set of SNPs is the set of SNPs defined by SEQ ID NOs: 8, 18, 19, 47, 50, 52, 56, 64, 73, 86, 87, 92, 94, 95, 97 and 99.

In preferred embodiments of the method of the invention, respectively, instead of or in addition to a SNP as defined in these claims, a corresponding tagging SNP is used, wherein said tagging SNP is provided in Table 4. Sequences of the tagging SNPs are presented in the sequence listing (SEQ ID NOs: 101 to 813).

**Table 4**

| SNP | type | Closest gene | distance to gene | Tagged_SNP | Tagged_SNP type | Tagged _SNP_r 2 |
|---|---|---|---|---|---|---|
| rs1630535 | INTERGENIC | ANXA2P3 | -129157 | | | |
| | | | | rs1872133 | INTERGENIC | 1 |
| | | | | rs1872132 | INTERGENIC | 1 |
| | | | | rs2054680 | INTERGENIC | 1 |
| | | | | rs336536 | INTERGENIC | 1 |
| | | | | rs163135 | INTERGENIC | 1 |
| | | | | rs12148406 | INTERGENIC | 1 |
| | | | | rs336528 | INTERGENIC | 1 |
| | | | | rs17270048 | INTERGENIC | 1 |
| | | | | rs17270055 | INTERGENIC | 1 |
| | | | | rs338446 | INTERGENIC | 1 |
| | | | | rs7182941 | INTERGENIC | 1 |
| | | | | rs7183074 | INTERGENIC | 1 |
| | | | | rs10851673 | INTERGENIC | 1 |
| | | | | rs12912083 | INTERGENIC | 1 |
| | | | | rs1680195 | INTERGENIC | 1 |
| | | | | rs16942291 | INTERGENIC | 1 |
| | | | | rs12148854 | INTERGENIC | 1 |
| | | | | rs8028748 | INTERGENIC | 1 |
| | | | | rs1680222 | INTERGENIC | 1 |
| | | | | rs1680221 | INTERGENIC | 1 |
| | | | | rs12899914 | INTERGENIC | 0,87 |
| rs4724701 | SYNONYMOUS_CODING | FBXL18 | 0 | | | |
| | | | | rs7808325 | INTRONIC | 0,87 |
| | | | | rs3801058 | INTRONIC | 0,87 |
| | | | | rs7806101 | INTRONIC | 0,9 |
| | | | | rs10216189 | INTRONIC | 1 |
| | | | | rs4724699 | INTRONIC | 0,96 |
| | | | | rs7799970 | INTRONIC | 0,96 |
| | | | | rs4077245 | INTRONIC | 0,96 |
| | | | | rs4560713 | INTRONIC | 1 |
| rs1265235 | INTERGENIC | Q5G014_HUMAN | -110164 | | | |
| | | | | rs1265237 | INTERGENIC | 1 |
| | | | | rs1265234 | INTERGENIC | 1 |
| | | | | rs7774098 | INTERGENIC | 0,84 |
| rs2414660 | INTERGENIC | ANXA2P3 | -163991 | | | |
| | | | | rs338424 | INTERGENIC | 1 |
| | | | | rs2136559 | INTERGENIC | 0,82 |
| | | | | rs7165903 | INTERGENIC | 0,92 |
| | | | | rs7173200 | INTERGENIC | 0,91 |
| | | | | rs7173250 | INTERGENIC | 0,85 |
| | | | | rs900634 | INTERGENIC | 0,9 |
| | | | | rs7166014 | INTERGENIC | 0,88 |
| | | | | rs4775243 | INTERGENIC | 0,92 |
| | | | | rs2136557 | INTERGENIC | 0,84 |
| rs1258962 3 | INTERGENIC | N/A | -9 | | | |
| | | | | rs1621025 | INTERGENIC | 0,85 |
| | | | | rs1769522 | INTERGENIC | 0,85 |
| | | | | ras11157633 | INTERGENIC | 1 |
| | | | | rs7158518 | INTERGENIC | 0,95 |
| rs279553 | SYNONYMOUS_CODING | JAGN1 | 0 | | | |
| | | | | rs279558 | 5PRIME_UTR | 0,89 |
| | | | | rs279542 | 3PRIME_UTR | 1 |
| | | | | rs33970214 | INTRONIC | 1 |
| rs279542 | 3PRIME_UTR | JAGN1 | 0 | | | |
| | | | | rs279558 | 5PRIME_UTR | 0,89 |
| | | | | rs279553 | SYNONYMO_US CODING | 1 |
| | | | | rs33970214 | INTRONIC | 1 |
| rs583338 | INTERGENIC | SYT4 | 14801 | | | |
| | | | | rs816750 | INTERGENIC | 0,9 |
| | | | | rs310095 | INTERGENIC | 1 |
| | | | | rs310091 | INTERGENIC | 1 |
| | | | | rs310086 | INTERGENIC | 1 |
| | | | | rs310087 | INTERGENIC | 1 |
| | | | | rs310089 | INTERGENIC | 1 |
| | | | | rs618486 | INTERGENIC | 1 |
| rs2774089 | INTERGENIC | SOX1 | 111483 | | | |
| | | | | rs2576497 | INTERGENIC | 0,97 |
| | | | | rs1946770 | INTERGENIC | 0,97 |
| | | | | rs100224 | INTERGENIC | 0,97 |
| | | | | rs188122 | INTERGENIC | 0,97 |
| rs1421780 | INTRONIC | Q6ZNE8_HUMAN | 0 | | | |
| rs4907674 | INTERGENIC | SOX1 | 126653 | | | |
| rs7676106 | INTERGENIC | LOC728856 | -48091 | | | |
| | | | | rs9312392 | INTERGENIC | 1 |
| | | | | rs9312393 | INTERGENIC | 1 |
| | | | | rs7689338 | INTERGENIC | 1 |
| | | | | rs4241859 | INTERGENIC | 1 |
| | | | | rs4241860 | INTERGENIC | 1 |
| | | | | rs4241862 | INTERGENIC | 1 |
| | | | | rs4273547 | INTERGENIC | 1 |
| | | | | rs4282263 | INTERGENIC | 1 |
| | | | | rs6553287 | INTERGENIC | 1 |
| | | | | rs6820247 | INTERGENIC | 0,83 |
| | | | | rs10866319 | INTERGENIC | 0,83 |
| rs9949324 | INTERGENIC | N/A | -9 | | | |
| | | | | rs9949519 | INTERGENIC | 1 |
| | | | | rs611509 | INTERGENIC | 1 |
| | | | | rs592612 | INTERGENIC | 0,92 |
| | | | | rs603942 | INTERGENIC | 1 |
| | | | | rs677233 | INTERGENIC | 1 |
| rs7704939 | 3PRIME_UTR | ARSB | 0 | | | |
| rs4518023 | INTERGENIC | CHD6 | -10401 | | | |
| | | | | rs6065347 | INTERGENIC | 1 |
| | | | | rs4810317 | INTERGENIC | 1 |
| rs4544214 | UPSTREAM | ARHGAP11A | 46 | | | |
| | | | | rs11632524 | INTRONIC | 0,81 |
| | | | | rs8037818 | INTRONIC | 1 |
| | | | | rs4643284 | UPSTREAM | 1 |
| rs6072343 | UPSTREAM | LPIN3 | -1372 | | | |
| | | | | rs6065333 | INTERGENIC | 0,92 |
| | | | | rs16985655 | INTERGENIC | 0,92 |
| | | | | rs16985677 | INTRONIC | 0,81 |
| rs965118 | INTRONIC | RHEB | 0 | | | |
| | | | | rs17713386 | DOWNSTREAM | 0,97 |
| | | | | rs4726029 | INTRONIC | 0,97 |
| | | | | rs875588 | INTRONIC | 0,97 |
| | | | | rs1109089 | INTRONIC | 0,96 |
| | | | | rs17713697 | INTRONIC | 0,87 |
| | | | | rs2074998 | INTRONIC | 0,97 |
| | | | | rs2074997 | INTRONIC | 0,97 |
| | | | | rs17713890 | INTRONIC | 0,97 |
| | | | | rs2299965 | INTRONIC | 0,97 |
| | | | | rs2284264 | INTRONIC | 0,97 |
| | | | | rs11772458 | INTRONIC | 1 |
| | | | | rs2299967 | INTRONIC | 0,97 |
| | | | | rs3789817 | INTRONIC | 0,97 |
| | | | | rs758666 | INTRONIC | 0,97 |
| | | | | rs736645 | INTRONIC | 0,97 |
| | | | | rs4298422 | INTRONIC | 0,96 |
| | | | | rs2374261 | INTRONIC | 0,97 |
| | | | | rs12112989 | INTRONIC | 0,97 |
| | | | | rs6980020 | INTRONIC | 0,97 |
| | | | | rs12112134 | INTRONIC | 0,97 |
| | | | | rs6948196 | INTRONIC | 0,97 |
| | | | | rs6943752 | INTRONIC | 0,87 |
| | | | | rs7794922 | UPSTREAM | 0,81 |
| rs1109089 | INTRONIC | RHEB | 0 | | | |
| | | | | rs17713386 | DOWNSTREAM | 1 |
| | | | | rs4726029 | INTRONIC | 1 |
| | | | | rs875588 | INTRONIC | 1 |
| | | | | rs7805967 | INTRONIC | 0,83 |
| | | | | rs17713697 | INTRONIC | 0,9 |
| | | | | rs2074998 | INTRONIC | 1 |
| | | | | rs2074997 | INTRONIC | 1 |
| | | | | rs17713890 | INTRONIC | 1 |
| | | | | rs2299961 | INTRONIC | 0,83 |
| | | | | rs965118 | INTRONIC | 0,96 |
| | | | | rs3753151 | INTRONIC | 0,83 |
| | | | | rs2299965 | INTRONIC | 1 |
| | | | | rs2284264 | INTRONIC | 1 |
| | | | | rs11772458 | INTRONIC | 1 |
| | | | | rs2299967 | INTRONIC | 1 |
| | | | | rs3789817 | INTRONIC | 1 |
| | | | | rs758666 | INTRONIC | 1 |
| | | | | rs736645 | INTRONIC | 1 |
| | | | | rs4298422 | INTRONIC | 1 |
| | | | | rs2374261 | INTRONIC | 1 |
| | | | | rs12112989 | INTRONIC | 1 |
| | | | | rs6980020 | INTRONIC | 1 |
| | | | | rs12112134 | INTRONIC | 1 |
| | | | | rs6948196 | INTRONIC | 1 |
| | | | | rs6943752 | INTRONIC | 0,89 |
| | | | | rs7794922 | UPSTREAM | 0,86 |
| rs2980872 | INTERGENIC | TRIB1 | -25643 | | | |
| | | | | rs2954006 | INTERGENIC | 0,93 |
| rs 12142266 | INTERGENIC | SEP15_HUMAN | -30281 | | | |
| rs9877859 | INTRONIC | PHLDB2 | 0 | | | |
| | | | | rs4682298 | INTRONIC | 0,84 |
| rs2025949 | INTRONIC | GPC6 | 0 | | | |
| | | | | rs7338831 | INTRONIC | 0,91 |
| | | | | rs9561295 | INTRONIC | 0,94 |
| | | | | rs9589698 | INTRONIC | 0,9 |
| | | | | rs9523990 | INTRONIC | 0,9 |
| | | | | rs9523991 | INTRONIC | 0,94 |
| | | | | rs4142598 | INTRONIC | 0,95 |
| | | | | rs9523998 | INTRONIC | 1 |
| | | | | rs9561304 | INTRONIC | 1 |
| | | | | rs956036 | INTRONIC | 0,95 |
| | | | | rs1475449 | INTRONIC | 0,95 |
| rs2070439 | INTERGENIC | RCAN1 | 23780 | | | |
| rs12617566 | INTERGENIC | XIRP2 | -32784 | | | |
| rs9589698 | INTRONIC | GPC6 | 0 | | | |
| | | | | rs7338831 | INTRONIC | 1 |
| | | | | rs9561295 | INTRONIC | 1 |
| | | | | rs9523990 | INTRONIC | 1 |
| | | | | rs9523991 | INTRONIC | 1 |
| | | | | rs4142598 | INTRONIC | 1 |
| | | | | rs9523998 | INTRONIC | 0,91 |
| | | | | rs2025949 | INTRONIC | 0,9 |
| | | | | rs9561304 | INTRONIC | 0,91 |
| | | | | rs956036 | INTRONIC | 0,95 |
| | | | | rs1475449 | INTRONIC | 0,89 |
| rs1958421 | INTERGENIC | N/A | -9 | | | |
| | | | | rs1958438 | INTERGENIC | 0,92 |
| | | | | rs10139321 | INTERGENIC | 0,92 |
| | | | | rs10132490 | INTERGENIC | 0,89 |
| | | | | rs10143458 | INTERGENIC | 0,91 |
| | | | | rs9671273 | INTERGENIC | 0,92 |
| | | | | rs1952534 | INTERGENIC | 0,92 |
| | | | | rs1958436 | INTERGENIC | 0,91 |
| | | | | rs11159605 | INTERGENIC | 0,95 |
| | | | | rs7359138 | INTERGENIC | 0,8 |
| | | | | rs1958435 | INTERGENIC | 0,92 |
| | | | | rs1958434 | INTERGENIC | 0,91 |
| | | | | rs1958433 | INTERGENIC | 0,92 |
| | | | | rs2149684 | INTERGENIC | 0,87 |
| | | | | rs2183203 | INTERGENIC | 0,91 |
| | | | | rs1958432 | INTERGENIC | 0,91 |
| | | | | rs2149683 | INTERGENIC | 0,92 |
| | | | | rs8010141 | INTERGENIC | 0,92 |
| | | | | rs1958430 | INTERGENIC | 0,92 |
| | | | | rs1958429 | INTERGENIC | 0,92 |
| | | | | rs1958428 | INTERGENIC | 0,92 |
| | | | | rs1958426 | INTERGENIC | 1 |
| | | | | rs1958425 | INTERGENIC | 0,92 |
| | | | | rs1958424 | INTERGENIC | 1 |
| | | | | rs1958423 | INTERGENIC | 1 |
| | | | | rs1958422 | INTERGENIC | 1 |
| | | | | rs10132081 | INTERGENIC | 1 |
| | | | | rs10132130 | INTERGENIC | 1 |
| | | | | rs10147226 | INTERGENIC | 1 |
| | | | | rs1958420 | INTERGENIC | 1 |
| | | | | rs10873350 | INTERGENIC | 1 |
| | | | | rs10140080 | INTERGENIC | 1 |
| | | | | rs9323737 | INTERGENIC | 1 |
| | | | | rs4545730 | INTERGENIC | 1 |
| | | | | rs1952530 | INTERGENIC | 1 |
| | | | | rs1952529 | INTERGENIC | 1 |
| | | | | rs12431659 | INTERGENIC | 1 |
| | | | | rs12434622 | INTERGENIC | 1 |
| | | | | rs10141403 | INTERGENIC | 1 |
| | | | | rs9323738 | INTERGENIC | 1 |
| | | | | rs9323739 | INTERGENIC | 1 |
| | | | | rs1952528 | INTERGENIC | 0,92 |
| | | | | rs1958418 | INTERGENIC | 1 |
| | | | | rs877800 | INTERGENIC | 1 |
| | | | | rs2372550 | INTERGENIC | 0,91 |
| | | | | rs1952545 | INTERGENIC | 1 |
| | | | | rs1952544 | INTERGENIC | 1 |
| | | | | rs10747302 | INTERGENIC | 1 |
| | | | | rs4899838 | INTERGENIC | 0,92 |
| | | | | rs11159610 | INTERGENIC | 0,9 |
| | | | | rs11159611 | INTERGENIC | 0,92 |
| | | | | rs7494095 | INTERGENIC | 0,92 |
| | | | | rs10132137 | INTERGENIC | 0,92 |
| | | | | rs4899839 | INTERGENIC | 0,92 |
| | | | | rs10135095 | INTERGENIC | 0,92 |
| | | | | rs2888357 | INTERGENIC | 0,92 |
| | | | | rs4143911 | INTERGENIC | 0,92 |
| | | | | rs1958458 | INTERGENIC | 0,91 |
| | | | | rs2372553 | INTERGENIC | 0,92 |
| | | | | rs10147836 | INTERGENIC | 0,87 |
| | | | | rs10139812 | INTERGENIC | 0,91 |
| | | | | rs1958456 | INTERGENIC | 0,92 |
| | | | | rs1958455 | INTERGENIC | 0,92 |
| | | | | rs2372523 | INTERGENIC | 0,87 |
| | | | | rs2372524 | INTERGENIC | 0,92 |
| | | | | rs10143348 | INTERGENIC | 0,92 |
| | | | | rs9323740 | INTERGENIC | 0,82 |
| | | | | rs10130591 | INTERGENIC | 0,87 |
| | | | | rs1958449 | INTERGENIC | 0,87 |
| rs7645289 | INTERGENIC | TBC1D5 | 290409 | | | |
| | | | | rs4132218 | INTERGENIC | 1 |
| | | | | rs7627583 | INTERGENIC | 1 |
| | | | | rs9859212 | INTERGENIC | 0,93 |
| | | | | rs6790525 | INTERGENIC | 0,94 |
| | | | | rs4688898 | INTERGENIC | 0,94 |
| | | | | rs9861146 | INTERGENIC | 0,84 |
| | | | | rs2203242 | INTERGENIC | 0,84 |
| rs2088138 | 3PRIME_UTR | UBE2Z | 0 | | | |
| rs2228246 | N/A | N/A | -9 | | | |
| rs2664537 | 3PRIME_UTR | ZHX3 | 0 | | | |
| | | | | rs2228246 | NON_SYNONYMOUS_CO DING | 0,82 |
| | | | | rs6072299 | DOWNSTRE AM | 0,93 |
| | | | | rs6072302 | 3PRIME_UTR | 0,82 |
| | | | | rs7261917 | INTRONIC | 0,82 |
| | | | | rs6065325 | INTRONIC | 0,82 |
| | | | | rs6072317 | INTRONIC | 0,82 |
| | | | | rs6065326 | INTRONIC | 0,94 |
| | | | | rs6072320 | INTRONIC | 0,94 |
| | | | | rs6072321 | INTRONIC | 0,82 |
| | | | | rs6072322 | INTRONIC | 0,82 |
| rs6500497 | 3PRIME_UTR | Q8N7R2_HUMAN | 0 | | | |
| | | | | rs7185630 | 3PRIME_UTR | 0,87 |
| | | | | rs2242166 | 3PRIME_UTR | 0,87 |
| | | | | rs2242164 | UPSTREAM | 0,87 |
| | | | | rs6500498 | 3PRIME_UTR | 1 |
| rs2839178 | INTRONIC | MCM3AP | 0 | | | |
| rs1125890 3 | INTRONIC | FRMD4A | 0 | | | |
| | | | | rs11258902 | INTRONIC | 1 |
| | | | | rs10159816 | INTRONIC | 1 |
| rs2244057 | INTERGENIC | HIF1A | -69366 | | | |
| | | | | rs2253980 | INTERGENIC | 0,94 |
| | | | | rs2246928 | INTERGENIC | 0,95 |
| | | | | rs1006529 | INTERGENIC | 1 |
| | | | | rs698028 | INTERGENIC | 0,84 |
| rs1459841 | INTERGENIC | CAST | -87312 | | | |
| rs7901463 | INTERGENIC | TFAM | 12223 | | | |
| | | | | rs10763541 | INTERGENIC | 1 |
| | | | | rs2081692 | INTERGENIC | 0,87 |
| | | | | rs7903523 | INTERGENIC | 0,87 |
| | | | | rs2101237 | INTERGENIC | 0,93 |
| | | | | rs2086784 | INTERGENIC | 0,87 |
| | | | | rs2393422 | INTERGENIC | 1 |
| | | | | rs1427219 | INTERGENIC | 0,87 |
| | | | | rs919166 | INTERGENIC | 1 |
| | | | | rs919167 | INTERGENIC | 1 |
| | | | | rs10826187 | INTERGENIC | 0,89 |
| | | | | rs10826188 | INTERGENIC | 1 |
| | | | | rs6481390 | INTERGENIC | 1 |
| | | | | rs7071862 | INTERGENIC | 1 |
| | | | | rs7896213 | INTERGENIC | 1 |
| | | | | rs7900673 | INTERGENIC | 1 |
| | | | | rs7916849 | INTERGENIC | 1 |
| | | | | rs10763545 | INTERGENIC | 1 |
| | | | | rs4948298 | INTERGENIC | 1 |
| | | | | rs4948299 | INTERGENIC | 0,87 |
| | | | | rs4948513 | INTERGENIC | 1 |
| | | | | rs2893777 | INTERGENIC | 0,81 |
| | | | | rs7084955 | INTERGENIC | 1 |
| | | | | rs7085387 | INTERGENIC | 1 |
| | | | | rs7073985 | INTERGENIC | 1 |
| | | | | rs7077653 | INTERGENIC | 1 |
| | | | | rs16912268 | INTERGENIC | 1 |
| | | | | rs10826193 | INTERGENIC | 1 |
| | | | | rs2114562 | INTERGENIC | 0,87 |
| | | | | rs2114563 | INTERGENIC | 0,92 |
| | | | | rs1013473 | INTERGENIC | 0,85 |
| | | | | rs2128909 | INTERGENIC | 0,9 |
| | | | | rs1030432 | INTERGENIC | 1 |
| | | | | rs10509091 | INTERGENIC | 1 |
| | | | | rs7077537 | INTERGENIC | 1 |
| | | | | rs4948301 | INTERGENIC | 1 |
| | | | | rs1125139 | INTERGENIC | 0,87 |
| | | | | rs1896248 | INTERGENIC | 0,86 |
| | | | | rs2114561 | INTERGENIC | 1 |
| rs584762 | INTERGENIC | PAX6 | 31249 | | | |
| | | | | rs585972 | INTERGENIC | 1 |
| | | | | rs604518 | INTERGENIC | 1 |
| | | | | rs685428 | INTERGENIC | 1 |
| | | | | rs624732 | INTERGENIC | 1 |
| | | | | rs761453 | INTERGENIC | 1 |
| | | | | rs2440250 | INTERGENIC | 1 |
| | | | | rs594462 | INTERGENIC | 0,95 |
| | | | | rs687947 | INTERGENIC | 0,95 |
| | | | | rs2473858 | INTERGENIC | 0,9 |
| | | | | rs677874 | INTERGENIC | 0,9 |
| rs761453 | INTERGENIC | PAX6 | 38491 | | | |
| | | | | rs585972 | INTERGENIC | 1 |
| | | | | rs604518 | INTERGENIC | 1 |
| | | | | rs685428 | INTERGENIC | 1 |
| | | | | rs624732 | INTERGENIC | 1 |
| | | | | rs584762 | INTERGENIC | 1 |
| | | | | rs2440250 | INTERGENIC | 1 |
| | | | | rs594462 | INTERGENIC | 0,95 |
| | | | | rs687947 | INTERGENIC | 0,95 |
| | | | | rs2473858 | INTERGENIC | 0,9 |
| | | | | rs677874 | INTERGENIC | 0,9 |
| rs6035712 | INTERGENIC | C20orf74 | 132505 | | | |
| | | | | rs6047077 | INTERGENIC | 0,91 |
| | | | | rs6035711 | INTERGENIC | 0,96 |
| rs6868846 | INTERGENIC | hsa-mir-582 | 129225 | | | |
| | | | | rs256353 | INTERGENIC | 1 |
| | | | | rs173945 | INTERGENIC | 1 |
| | | | | rs256348 | INTERGENIC | 0,96 |
| | | | | rs159608 | INTERGENIC | 0,96 |
| rs554710 | UPSTREAM | SURF6 | -15721 | | | |
| rs1013120 | INTERGENIC | HS3ST3A1 | 39975 | | | |
| | | | | rs968026 | INTERGENIC | 0,95 |
| | | | | rs11078172 | INTERGENIC | 1 |
| | | | | rs12952261 | INTERGENIC | 0,9 |
| rs649867 | INTRONIC | Q8N7E7_HUMAN | 0 | | | |
| rs6072317 | INTRONIC | ZHX3 | 0 | | | |
| | | | | rs6072268 | INTRONIC | 0,82 |
| | | | | rs17264110 | INTRONIC | 0,82 |
| | | | | rs6072269 | INTRONIC | 0,82 |
| | | | | rs6072275 | INTRONIC | 0,82 |
| | | | | rs6072286 | INTRONIC | 1 |
| | | | | rs2228246 | NON_SYNONYMOUS_CODING | 1 |
| | | | | rs6072299 | DOWNSTREAM | 1 |
| | | | | rs6072300 | 3PRIME_UTR | 0,94 |
| | | | | rs6072302 | 3PRIME_UTR | 1 |
| | | | | rs2664537 | 3PRIME_UTR | 0.82 |
| | | | | rs7261917 | INTRONIC | 1 |
| | | | | rs6065325 | INTRONIC | 1 |
| | | | | rs6065326 | INTRONIC | 1 |
| | | | | rs6072320 | INTRONIC | 1 |
| | | | | rs6072321 | INTRONIC | 1 |
| | | | | rs6072322 | INTRONIC | 1 |
| | | | | rs8121001 | INTRONIC | 0,88 |
| | | | | rs17181845 | UPSTREAM | 0,94 |
| | | | | rs6072338 | UPSTREAM | 0,94 |
| rs4795069 | INTERGENIC | SLFN5 | 14839 | | | |
| rs1340358 4 | INTERGENIC | CCDC141 | 92384 | | | |
| | | | | rs10171173 | INTERGENIC | 0,86 |
| | | | | rs2200826 | INTERGENIC | 0,9 |
| | | | | rs10497528 | INTERGENIC | 0,81 |
| | | | | rs7574599 | INTERGENIC | 0,9 |
| | | | | rs6747725 | INTERGENIC | 0,9 |
| | | | | rs924800 | INTERGENIC | 0,96 |
| | | | | rs10930847 | INTERGENIC | 0,96 |
| | | | | rs1847420 | INTERGENIC | 0,96 |
| | | | | rs4894072 | INTERGENIC | 0,96 |
| rs6948196 | INTRONIC | RHEB | 0 | | | |
| | | | | rs17713386 | DOWNSTREAM | 1 |
| | | | | rs4726029 | INTRONIC | 1 |
| | | | | rs875588 | INTRONIC | 1 |
| | | | | rs1109089 | INTRONIC | 1 |
| | | | | rs7805967 | INTRONIC | 0,82 |
| | | | | rs17713697 | INTRONIC | 0,9 |
| | | | | rs2074998 | INTRONIC | 1 |
| | | | | rs2074997 | INTRONIC | 1 |
| | | | | rs17713890 | INTRONIC | 1 |
| | | | | rs2299961 | INTRONIC | 0,82 |
| | | | | rs965118 | INTRONIC | 0,97 |
| | | | | rs3753151 | INTRONIC | 0,82 |
| | | | | rs2299965 | INTRONIC | 1 |
| | | | | rs2284264 | INTRONIC | 1 |
| | | | | rs11772458 | INTRONIC | 1 |
| | | | | rs2299967 | INTRONIC | 1 |
| | | | | rs3789817 | INTRONIC | 1 |
| | | | | rs758666 | INTRONIC | 1 |
| | | | | rs736645 | INTRONIC | 1 |
| | | | | rs4298422 | INTRONIC | 1 |
| | | | | rs2374261 | INTRONIC | 1 |
| | | | | rs12112989 | INTRONIC | 1 |
| | | | | rs6980020 | INTRONIC | 1 |
| | | | | rs12112134 | INTRONIC | 1 |
| | | | | rs6943752 | INTRONIC | 0,9 |
| | | | | rs7794922 | UPSTREAM | 0,84 |
| rs2662090 | INTRONIC | SRGAP3 | 0 | | | |
| | | | | rs2670000 | INTRONIC | 0,97 |
| rs7685314 | INTERGENIC | LOC728856 | -28752 | | | |
| | | | | rs6820247 | INTERGENIC | 0,83 |
| | | | | rs7437007 | INTERGENIC | 1 |
| | | | | rs10866319 | INTERGENIC | 0,83 |
| rs2074997 | INTRONIC | RHEB | 0 | | | |
| | | | | rs17713386 | DOWNSTREAM | 1 |
| | | | | rs4726029 | INTRONIC | 1 |
| | | | | rs875588 | INTRONIC | 1 |
| | | | | rs1109089 | INTRONIC | 1 |
| | | | | rs7805967 | INTRONIC | 0,81 |
| | | | | rs17713697 | INTRONIC | 0,9 |
| | | | | rs2074998 | INTRONIC | 1 |
| | | | | rs17713890 | INTRONIC | 1 |
| | | | | rs2299961 | INTRONIC | 0,81 |
| | | | | rs965118 | INTRONIC | 0,97 |
| | | | | rs3753151 | INTRONIC | 0,81 |
| | | | | rs2299965 | INTRONIC | 1 |
| | | | | rs2284264 | INTRONIC | 1 |
| | | | | rs11772458 | INTRONIC | 1 |
| | | | | rs2299967 | INTRONIC | 1 |
| | | | | rs3789817 | INTRONIC | 1 |
| | | | | rs758666 | INTRONIC | 1 |
| | | | | rs736645 | INTRONIC | 1 |
| | | | | rs4298422 | INTRONIC | 1 |
| | | | | rs2374261 | INTRONIC | 1 |
| | | | | rs12112989 | INTRONIC | 1 |
| | | | | rs6980020 | INTRONIC | 1 |
| | | | | rs12112134 | INTRONIC | 1 |
| | | | | rs6948196 | INTRONIC | 1 |
| | | | | rs6943752 | INTRONIC | 0,9 |
| | | | | rs7794922 | UPSTREAM | 0,84 |
| rs6500498 | 3PRIME_UTR | Q8N7R2_HUMAN | 0 | | | |
| | | | | rs7185630 | 3PRIME_UTR | 0,87 |
| | | | | rs2242166 | 3PRIME_UTR | 0,87 |
| | | | | rs2242164 | UPSTREAM | 0,87 |
| | | | | rs6500497 | 3PRIME_UTR | 1 |
| rs1204798 | INTRONIC | NT5DC1 | 0 | | | |
| | | | | rs1204842 | INTRONIC | 1 |
| | | | | rs1204843 | INTRONIC | 1 |
| | | | | rs742930 | INTRONIC | 1 |
| | | | | rs7770203 | INTRONIC | 1 |
| | | | | rs1211388 | INTRONIC | 0,83 |
| | | | | rs1204851 | INTRONIC | 1 |
| | | | | rs1209221 | INTRONIC | 1 |
| | | | | rs1204782 | INTRONIC | 1 |
| | | | | rs1204783 | INTRONIC | 1 |
| | | | | rs1204784 | INTRONIC | 0,88 |
| | | | | rs1204785 | INTRONIC | 0,83 |
| | | | | rs1204786 | INTRONIC | 0,94 |
| | | | | rs1204788 | INTRONIC | 1 |
| | | | | rs1204789 | INTRONIC | 0,88 |
| | | | | rs1204794 | INTRONIC | 0,83 |
| | | | | rs1204797 | INTRONIC | 0,88 |
| | | | | rs1204799 | INTRONIC | 0,83 |
| | | | | rs1204800 | INTRONIC | 1 |
| | | | | rs1204801 | INTRONIC | 1 |
| | | | | rs1204802 | INTRONIC | 0,82 |
| | | | | rs1209223 | INTRONIC | 1 |
| | | | | rs1204807 | INTRONIC | 1 |
| | | | | rs1204814 | INTRONIC | 1 |
| | | | | rs926829 | INTRONIC | 1 |
| | | | | rs1204817 | INTRONIC | 0,83 |
| | | | | rs1204818 | INTRONIC | 0,82 |
| rs1214364 7 | INTRONIC | COG2 | 0 | | | |
| | | | | rs4846999 | INTRONIC | 0,81 |
| | | | | rs12564388 | INTRONIC | 0,81 |
| | | | | rs1887492 | INTRONIC | 0,82 |
| | | | | rs11122569 | INTRONIC | 0,86 |
| | | | | rs11122570 | INTRONIC | 0,82 |
| | | | | rs3736983 | INTRONIC | 0,82 |
| | | | | rs12041242 | INTRONIC | 0,84 |
| | | | | rs12045973 | INTRONIC | 0,84 |
| rs3801033 | INTRONIC | ZNF12 | 0 | | | |
| | | | | rs3801034 | INTRONIC | 0,85 |
| | | | | rs7798471 | INTRONIC | 0,8 |
| rs1099704 4 | INTRONIC | CTNNA3 | 0 | | | |
| | | | | rs1876334 | INTRONIC | 0,84 |
| | | | | rs10822782 | INTRONIC | 0,95 |
| | | | | rs10822783 | INTRONIC | 0,95 |
| | | | | rs1911479 | INTRONIC | 0,95 |
| | | | | rs10997021 | INTRONIC | 0,95 |
| | | | | rs10822784 | INTRONIC | 0,95 |
| | | | | rs4609495 | INTRONIC | 0,95 |
| | | | | rs4745897 | INTRONIC | 0,95 |
| | | | | rs10762045 | INTRONIC | 0,95 |
| | | | | rs10997032 | INTRONIC | 0,95 |
| | | | | rs12763572 | INTRONIC | 0,95 |
| | | | | rs10822791 | INTRONIC | 0,91 |
| | | | | rs10762046 | INTRONIC | 0,91 |
| | | | | rs10822792 | INTRONIC | 0,95 |
| | | | | rs9804181 | INTRONIC | 0,95 |
| | | | | rs10997035 | INTRONIC | 0,95 |
| | | | | rs10997037 | INTRONIC | 0,95 |
| | | | | rs10822794 | INTRONIC | 0,95 |
| | | | | rs10762051 | INTRONIC | 0,91 |
| | | | | rs12357109 | INTRONIC | 0,95 |
| | | | | rs1911485 | INTRONIC | 0,81 |
| | | | | rs1911486 | INTRONIC | 1 |
| | | | | rs10997048 | INTRONIC | 1 |
| | | | | rs10822801 | INTRONIC | 0,95 |
| | | | | rs4523585 | INTRONIC | 0,95 |
| rs7350731 | INTERGENIC | N/A | -9 | | | |
| rs9323737 | INTERGENIC | N/A | -9 | | | |
| | | | | rs1958438 | INTERGENIC | 0,92 |
| | | | | rs10139321 | INTERGENIC | 0,92 |
| | | | | rs10132490 | INTERGENIC | 0,89 |
| | | | | rs10143458 | INTERGENIC | 0,91 |
| | | | | rs9671273 | INTERGENIC | 0,92 |
| | | | | rs1952534 | INTERGENIC | 0,92 |
| | | | | rs1958436 | INTERGENIC | 0,91 |
| | | | | rs11159605 | INTERGENIC | 0,95 |
| | | | | rs7359138 | INTERGENIC | 0,8 |
| | | | | rs1958435 | INTERGENIC | 0,92 |
| | | | | rs1958434 | INTERGENIC | 0,91 |
| | | | | rs1958433 | INTERGENIC | 0,92 |
| | | | | rs2149684 | INTERGENIC | 0,87 |
| | | | | rs2183203 | INTERGENIC | 0,91 |
| | | | | rs1958432 | INTERGENIC | 0,91 |
| | | | | rs2149683 | INTERGENIC | 0,92 |
| | | | | rs8010141 | INTERGENIC | 0,92 |
| | | | | rs1958430 | INTERGENIC | 0,92 |
| | | | | rs1958429 | INTERGENIC | 0,92 |
| | | | | rs1958428 | INTERGENIC | 0,92 |
| | | | | rs1958426 | INTERGENIC | 1 |
| | | | | rs1958425 | INTERGENIC | 0,92 |
| | | | | rs1958424 | INTERGENIC | 1 |
| | | | | rs1958423 | INTERGENIC | 1 |
| | | | | rs1958422 | INTERGENIC | 1 |
| | | | | rs10132081 | INTERGENIC | 1 |
| | | | | rs10132130 | INTERGENIC | 1 |
| | | | | rs10147226 | INTERGENIC | 1 |
| | | | | rs1958421 | INTERGENIC | 1 |
| | | | | rs1958420 | INTERGENIC | 1 |
| | | | | rs10873350 | INTERGENIC | 1 |
| | | | | rs10140080 | INTERGENIC | 1 |
| | | | | rs4545730 | INTERGENIC | 1 |
| | | | | rs1952530 | INTERGENIC | 1 |
| | | | | rs1952529 | INTERGENIC | 1 |
| | | | | rs12431659 | INTERGENIC | 1 |
| | | | | rs12434622 | INTERGENIC | 1 |
| | | | | rs10141403 | INTERGENIC | 1 |
| | | | | rs9323738 | INTERGENIC | 1 |
| | | | | rs9323739 | INTERGENIC | 1 |
| | | | | rs1952528 | INTERGENIC | 0,92 |
| | | | | rs1958418 | INTERGENIC | 1 |
| | | | | rs877800 | INTERGENIC | 1 |
| | | | | rs2372550 | INTERGENIC | 0,91 |
| | | | | rs1952545 | INTERGENIC | 1 |
| | | | | rs1952544 | INTERGENIC | 1 |
| | | | | rs10747302 | INTERGENIC | 1 |
| | | | | rs4899838 | INTERGENIC | 0,92 |
| | | | | rs11159610 | INTERGENIC | 0,9 |
| | | | | rs11159611 | INTERGENIC | 0,92 |
| | | | | rs7494095 | INTERGENIC | 0,92 |
| | | | | rs10132137 | INTERGENIC | 0,92 |
| | | | | rs4899839 | INTERGENIC | 0,92 |
| | | | | rs10135095 | INTERGENIC | 0,92 |
| | | | | rs2888357 | INTERGENIC | 0,92 |
| | | | | rs4143911 | INTERGENIC | 0,92 |
| | | | | rs1958458 | INTERGENIC | 0,91 |
| | | | | rs2372553 | INTERGENIC | 0,92 |
| | | | | rs10147836 | INTERGENIC | 0,87 |
| | | | | rs10139812 | INTERGENIC | 0,91 |
| | | | | rs1958456 | INTERGENIC | 0,92 |
| | | | | rs1958455 | INTERGENIC | 0,92 |
| | | | | rs2372523 | INTERGENIC | 0,87 |
| | | | | rs2372524 | INTERGENIC | 0,92 |
| | | | | rs10143348 | INTERGENIC | 0,92 |
| | | | | rs9323740 | INTERGENIC | 0,82 |
| | | | | rs10130591 | INTERGENIC | 0,87 |
| | | | | rs1958449 | INTERGENIC | 0,87 |
| rs4881394 | INTERGENIC | AKR1C3 | -22872 | | | |
| | | | | rs4525119 | INTERGENIC | 0,8 |
| | | | | rs4881388 | INTERGENIC | 0,8 |
| | | | | rs10795234 | INTERGENIC | 0,96 |
| | | | | rs7074522 | INTERGENIC | 0,96 |
| | | | | rs10795241 | INTERGENIC | 0,8 |
| | | | | rs2096422 | INTERGENIC | 0,85 |
| rs4799159 | INTERGENIC | SALL3 | -459812 | | | |
| | | | | rs185750 | INTERGENIC | 0,85 |
| | | | | rs2578210 | INTERGENIC | 0,85 |
| | | | | rs12456117 | INTERGENIC | 0,93 |
| | | | | rs7506508 | INTERGENIC | 0,93 |
| | | | | rs9952440 | INTERGENIC | 1 |
| rs356999 | INTERGENIC | BCL11A | 30951 | | | |
| | | | | rs357003 | INTERGENIC | 1 |
| | | | | rs357002 | INTERGENIC | 1 |
| | | | | rs356998 | INTERGENIC | 1 |
| rs2270101 | INTERGENIC | FAM126A | -47256 | | | |
| | | | | rs1001027 | INTERGENIC | 0,96 |
| | | | | rs1001026 | INTERGENIC | 0.96 |
| | | | | rs13227316 | INTERGENIC | 0,96 |
| | | | | rs11765548 | INTERGENIC | 0,96 |
| | | | | rs12700402 | INTERGENIC | 0,96 |
| rs618670 | INTERGENIC | N/A | -9 | | | |
| rs7788668 | DOWNSTREAM | FAM126A | -2622 | | | |
| | | | | rs2286497 | INTERGENIC | 0,92 |
| | | | | rs2033670 | INTERGENIC | 0,92 |
| | | | | rs13227654 | INTERGENIC | 0,83 |
| | | | | rs11561822 | INTERGENIC | 0,96 |
| | | | | rs11764386 | DOWNSTREAM | 1 |
| | | | | rs10488277 | 3PRIME_UTR | 0,96 |
| | | | | rs2286493 | 3PRIME_UTR | 0,96 |
| | | | | rs2286491 | INTRONIC | 1 |
| | | | | rs2286490 | INTRONIC | 1 |
| | | | | rs11772725 | INTRONIC | 0,95 |
| | | | | rs11772749 | INTRONIC | 1 |
| | | | | rs11764613 | INTRONIC | 0,83 |
| | | | | rs11768060 | INTRONIC | 0,82 |
| | | | | rs17147527 | INTRONIC | 1 |
| | | | | rs17147529 | INTRONIC | 0,85 |
| | | | | rs11762669 | INTRONIC | 0,88 |
| | | | | rs13225964 | INTRONIC | 0,83 |
| | | | | rs11771543 | INTERGENIC | 0,96 |
| | | | | rs17370052 | INTERGENIC | 0,96 |
| | | | | rs9647996 | INTERGENIC | 0,96 |
| | | | | rs12674362 | INTERGENIC | 0,81 |
| | | | | rs13230424 | INTERGENIC | 0,84 |
| | | | | rs10950935 | INTERGENIC | 0,88 |
| rs902923 | INTRONIC | ROR2 | 0 | | | |
| | | | | rs10991949 | INTERGENIC | 0,88 |
| | | | | rs768056 | INTERGENIC | 0,94 |
| | | | | rs1412466 | INTERGENIC | 0,88 |
| | | | | rs16907585 | INTERGENIC | 0,89 |
| | | | | rs7855644 | INTERGENIC | 0,89 |
| | | | | rs1492679 | INTERGENIC | 1 |
| | | | | rs2131301 | DOWNSTREAM | 1 |
| | | | | rs9409427 | INTRONIC | 1 |
| rs9332172 | INTRONIC | CYP2C9 | 0 | | | |
| | | | | rs17521564 | INTERGENIC | 1 |
| | | | | rs7893293 | INTERGENIC | 0,9 |
| | | | | rs2860905 | INTRONIC | 0,9 |
| | | | | rs4086116 | INTRONIC | 1 |
| | | | | rs4917639 | INTRONIC | 1 |
| | | | | rs1934963 | INTRONIC | 1 |
| | | | | rs4918797 | DOWNSTREAM | 1 |
| rs6873640 | INTERGENIC | ZNF608 | -56628 | | | |
| | | | | rs7725329 | INTERGENIC | 0,97 |
| rs9302822 | INTERGENIC | Q8N9J9_HUMAN | 114376 | | | |
| | | | | rs17539244 | INTERGENIC | 0,84 |
| | | | | rs1019191 | INTERGENIC | 0,84 |
| | | | | rs17442566 | INTERGENIC | 0,84 |
| | | | | rs17442866 | INTERGENIC | 0,81 |
| | | | | rs8050684 | INTERGENIC | 0,81 |
| | | | | rs17540392 | INTERGENIC | 0,81 |
| rs4810622 | INTRONIC | ZMYND8 | 0 | | | |
| | | | | rs4809630 | INTRONIC | 0,93 |
| | | | | rs13038759 | INTRONIC | 0,82 |
| | | | | rs761021 | INTRONIC | 0,82 |
| | | | | rs6124987 | INTRONIC | 1 |
| | | | | rs3803941 | INTRONIC | 1 |
| | | | | rs6124990 | INTRONIC | 0,92 |
| rs3753151 | INTRONIC | RHEB | 0 | | | |
| | | | | rs17713386 | DOWNSTREAM | 0,84 |
| | | | | rs4726029 | INTRONIC | 0,82 |
| | | | | rs875588 | INTRONIC | 0,82 |
| | | | | rs1109089 | INTRONIC | 0,83 |
| | | | | rs7805967 | INTRONIC | 1 |
| | | | | rs2074998 | INTRONIC | 0,84 |
| | | | | rs2074997 | INTRONIC | 0,81 |
| | | | | rs17713890 | INTRONIC | 0,82 |
| | | | | rs2299961 | INTRONIC | 1 |
| | | | | rs2299962 | INTRONIC | 0,9 |
| | | | | rs2299965 | INTRONIC | 0,82 |
| | | | | rs2284264 | INTRONIC | 0,82 |
| | | | | rs2299967 | INTRONIC | 0,84 |
| | | | | rs3789817 | INTRONIC | 0,82 |
| | | | | rs758666 | INTRONIC | 0,82 |
| | | | | rs736645 | INTRONIC | 0,82 |
| | | | | rs4298422 | INTRONIC | 0,84 |
| | | | | rs2374261 | INTRONIC | 0,82 |
| | | | | rs12112989 | INTRONIC | 0,82 |
| | | | | rs6980020 | INTRONIC | 0,82 |
| | | | | rs12112134 | INTRONIC | 0,81 |
| | | | | rs6948196 | INTRONIC | 0,82 |
| rs1049083 2 | SYNONYMOUS_CODING | CNTN3 | 0 | | | |
| rs1545384 | INTRONIC | CNTN3 | 0 | | | |
| | | | | rs1809043 | INTRONIC | 0,9 |
| | | | | rs2197742 | INTRONIC | 0,95 |
| rs1884641 | INTERGENIC | C20orf75 | 5233 | | | |
| | | | | rs6117052 | UPSTREAM | 1 |
| | | | | rs6076925 | INTERGENIC | 0,81 |
| rs1958438 | INTERGENIC | N/A | -9 | | | |
| | | | | rs10139321 | INTERGENIC | 1 |
| | | | | rs10132490 | INTERGENIC | 1 |
| | | | | rs10143458 | INTERGENIC | 1 |
| | | | | rs9671273 | INTERGENIC | 1 |
| | | | | rs1952534 | INTERGENIC | 1 |
| | | | | rs1958436 | INTERGENIC | 1 |
| | | | | rs11159605 | INTERGENIC | 1 |
| | | | | rs7359138 | INTERGENIC | 0,9 |
| | | | | rs1958435 | INTERGENIC | 1 |
| | | | | rs1958434 | INTERGENIC | 1 |
| | | | | rs1958433 | INTERGENIC | 1 |
| | | | | rs2149684 | INTERGENIC | 0,96 |
| | | | | rs2183203 | INTERGENIC | 1 |
| | | | | rs1958432 | INTERGENIC | 1 |
| | | | | rs2149683 | INTERGENIC | 1 |
| | | | | rs8010141 | INTERGENIC | 1 |
| | | | | rs1958430 | INTERGENIC | 1 |
| | | | | rs1958429 | INTERGENIC | 1 |
| | | | | rs1958428 | INTERGENIC | 1 |
| | | | | rs1958426 | INTERGENIC | 0,92 |
| | | | | rs1958425 | INTERGENIC | 1 |
| | | | | rs1958424 | INTERGENIC | 0,92 |
| | | | | rs1958423 | INTERGENIC | 0,92 |
| | | | | rs1958422 | INTERGENIC | 0,92 |
| | | | | rs10132081 | INTERGENIC | 0,92 |
| | | | | rs10132130 | INTERGENIC | 0,91 |
| | | | | rs10147226 | INTERGENIC | 0,91 |
| | | | | rs1958421 | INTERGENIC | 0,92 |
| | | | | rs1958420 | INTERGENIC | 0,92 |
| | | | | rs10873350 | INTERGENIC | 0,92 |
| | | | | rs10140080 | INTERGENIC | 0,92 |
| | | | | rs9323737 | INTERGENIC | 0,92 |
| | | | | rs4545730 | INTERGENIC | 0,92 |
| | | | | rs1952530 | INTERGENIC | 0,92 |
| | | | | rs1952529 | INTERGENIC | 0,92 |
| | | | | rs12431659 | INTERGENIC | 0,91 |
| | | | | rs12434622 | INTERGENIC | 0,95 |
| | | | | rs10141403 | INTERGENIC | 0,91 |
| | | | | rs9323738 | INTERGENIC | 0,92 |
| | | | | rs9323739 | INTERGENIC | 0,92 |
| | | | | rs1952528 | INTERGENIC | 0,84 |
| | | | | rs1958418 | INTERGENIC | 0,92 |
| | | | | rs877800 | INTERGENIC | 0,91 |
| | | | | rs2372550 | INTERGENIC | 0,82 |
| | | | | rs1952545 | INTERGENIC | 0,92 |
| | | | | rs1952544 | INTERGENIC | 0,92 |
| | | | | rs10747302 | INTERGENIC | 0,95 |
| | | | | rs4899838 | INTERGENIC | 1 |
| | | | | rs11159610 | INTERGENIC | 1 |
| | | | | rs11159611 | INTERGENIC | 1 |
| | | | | rs7494095 | INTERGENIC | 1 |
| | | | | rs10132137 | INTERGENIC | 1 |
| | | | | rs4899839 | INTERGENIC | 1 |
| | | | | rs10135095 | INTERGENIC | 1 |
| | | | | rs2888357 | INTERGENIC | 1 |
| | | | | rs4143911 | INTERGENIC | 1 |
| | | | | rs1958458 | INTERGENIC | 1 |
| | | | | rs2372553 | INTERGENIC | 1 |
| | | | | rs10147836 | INTERGENIC | 0,95 |
| | | | | rs10139812 | INTERGENIC | 1 |
| | | | | rs1958456 | INTERGENIC | 1 |
| | | | | rs1958455 | INTERGENIC | 1 |
| | | | | rs2372523 | INTERGENIC | 0,95 |
| | | | | rs2372524 | INTERGENIC | 1 |
| | | | | rs10143348 | INTERGENIC | 1 |
| | | | | rs9323740 | INTERGENIC | 0,91 |
| | | | | rs10130591 | INTERGENIC | 0,95 |
| | | | | rs12894765 | INTERGENIC | 0,87 |
| | | | | rs7157077 | INTERGENIC | 0,87 |
| | | | | rs4904138 | INTERGENIC | 0,87 |
| | | | | rs1952541 | INTERGENIC | 0,87 |
| | | | | rs1958451 | INTERGENIC | 0,87 |
| | | | | rs12882544 | INTERGENIC | 0,87 |
| | | | | rs1958449 | INTERGENIC | 0,95 |
| | | | | rs1952538 | INTERGENIC | 0,87 |
| rs4917639 | INTRONIC | CYP2C9 | 0 | | | |
| | | | | rs17521564 | INTERGENIC | 1 |
| | | | | rs7893293 | INTERGENIC | 0,91 |
| | | | | rs2860905 | INTRONIC | 0,91 |
| | | | | rs4086116 | INTRONIC | 1 |
| | | | | rs9332172 | INTRONIC | 1 |
| | | | | rs1934963 | INTRONIC | 1 |
| | | | | rs4918797 | DOWNSTREAM | 1 |
| rs2279103 | NON_SYNONYMOUS_C ODING | CTDP1 | 0 | | | |
| | | | | rs3809939 | INTRONIC | 0,86 |
| | | | | rs8098133 | INTRONIC | 0,95 |
| | | | | rs3859315 | INTRONIC | 1 |
| | | | | rs3859316 | INTRONIC | 1 |
| | | | | rs898619 | INTRONIC | 1 |
| | | | | rs3809936 | INTRONIC | 1 |
| | | | | rs3786235 | INTRONIC | 1 |
| | | | | rs8087647 | INTRONIC | 1 |
| | | | | rs8084175 | INTRONIC | 1 |
| | | | | rs9953991 | INTRONIC | 0,82 |
| | | | | rs9946977 | 3PRIME_UTR | 1 |
| | | | | rs12605690 | DOWNSTREAM | 0,82 |
| | | | | rs554659 | INTERGENIC | 0,82 |
| | | | | rs551017 | INTERGENIC | 0,82 |
| | | | | rs665138 | INTERGENIC | 0,82 |
| | | | | rs523386 | INTERGENIC | 1 |
| | | | | rs652717 | INTERGENIC | 0,82 |
| | | | | rs496036 | INTERGENIC | 1 |
| | | | | rs576937 | INTERGENIC | 0,81 |
| | | | | rs621490 | INTERGENIC | 0,95 |
| rs7307064 | INTRONIC | RECQL | 0 | | | |
| | | | | rs7307519 | INTRONIC | 1 |
| | | | | rs7976409 | INTRONIC | 1 |
| | | | | rs7976415 | INTRONIC | 1 |
| | | | | rs7956315 | INTRONIC | 1 |
| | | | | rs17627102 | INTRONIC | 0,85 |
| | | | | rs7310464 | INTRONIC | 0,91 |
| | | | | rs1061626 | 5PRIME_UTR | 0,86 |
| rs6793017 | INTRONIC | CNTN4 | 0 | | | |
| | | | | rs6763008 | INTRONIC | 0,88 |
| | | | | rs6774320 | INTRONIC | 0,95 |
| rs523386 | INTERGENIC | Q6ZVY3_HUMAN | 10572 | | | |
| | | | | rs3809939 | INTRONIC | 0,86 |
| | | | | rs8098133 | INTRONIC | 0,95 |
| | | | | rs3859315 | INTRONIC | 1 |
| | | | | rs3859316 | INTRONIC | 1 |
| | | | | rs898619 | INTRONIC | 1 |
| | | | | rs3809936 | INTRONIC | 1 |
| | | | | rs3786235 | INTRONIC | 1 |
| | | | | rs8087647 | INTRONIC | 1 |
| | | | | rs2279103 | NON_SYNON | 1 |
| | | | | | YMOUS_CODING | |
| | | | | rs8084175 | INTRONIC | 1 |
| | | | | rs9953991 | INTRONIC | 0,82 |
| | | | | rs9946977 | 3PRIME_UTR | 1 |
| | | | | rs12605690 | DOWNSTREAM | 0,82 |
| | | | | rs554659 | INTERGENIC | 0,82 |
| | | | | rs551017 | INTERGENIC | 0,82 |
| | | | | rs665138 | INTERGENIC | 0,82 |
| | | | | rs652717 | INTERGENIC | 0,82 |
| | | | | rs496036 | INTERGENIC | 1 |
| | | | | rs576937 | INTERGENIC | 0,81 |
| | | | | rs621490 | INTERGENIC | 0,95 |
| rs7719325 | INTERGENIC | ZNF608 | -493552 | | | |
| | | | | rs17151230 | INTERGENIC | 0,95 |
| | | | | rs17151248 | INTERGENIC | 1 |
| rs1984151 | INTERGENIC | EPHA7 | 399827 | | | |
| | | | | rs2325528 | INTERGENIC | 1 |
| | | | | rs9452447 | INTERGENIC | 1 |
| | | | | rs9294577 | INTERGENIC | 1 |
| | | | | rs2152543 | INTERGENIC | 1 |
| | | | | rs4707810 | INTERGENIC | 1 |
| | | | | rs6904831 | INTERGENIC | 0,96 |
| | | | | rs2000361 | INTERGENIC | 0,95 |
| | | | | rs7765999 | INTERGENIC | 0,85 |
| | | | | rs6925802 | INTERGENIC | 0,83 |
| | | | | rs9294582 | INTERGENIC | 0,85 |
| | | | | rs7752145 | INTERGENIC | 0,88 |
| | | | | rs1238901 | INTERGENIC | 0,85 |
| | | | | rs1219036 | INTERGENIC | 0,82 |
| | | | | rs1219037 | INTERGENIC | 0,81 |
| | | | | rs2248260 | INTERGENIC | 0,85 |
| | | | | rs2801552 | INTERGENIC | 0,82 |
| | | | | rs2801556 | INTERGENIC | 0,82 |
| | | | | rs2633619 | INTERGENIC | 0,82 |
| | | | | rs2801559 | INTERGENIC | 0,82 |
| | | | | rs1601856 | INTERGENIC | 0,82 |
| rs1454333 | INTERGENIC | TMSL3 | 82867 | | | |
| | | | | rs17017774 | INTERGENIC | 0,93 |
| | | | | rs1377917 | INTERGENIC | 0,93 |
| rs1125001 7 | INTERGENIC | Q6ZVI4_HUMAN | -12575 | | | |
| | | | | rs10095339 | DOWNSTREAM | 0,83 |
| | | | | rs10103466 | DOWNSTREAM | 0,96 |
| rs7146332 | INTERGENIC | LRFN5 | -298872 | | | |
| | | | | rs8005889 | INTERGENIC | 0,97 |
| | | | | rs4128092 | INTERGENIC | 0,96 |
| | | | | rs7161171 | INTERGENIC | 0,97 |
| | | | | rs6572078 | INTERGENIC | 0,88 |
| | | | | rs3866730 | INTERGENIC | 1 |
| | | | | rs6572079 | INTERGENIC | 1 |
| | | | | rs8019332 | INTERGENIC | 1 |
| | | | | rs4128087 | INTERGENIC | 1 |
| | | | | rs11628088 | INTERGENIC | 1 |
| | | | | rs 12892392 | INTERGENIC | 1 |
| | | | | rs1431032 | INTERGENIC | 1 |
| rs7175096 | INTERGENIC | N/A | -9 | | | |
| | | | | rs12960630 | INTERGENIC | 0,89 |
| | | | | rs17260406 | INTERGENIC | 0,94 |
| | | | | rs17074095 | INTERGENIC | 1 |
| rs720733 | INTERGENIC | PCDH18 | -443488 | | | |
| | | | | rs6825957 | INTERGENIC | 1 |
| | | | | rs1914603 | INTERGENIC | 1 |
| | | | | rs10857200 | INTERGENIC | 1 |
| | | | | rs10857201 | INTERGENIC | 1 |
| | | | | rs6825255 | INTERGENIC | 1 |
| | | | | rs6853959 | INTERGENIC | 1 |
| | | | | rs4864386 | INTERGENIC | 1 |
| rs2299965 | INTRONIC | RHEB | 0 | | | |
| | | | | rs17713386 | DOWNSTREAM | 1 |
| | | | | rs4726029 | INTRONIC | 1 |
| | | | | rs875588 | INTRONIC | 1 |
| | | | | rs1109089 | INTRONIC | 1 |
| | | | | rs7805967 | INTRONIC | 0,82 |
| | | | | rs17713697 | INTRONIC | 0,9 |
| | | | | rs2074998 | INTRONIC | 1 |
| | | | | rs2074997 | INTRONIC | 1 |
| | | | | rs17713890 | INTRONIC | 1 |
| | | | | rs2299961 | INTRONIC | 0,82 |
| | | | | rs965118 | INTRONIC | 0,97 |
| | | | | rs3753151 | INTRONIC | 0,82 |
| | | | | rs2284264 | INTRONIC | 1 |
| | | | | rs11772458 | INTRONIC | 1 |
| | | | | rs2299967 | INTRONIC | 1 |
| | | | | rs3789817 | INTRONIC | 1 |
| | | | | rs758666 | INTRONIC | 1 |
| | | | | rs736645 | INTRONIC | 1 |
| | | | | rs4298422 | INTRONIC | 1 |
| | | | | rs2374261 | INTRONIC | 1 |
| | | | | rs12112989 | INTRONIC | 1 |
| | | | | rs6980020 | INTRONIC | 1 |
| | | | | rs12112134 | INTRONIC | 1 |
| | | | | rs6948196 | INTRONIC | 1 |
| | | | | rs6943752 | INTRONIC | 0,9 |
| | | | | rs7794922 | UPSTREAM | 0,84 |
| rs2299967 | INTRONIC | RHEB | 0 | | | |
| | | | | rs17713386 | DOWNSTREAM | 1 |
| | | | | rs4726029 | INTRONIC | 1 |
| | | | | rs875588 | INTRONIC | 1 |
| | | | | rs1109089 | INTRONIC | 1 |
| | | | | rs7805967 | INTRONIC | 0,84 |
| | | | | rs17713697 | INTRONIC | 0,9 |
| | | | | rs2074998 | INTRONIC | 1 |
| | | | | rs2074997 | INTRONIC | 1 |
| | | | | rs17713890 | INTRONIC | 1 |
| | | | | rs2299961 | INTRONIC | 0,84 |
| | | | | rs965118 | INTRONIC | 0,97 |
| | | | | rs3753151 | INTRONIC | 0,84 |
| | | | | rs2299965 | INTRONIC | 1 |
| | | | | rs2284264 | INTRONIC | 1 |
| | | | | rs11772458 | INTRONIC | 1 |
| | | | | rs3789817 | INTRONIC | 1 |
| | | | | rs758666 | INTRONIC | 1 |
| | | | | rs736645 | INTRONIC | 1 |
| | | | | rs4298422 | INTRONIC | 1 |
| | | | | rs2374261 | INTRONIC | 1 |
| | | | | rs12112989 | INTRONIC | 1 |
| | | | | rs6980020 | INTRONIC | 1 |
| | | | | rs12112134 | INTRONIC | 1 |
| | | | | rs6948196 | INTRONIC | 1 |
| | | | | rs6943752 | INTRONIC | 0,9 |
| | | | | rs7794922 | UPSTREAM | 0,84 |
| rs7550277 | INTRONIC | RGS7 | 0 | | | |
| | | | | rs11589937 | INTRONIC | 0,86 |
| | | | | rs1110128 | INTRONIC | 0,86 |
| | | | | rs6702310 | INTRONIC | 1 |
| | | | | rs11583779 | INTRONIC | 1 |
| | | | | rs11584248 | INTRONIC | 1 |
| | | | | rs1996805 | INTRONIC | 1 |
| | | | | rs11587158 | INTRONIC | 1 |
| | | | | rs11584771 | INTRONIC | 1 |
| | | | | rs10926454 | INTRONIC | 1 |
| | | | | rs7539742 | INTRONIC | 1 |
| | | | | rs6429256 | INTRONIC | 1 |
| | | | | rs6684760 | INTRONIC | 1 |
| rs635546 | INTERGENIC | L3MBTL4 | -16980 | | | |
| rs301191 | INTERGENIC | N/A | -9 | | | |
| | | | | rs177295 | INTERGENIC | 1 |
| | | | | rs301193 | INTERGENIC | 1 |
| | | | | rs2062246 | INTERGENIC | 1 |
| | | | | rs4857681 | INTERGENIC | 1 |
| | | | | rs4857686 | INTERGENIC | 1 |
| | | | | rs11710431 | INTERGENIC | 1 |
| | | | | rs4857698 | INTERGENIC | 1 |
| | | | | rs13091297 | INTERGENIC | 1 |
| | | | | rs9798968 | INTERGENIC | 0,86 |
| | | | | rs11922088 | INTERGENIC | 1 |
| rs301193 | INTERGENIC | N/A | -9 | | | |
| | | | | rs177295 | INTERGENIC | 1 |
| | | | | rs301191 | INTERGENIC | 1 |
| | | | | rs2062246 | INTERGENIC | 1 |
| | | | | rs4857681 | INTERGENIC | 1 |
| | | | | rs4857686 | INTERGENIC | 1 |
| | | | | rs11710431 | INTERGENIC | 1 |
| | | | | rs4857698 | INTERGENIC | 1 |
| | | | | rs13091297 | INTERGENIC | 1 |
| | | | | rs9798968 | INTERGENIC | 0,86 |
| | | | | rs11922088 | INTERGENIC | 1 |
| rs12583395 | INTERGENIC | NBEA | -296678 | | | |
| rs4388301 | INTERGENIC | TNFAIP3 | 77933 | | | |
| rs4939517 | INTRONIC | CYBASC3 | 0 | | | |
| | | | | rs7111608 | INTRONIC | 1 |
| | | | | rs11230659 | INTRONIC | 1 |
| | | | | rs6591651 | INTRONIC | 1 |
| | | | | rs1037448 | INTRONIC | 1 |
| | | | | rs10750955 | UPSTREAM | 1 |
| | | | | rs3741265 | SYNONYMOUS_CODING | 0,94 |
| | | | | rs10897158 | INTRONIC | 0,94 |
| | | | | rs921635 | INTRONIC | 0,94 |
| | | | | rs3018727 | INTRONIC | 0,94 |
| | | | | rs3019198 | SYNONYMOUS_CODING | 0,93 |
| | | | | rs2860519 | INTRONIC | 0,93 |
| | | | | rs3809083 | INTRONIC | 0,94 |
| | | | | rs2943807 | INTRONIC | 0,94 |
| | | | | rs2943806 | INTRONIC | 0,87 |
| | | | | rs896831 | INTRONIC | 0,87 |
| | | | | rs879647 | INTRONIC | 0,87 |
| | | | | rs3017602 | INTRONIC | 0,87 |
| | | | | rs2943805 | INTRONIC | 0,87 |
| | | | | rs17702 | 3PRIME_UTR | 0,87 |
| | | | | rs1377456 | DOWNSTREAM | 0,87 |
| | | | | rs720888 | DOWNSTRE | 0,84 |
| | | | | | AM | |
| | | | | rs720891 | DOWNSTREAM | 0,87 |
| | | | | rs6591654 | DOWNSTREAM | 0,87 |
| | | | | rs6591655 | INTERGENIC | 0,87 |
| | | | | rs729404 | INTERGENIC | 0,87 |
| | | | | rs729347 | INTERGENIC | 0,87 |
| | | | | rs3018729 | INTERGENIC | 0,87 |
| | | | | rs2924436 | INTERGENIC | 0,87 |
| | | | | rs11230701 | INTERGENIC | 0,87 |
| | | | | rs3019186 | INTERGENIC | 0,87 |
| | | | | rs896829 | INTERGENIC | 0,87 |
| | | | | rs748902 | INTERGENIC | 0,94 |
| | | | | rs730338 | INTERGENIC | 0,85 |
| | | | | rs2943800 | UPSTREAM | 0,94 |
| | | | | rs2957860 | UPSTREAM | 0,93 |
| | | | | rs2924441 | UPSTREAM | 0,94 |
| | | | | rs2924446 | DOWNSTREAM | 0,87 |
| | | | | rs3017605 | DOWNSTREAM | 0,81 |
| | | | | rs2100388 | INTERGENIC | 0,87 |
| rs1037448 | INTRONIC | TMEM138 | 0 | | | |
| | | | | rs7111608 | INTRONIC | 1 |
| | | | | rs11230659 | INTRONIC | 1 |
| | | | | rs6591651 | INTRONIC | 1 |
| | | | | rs4939517 | INTRONIC | 1 |
| | | | | rs10750955 | UPSTREAM | 1 |
| | | | | rs3741265 | SYNONYMOUS_CODING | 0,94 |
| | | | | rs10897158 | INTRONIC | 0,94 |
| | | | | rs921635 | INTRONIC | 0,94 |
| | | | | rs3018727 | INTRONIC | 0,94 |
| | | | | rs3019198 | SYNONYMOUS_CODING | 0,94 |
| | | | | rs2860519 | INTRONIC | 0,94 |
| | | | | rs3809083 | INTRONIC | 0,94 |
| | | | | rs2943807 | INTRONIC | 0,94 |
| | | | | rs2943806 | INTRONIC | 0,87 |
| | | | | rs896831 | INTRONIC | 0,88 |
| | | | | rs879647 | INTRONIC | 0,87 |
| | | | | rs3017602 | INTRONIC | 0,88 |
| | | | | rs2943805 | INTRONIC | 0,88 |
| | | | | rs17702 | 3PRIME_UTR | 0,87 |
| | | | | rs1377456 | DOWNSTREAM | 0,88 |
| | | | | rs720888 | DOWNSTREAM | 0,84 |
| | | | | rs720891 | DOWNSTREAM | 0,88 |
| | | | | rs6591654 | DOWNSTREAM | 0,88 |
| | | | | rs6591655 | INTERGENIC | 0,88 |
| | | | | rs729404 | INTERGENIC | 0,88 |
| | | | | rs729347 | INTERGENIC | 0,87 |
| | | | | rs3018729 | INTERGENIC | 0,88 |
| | | | | rs2924436 | INTERGENIC | 0,88 |
| | | | | rs11230701 | INTERGENIC | 0,88 |
| | | | | rs3019186 | INTERGENIC | 0,88 |
| | | | | rs896829 | INTERGENIC | 0,88 |
| | | | | rs748902 | INTERGENIC | 0,94 |
| | | | | rs730338 | INTERGENIC | 0,86 |
| | | | | rs2943800 | UPSTREAM | 0,94 |
| | | | | rs2957860 | UPSTREAM | 0,94 |
| | | | | rs2924441 | UPSTREAM | 0,94 |
| | | | | rs2924446 | DOWNSTREAM | 0,88 |
| | | | | rs3017605 | DOWNSTREAM | 0,82 |
| | | | | rs2100388 | INTERGENIC | 0,88 |
| rs2348427 | INTRONIC | ENPEP | 0 | | | |
| | | | | rs 1126483 | NON_SYNONYMOUS_CO DING | 1 |
| | | | | rs12503640 | INTRONIC | 1 |
| | | | | rs2881913 | INTRONIC | 1 |
| | | | | rs2348429 | INTRONIC | 1 |
| | | | | rs17551888 | INTRONIC | 1 |
| | | | | rs6842486 | INTRONIC | 1 |
| | | | | rs10015807 | INTRONIC | 1 |
| | | | | rs12506732 | INTRONIC | 0,96 |
| | | | | rs2348431 | INTRONIC | 0,96 |
| | | | | rs6813802 | INTRONIC | 0,96 |
| | | | | rs3796889 | INTRONIC | 0,96 |
| | | | | rs3796888 | INTRONIC | 1 |
| | | | | rs2348433 | INTRONIC | 1 |
| | | | | rs1448808 | INTRONIC | 0,8 |
| rs1891877 | INTRONIC | PIP4K2A | 0 | | | |
| rs8095186 | INTERGENIC | PIK3C3 | 251736 | | | |
| | | | | rs346457 | INTERGENIC | 0,92 |
| rs9480684 | INTRONIC | AIM1 | 0 | | | |
| | | | | rs1770728 | INTRONIC | 0,93 |
| | | | | rs1770731 | INTRONIC | 0,91 |
| | | | | rs1770732 | INTRONIC | 0,93 |
| | | | | rs1676016 | INTRONIC | 0,93 |
| | | | | rs2297971 | INTRONIC | 0,93 |
| | | | | rs2066202 | INTRONIC | 0,86 |
| | | | | rs2054366 | INTRONIC | 1 |
| | | | | rs2615206 | INTRONIC | 1 |
| | | | | rs2615207 | INTRONIC | 1 |
| | | | | rs2642469 | INTRONIC | 1 |
| | | | | rs1037955 | INTRONIC | 1 |
| | | | | rs965347 | INTRONIC | 1 |
| | | | | rs4946764 | INTRONIC | 1 |
| | | | | rs9486398 | INTRONIC | 1 |
| | | | | rs9486399 | INTRONIC | 1 |
| | | | | rs9480685 | INTRONIC | 1 |
| | | | | rs9486403 | INTRONIC | 1 |
| | | | | rs9480686 | INTRONIC | 1 |
| | | | | rs9486404 | INTRONIC | 1 |
| | | | | rs3747790 | 3PRIME_UTR | 0,93 |
| | | | | rs13319 | 3PRIME_UTR | 1 |
| | | | | rs9320182 | 3PRIME_UTR | 1 |
| | | | | rs9320183 | 3PRIME_UTR | 1 |
| | | | | rs9486410 | SYNONYMOUS_CODING | 1 |
| | | | | rs9486411 | INTRONIC | 1 |
| | | | | rs9486414 | INTRONIC | 1 |
| | | | | rs9486415 | INTRONIC | 1 |
| | | | | rs6926307 | INTRONIC | 1 |
| | | | | rs6926670 | INTRONIC | 1 |
| | | | | rs6926833 | INTRONIC | 1 |
| | | | | rs6907164 | INTRONIC | 1 |
| | | | | rs17067368 | INTRONIC | 1 |
| | | | | rs9480689 | INTRONIC | 1 |
| | | | | rs4523123 | INTRONIC | 1 |
| | | | | rs7741101 | INTRONIC | 1 |
| | | | | rs7776287 | INTRONIC | 1 |
| | | | | rs7776294 | INTRONIC | 1 |
| | | | | rs12523774 | INTRONIC | 0,92 |
| | | | | rs6903754 | INTRONIC | 1 |
| | | | | rs4945759 | INTRONIC | 1 |
| | | | | rs9689006 | INTRONIC | 1 |
| | | | | rs7770930 | INTRONIC | 1 |

***Table 4:* Subset of 100 associated SNPs with tagged SNPs (r² > 0.8).** SNP: reference sequence identification number according to dbSNP; type: location/function of SNP; CHR: chromosome number; MAP: physical location of SNP on genome build HG18. tagged SNP r²: Linkage disequilibrium (LD) in the observed regions was assessed by calculating r² according to Hill et al. 1968.²⁵

It is known in the art that two or more alleles, polymorphisms or SNPs may be associated in a non-random manner. Such SNPs are also referred to as being in linkage disequilibrium and are inherited together. A genomic region comprising a group of SNPs being in linkage disequilibrium may also be referred to as haplotype block. SNPs being located within the same haplotype block may also be referred to as "tagged SNPs". Each of the SNPs provided in Table 1 may be replaced with a corresponding tagged SNP from Table 4 for the purpose of the present invention. Corresponding tagged SNPs for a given SNP provided in column 1 of Table 4 are those tagged SNPs which are listed in column 5 of Table 4 subsequent to the given SNP. For example, SNPs rs1872133, rs1872132, until SNP rs12899914 are tagged SNPs corresponding to SNP rs1630535. Preferred tagged SNPs are those with a correlation coefficient (r2 value) of 1. The risk alleles of the tagged SNPs are those alleles which co-occur with the risk allele of the respective SNP as listed in Table 1. "Co-occurrence" in this context refers to those alleles of a group of SNPs within a haplotype block which occur together. Determining co-occurrence is within the skills of the skilled person.

The figures show:
**Figure 1**
   Distribution of risk alleles in cases and controls applying the subset of 18 SNPs according to the invention in the discovery sample (see Table 3).
**Figure 2**
   Receiver Operating Characteristics (ROC) curve of the number of risk alleles as a predictor applying the subset of 18 SNPs according to the invention in the discovery sample. The area under the curve is 1.0 hence yielding a perfect classification.
**Figure 3**
   Distribution of risk alleles in cases and controls applying SNPs as defined by SEQ ID NOs: 1 to 100 in the discovery sample.
**Figure 4**
   Receiver Operating Characteristics (ROC) curve of the number of risk alleles as a predictor applying SNPs as defined by SEQ ID NOs: 1 to 100 in the discovery sample. The area under the curve is 1.0 hence yielding a perfect classification.

The following examples illustrate the invention but should not be construed as being limiting.

### Example 1

### Methods

### Discovery sample - patient recruitment

We recruited 397 patients aged 18 to 75 years who were admitted to the hospital of the Max Planck Institute of Psychiatry (MPI), Munich, Germany, for treatment of a depressive disorder presenting with a unipolar depressive episode (85.7%), bipolar disorder (12.4%, 6.1% bipolar I and 6.3% bipolar II), or other primary diagnosis with current depression (0.5% dysthymia, 0.7% adjustment disorder). Patients were diagnosed by psychiatrists according to the Diagnostic and Statistical Manual of Mental Disorders (DSM) IV criteria and were included in the study within the first three days after in-patient admission. Patients with depressive disorder due to a general medical or neurological condition were excluded, as were patients with a lifetime diagnosis of intravenous drug abuse and depressive symptoms secondary to alcohol or substance abuse or dependency. We recorded ethnicity using a self-report sheet for nationality, first language and ethnicity of the patient and of all four grandparents. All individuals included were Caucasian, 85.1 % were of German origin. The study was approved by the local ethics committee, and written informed consent was obtained from all subjects.

The study is designed as a naturalistic pharmacogenetic study (Munich Antidepressant Response Signature (MARS) project) to reveal genetic biomarkers specific for depressive disorder, clinical outcome and severe side effects. All patients are treated with antidepressants according to doctor's choice. Concomitant psychotropic medication with mood stabilizers, neuroleptics, benzodiazepines and hypnotics is allowed. For all patients plasma concentrations of antidepressants were monitored to assure clinical effective drug levels.

### Replication sample

The German replication sample consisted of 501 Caucasian inpatients from the psychiatric hospital of the University of Münster and from a second patient recruitment in the Max Planck Institute of Psychiatry (MPI), Munich. Gender distribution (p>.2) and age (p>.9) did not differ between samples. Overall, 85% of these patients suffered from major depression, while 15% were in a depressive episode of a bipolar disorder. TESI positive were N=42/8.4%, patients completely lacking suicidal ideation N=149/29.7% and N=434/86.6% without increase in suicidal ideation. Psychiatrists ascertained DSM IV diagnosis. Patients were rated weekly from admission to discharge (Munich) or until week 6 (Münster) using the 21-item HAM-D rating scale. Ethnicity was recorded using the same self-report questionnaire as in the MARS study. All patients were Caucasian and 90.7% were of German origin; the remaining patients were of European descent (Central Europe: 3.9%; Eastern Europe: 5.3%; Mediterranean: 0.1%). Same inclusion/exclusion criteria applied as in the MARS sample, and outcome under antidepressant treatment was evaluated accordingly.

### Psychopathology and phenotype definition

Severity of depressive symptoms was assessed at admission by trained raters using the 21-item Hamilton Depression Rating Scale (HAM-D) ^{26, 27}. Patients fulfilling the criteria for at least a moderate to severe depressive episode (HAM-D- >= 14) were eligible. Ratings were performed within five days of admission and then weekly until discharge. The severity of suicidal ideation was rated by item 3 "suicide" of the HAM-D, with 0 "absent"; 1 "subject feels life is not worth living"; 2 "wishes he/she were dead or any thoughts of possible death to self"; 3 "suicidal ideas or gestures" and 4 "attempts at suicide". TESI was defined as an increase of suicidal thoughts in patients without suicidal ideation (item 3 of the HAM-D = zero) at hospital admission (n = 32). The non-TESI comparison groups were 1) all individuals without increase in suicidality independent of baseline suicidality (no increase of the HAM-D item 3 over time; n = 329) and 2) a sub-group of 1), patients rating zero on item 3 throughout treatment (item 3 of the HAM-D = zero at all visits; n = 79). In the STAR*D trial the suicide item (item number 12) of the Quick Inventory of Depression Symptomatology-Self Report (QIDS-SR) was used to define TESI Both scales should identify similar patients as the QIDS-SR has been shown to correlate well with the HAM-D ²⁸⁻³¹. The observation period for TESI was the first 12 weeks following in-patient admission, congruent with the observation period used in Laje et al., 2007 32

### DNA preparation

After enrolment in the study 40 ml of EDTA blood were drawn from each patient. DNA was extracted from fresh blood using the Puregene® whole blood DNA-extraction kit (Gentra Systems Inc; MN).

### Statistical Analysis of genetic associations

Exact tests on Hardy-Weinberg equilibrium (HWE) were performed for all SNPs ³³. SNPs with a minor allele frequency below 2.5 %, with a call rate of less than 98 %, or displaying HWE deviation at an error level of below 10⁻⁵ were excluded from the analysis. Final analysis was done in 371.335 SNPs. Both allelic and genotypic association tests were applied. To avoid false positive associations due to small cell sizes we used permutation-based p-value estimates (100,000 permutations, in 4 best SNPs 100,000,000 permutations) in addition to asymptotic p-values. If not otherwise specified, permutation-based p-values are reported. Genetic association was tested using the WG-permer, a C++ based statistical program for rapid permutation (http://www.wg-permer.org) Phenotypic analyses were performed using SPSS version 16.0.

### Results

The comparisons of disease-related and sociodemographic variables between TESI positive and negative patients using both the broader (comparison group 1) and more restricted definition (comparison group 2) are listed in Table 5. Except for age at onset there were no significant differences among these groups. 59.3 % of patients with TESI developed this side effect within the first 2 weeks after hospital admission. Current medication at the onset of suicidal ideation were SSRIs in 37.5 %, tricyclic antidepressants in 34.3 %, venlafaxine or duloxetine as dual serotonin and noradrenalin reuptake inhibitors in 25 %, mirtazapine in 28 % and reboxetine as a selective noradrenalin reuptake inhibitor in 12.5 %. Antidepressant monotherapy was administered to 62.5 % of the patients, while 37.5 % received combination therapy. There were no significant differences in psychotropic medication among the three groups (Table 6).

**Table 5**

| **Characteristics of discovery sample** | **TESI** | **Non-TESI (1)** | **Non-TESI (2)** | χ² **or t-test** |
|---|---|---|---|---|
| | n =32 | n=329 | n=79 | |
| | 8.1 % | 82.6 % | 19.9 % | |
| Male | 43.8% | 44.8 % | 49.5 % | NS |
| Female | 56.3 % | 55.2 % | 50.5 % | NS |
| Age (mean +/- SD) | 47.1 (16.1) | 49.3 (14.1) | 50.8 (14.3) | NS |
| Employment status | | | | |
| *employed* | 62.9 % | 63.4 % | 61.1 % | NS |
| *retired* | 25.7 % | 24.1 % | 23.4 % | NS |
| *unemployed* | 11.4 % | 12.5 % | 15.5 % | NS |
| Living with partner / children / personnel | 54.3 % | 60.9 % | 64.5 % | NS |
| | 45.7 % | 39.1 % | 35.5 % | NS |
| Single | | | | |
| Age at onset (mean. +/- SD) | 32.6 (16.0) | 37.3(15.4) | 40.6 (15.6) | * |
| HAM-D at inclusion (mean. +/- SD) | 24.5 (7.1) | 26.6 (6.6) | 22.8 (6.5) | NS |
| Previous episodes (mean. +/- SD) | 3.2 (4.0) | 2.8 (5.3) | 2.7 (5.5) | NS |
| Recurrent depression | 56.3 % | 58.2 % | 60.4 % | NS |
| Psychotic features | 18.8 % | 11.9 % | 13.9 % | NS |
| Illness duration in years (mean. +/- SD) | 13.9 (15.4) | 10.6(10.5) | 10.3(10.1) | NS |
| Duration of current episode in weeks (mean. +/- SD) | 38.0 (58.4) | 39.1 (69.3) | 28.4 (21.7) | NS |
| Family history of depression | 46.9 % | 46.6 % | 40.5 % | NS |
| History of attempted suicide | 18.8 % | 28.7 % | 16.2 % | NS |

***Table** 5:* **Sociodemographic and clinical features** of patients in the discovery sample developing TESI compared with patients without worsening of suicidal thoughts under psychopharmacological treatment (1) and with patients completely lacking suicidal ideation (2). * p = 0.012 TESI vs. Non-TESI (2). NS = not significant.

**Table 6**

| **Medication** | **TESI** | **Non-TESI (1)** | **Non-TESI (2)** | |
|---|---|---|---|---|
| | n=32 | n=79 | n=329 | t-test |
| SSRI | 37.5 % | 37.8 % | 35.7 % | NS |
| Tricyclic antidepressants | 34.4 % | 20.4 % | 22.9 % | NS |
| SNRI | 25 % | 19.4 % | 19.2 % | NS |
| Mirtazapine | 28 % | 35.7 % | 34 % | NS |
| NRI | 12.5 % | 2 % | 4 % | NS |
| Neuroleptics | 21.9 % | 17.3 % | 22 % | NS |
| Mood stabilizers | 46.9 % | 35.7 % | 29.9 % | NS |
| Benzodiazepines | 50 % | 20.4 % | 30% | * # |

***Table* 6: Psychotropic medication at onset of TESI compared with medication of control group in the discovery sample**
Medication was according to doctor's choice. A combination of two or more antidepressants was also allowed (TESI=37.5%; Non-TESI (1) = 23.5%; Non-TESI (2) = 24.4 %; no significant difference). * TESI vs Non-TESI (1) p < 0.05; # TESI vs Non-TESI (2) p < 0.05. NS = not significant

Provided are 100 SNPs, which were significantly associated with TESI with an empiric p-value < 0.001 comparing to both non-TESI groups (see Table 1). SNP rs1630535 showed a p value of 1.3x10⁻⁷, OR 10.535, Cl 4.2-26.4 comparing to narrow non-TESI group, and p value of 2.45x10⁻⁷, OR 4.16, Cl 2.3-7.4 comparing to broader non-TESI group. Fisher Product Method over all 100 SNPs revealed a significant association comparing TESI vs non-TESI(2) with allelic p=1x10⁻⁵, genotypic p=1x10⁻⁵ and combination of allelic and genotypic p=1x10⁻⁵. A discriminant analysis patients using this subset of SNPs revealed a 100 % probability to classify TESI vs non-TESI cases correctly, no matter if narrow or broader non-TESI definition was applied (narrow definition, 32 vs 79: chi square = 273 and df = 91; broader definition, 32 vs 329: Chi square =429 and df = 96). Furthermore, the distribution of the number of risk alleles present in each individual yielded two separate groups, with TESI cases having 85 and more risk alleles while non-TESI individuals having 80 and less risk allies (see Figure 3).

100 SNPs with p<0.001 from the discovery sample were tested in a second independent German Caucasian sample (referred to as "replication sample"), revealing 16 SNPs particularly significantly associated with TESI in both discovery and replication sample.

**Table 7: Sequences of the SNPs of SEQ ID NOs: 1 to 100; in no particular order. The polymorphic position is indicated by square brackets, for example [A/G] in case of rs 6793017**

| SNP rs# | Sequence |
|---|---|
| rs67 9301 7 | ATAATTGTTTGAGTCACAGAGGTTCCTACNACTCCATTAAGACAGAAGGTATAGCCTAGAGTTGGTGGGTCTGAGACAGTGTCTCCAAAGTCTGAAGG ATAATAACTGAATATAGACTCCAAAATTTAAGGCTTCAGCATCCTCAGAGTCCTTGCATTTGTGAGAGCTATGTCTGTGATGTTGCCGTTTGTCACCAG GAT[A/G]CTTTATGTTGTCTTTTAAGTGTTAGGTGTCCAGATGCAGATCACCTGCATCTTGCCTCATATGTCTCTCTGAATGTAAAGATTTTGCCTACATC TGATGAAAACTGTCCACTGTTCACATTATTTGTCATATGTATCTGCTTGGAGCAGAGTAAGACACTTCTAAGGGAACTTGACATTTCATGACCCAGGAT ATCAGAT |
| rs12 6523 5 | ACTGCATCTGCGTAGACNTGTCATCCAGTATACTCATGCGATGTCACTCATCTGCCCCCTCCTACATAAATGATACACAAAAAGTCAACAATCAGAGA AACCCAAGCTCCTGGATGTCAACATCCACTTCTGCTTGGAGTTGGTTCCTTTCATGGAGACATACACCCCTCGCCACACACCTGCCACCTCCCTGACC CTC[A/G]CAATCCCTGGCATGAGAAGCTCCACCTTGTCAAGAGCAGACATTCTGAAAGGCTGAAGCCAGGAACCTTAAAGGAAACATTCTGCCAAGTC CACCACGTAGGATCAATGGAGATGGTGGTAGTGTGGTTATTGGCTTACCCAGCTCTGGGTGTAGAGTGCTTTGGGGAGGAGGAAGGAAGAAGAGCT GATCCTGCCTTCT |
| rs48 8139 4 | ATCAGTGGCAGAGACTCTCCCTGCTCTCATGGGCTCTGTCTATTCCCTCTCTACATAATNAGAGTAAAATCCAAAGAGCATGAGGACCAAATCAAATAT GACAAAGACTCCTTAGAGGCGTGTCAGTACTGGTAACACCTGTTTTCACAACTCACGGTGACTAAGGCACAGACCACCCCACACAGTGGCACGGAT TCTC[C/T]TCTTGAACAGACTTTCCCCAGGCTCCTCTGAGTCTCTTTTGAAATAGGCCTCGCCTTGGTCTGTAAGGATCTGAACACACCCAAACATAATT TCTAACAGTTCAAGGCTACATCAATAGATAACCAAGCCCTGTTAAAGAGCCAGCCTGAGAAAACTCACGGCTTCCAAAAGAATTCAGTATTTGTTCCAG CAATGTCTG |
| rs47 2470 1 | CCCTGGTTCCACCGCAGGCGAAGTCAAGATCCAAAGCAAAGTCGAGGACACAGTGACCGTGTTTTAAAACCGCCATGAACCAGGGCGGACCNCGA CACCCAGATGAGAGCCGTGAGGACAGCACTGAGAATAATCACTTAGGGCTTTATTTACTGACACAAGAGGACTCCACGGTGTAGTGTTACATTTTTAA AAATCA[A/G]ATTACAAAACAATATGAGAACCCTATCTCCAGTTCATTCTCCAGGGAAAGTTGGCAGCATTCACTGTTGGGAAACCTTCACTTAAAAATA GGTTGATGTTGGCCAGGCGTTGTGGCTCACGCCTGTAATCCCAGCACTTTGGGAGGCTGAGGTAAGCGGATCACCTGAGGTCAGGAGTTAGAGACC AGCCTGGGCAACAT |
| rs63 5546 | TCTGCCTGCCTGGGGCCACCACCCACCCAGTCATACACATAGGACAAGCTCAAGAGCCCTCCAACTCACTTAGTCACTTCCCCCAAATCAGATGTGA CTGAATCCTATCGATTTTGCTTCCTAAATAGCCTGGGAACAGCCCCTCTCCACTCTGGCTGCCATTTTCCTATGGAAATGACAGTGTTTCTCACCTAGA TTAC[C/T]TTCACCACCTCCCACATAGCCTCCCTGCCTCCAGCCTTCTCCCTTCTTGGATATGCACCAAAGTGACCTTTATCTAATACCCAGGGTTAATC ACTTCCCACTCCTACTGAGAACANTTCAATGGCTCCTTATTGCCCCCCAGTTTCTCAAATGCCTTAGATCAGTGGTCACCAACCATTTTGGCACAAGGG ACCAGTTTC |
| rs18 8464 1 | ATGGATTTTAAAAGGATCTCAAATGGTATCTTCTAAAATTCTAAGCATTCTATCAATCCTCATGCTGAAATATGACTTGGTGAGTGACACTTCAAAAAGA AAATAAACATGCGTCTCAACCCATTACTACTTTATTTCCTCCAGATCATTTCCTCACAGGCAGGGCAGCCACGTCAGTGAACCCACTCAGATATCTTGG T[G/T]TCTACCACTTGGAAGCCTCTTTTCTACATTCCATTTTGGGAGAGAGCTGGCTCTTCCCGGAGACTTGACAACCTGGTACCGTCTCCTCCCACAA TCACCCTCCCATTCTTCTGGCTTATCTCTGGTGTCAGAGAAGAATGTAATCTTTAGGCGTGTACATTCTGACTCCCATGTTTTGGGGAAGACCCCTCAG CGTAGCT |
| rs93 0282 2 | AATACCTAAGTAGGGTGATTGGAGGTCAAGGACCCTTGTAGGAATGAAGTGGATTTGCCTGGATGTAAAACTACATCTAAGATGTTATGAGTGAGGAA GAGCCCAAAACTCAGAAATCTGTTTGAAAAACTTTGTTAGAAAGCANACATACACATGCAATTGTTTTACATAGAGGGCAATACTGCAAAAGATAATGA AGG[A/G]ACTATAAACAGTGATTATCTTTGTTGTAGCTGTGCAGTATGACTAAGAGAGGGGCAGGGAAGAGATGAAGCTTTTGCTTTTAAGATTNCACC TTTATTCTTTGTGATTTTCAGCAAAGATGCCCTATTTGGATAATTAAAAAATANTTAAGAGAAAAGCCTCCATTTTGCAAACAACCTGTGATCACTTTACC CCTGAGC |
| rs38 0103 3 | ACCTGACTACTGTACTTGCTATCATGCACATTAAGTGAAACGATATCAACCGTAACCCCTATAACCTGTGCTTTCCCTGAACCCACTGCCAAAATATCA ACAGATCAAATGAGAAAAAAATCTGAGAGACAATACTTTTCACCACCTGGGATCACTATATGCNGCCAAGCTAGGATGACAGTCATCTAACAAAAATAT TC[C/T]GTTGCAGGAAAAGCAAGCTATTTGTTGCACAAGGAGGTTCCTAAGATAAGTCCAAGTAGAGAGTTGTTCTGGAGGAAGTGGGTCTGCACTTTA CACCTTCTCATGTCACATCAAGCTGGGAGAGATAGGTCATGATCTATCTCTTATCCTTGCCTTANGCTACGATCACTGTGTGCCTGATCAATGCCCAG CACATTTCA |
| rs26 6209 0 | CTTCAGGTGGTTGAAGCTTAAATGAGGCGGACAGAGATGAGCTACAGAGCCCCAGGCTGTAATTTGCGCTAACCAGTGGATGACCTNCCAAGGCATG AGTTAGGCATTTGGGATTACATCTTCAACTTCTTGGAGCAGTGAGACACGGTGGACAGAATATCAGCTTACCCCTCAGCTCTGCTTTTTTGGGGAATT CTTGG[A/C]CAGGAGATAAAATGAGAGGACATTATTGCTGGAAGCATCTATAGAGAGCCTCTAGGACAGTAGTTACCAAACTGAAGTGTGCATTTGAAC CATCTGGGAAGCTTGTTAAAAATGCAGGTTCTCAGCCAGGCGCAGTGGCTCATGCCTGTAATCCCAACACTTTGGGAACCGAGGTGTGCAGATTGCT TGAGCTCGTGAGT |
| rs27 9553 | TAGCTAATGTTGTTGCGGGGAAAGGAGAGAAGGCCCAAGAGAGAGGGCAAAANGCTCAGCAAATACGGGTATTCCCACTGATAGGGCATGGCCACC TGATCATGTGACAAGAGCCTCAGGTGTCCCACGCTCATCTTAGCAACCAGCAGCAGCCATATGACCAGATGTACGTAGATCAGCTTCTTGATTTCATA CTTGAG[A/G]GTCACACTGTGGGGCAGAGCAGAGGGAGAAGTATCTTTCAAATTTTTCTTACCACCAACTATATGCCAGACAACTGTACTGGACACTGG GATACAAATTTGAATAAAACAGCCTAGAAGCCGGACACAAAGAACAGAGGAGGAAAAAGTAATTAGAAAATGAAGTGAAAAAGTTCTNGTAAATATATG TTCCCAGATATA |
| rs27 9542 | GTGTGTTGCTTGAAAGGTGTGGCCTTAGACACTAGGAGATGATGGGTAGAAGCTCCTTGAGAAACCTAGCAGTGGCCATAGAGAAGTAGACTGGGTA TGGGGAACCTAAGTCATAGTTGTAACCTACAGCTGATTGGTAAGAACTATGGGCCAGACATTTAATCTCTCCATCTATGAAATAGTCCATTCTCATTTAT CTA[C/G]CTTAGGTTATGGGCTCTTCTTTTAGTTGGATATTCACATTTATCTTTCAGTGTTAACTGGGTATCAGTTATGAGTCTACTACTGTATAATGGCA TCCAAAAGAATTTNAAAGTACCTCNGTTTATAGGGATTTACAATTCAGTAGAAACTTGTTATTCATGTGAAACAGTGAACATAATATCTAATGATGTGGA GCAAGT |
| rs11 2500 17 | TCTTATAATAAATCTATCTGTCTATATCTATATCTACATCTGTCCTCCTACTGGTTCTGTTTCTCTGGACAACCCTAAGACTAGTATATAAAAAATAGANC AAACTTTCAGATTGAGAAAAAAGACATACAAGGCAAATGCATAAGAGATAAGTTTTATGATATATTGATATTCAACATCTTAACATAATTAAATGAAGA[C/ T]GGGAAGTCATTGAAAGAACAAAGGAGAGTTCATGTTTACTGCTACAATGTGCAATGAAGATATAAGTTATTAATATTTTTGGAAATTTTAAAACATCTC TCCCATANCTATACAAATCAAGTGGACCAAAAAATTGAGTAAGGATGTGGAAAAACCAGCATTGTCAGTAAGCTAGAACACATAAATATATATATTGAAGT TG |
| rs10 1312 0 | CAAACTTAAAAATAACAATTTTCCCAGAAGGCCAAATGTTTAGGGAACATTTTCCCCCAGAGGACTCAGGGGTAAAACGAATCTGAAGACCTTAATTAT TCAATGTCCCCAAGCCTCTCTTCCTAAACGTGGAATGNGGATGAGAAGGAATCACTTACAAGAGAGAGAACTGTGTGCTAAATATCAGGAGACAAGTC GCC[A/C]AAAATTCTAGGTAATGGTAGTAAAGTAAGAATAGGCANCCTAAGGTATCAAAGGTCAAACGTGGTCCCCCAAACACCTGACCTGCAAACCTA GGCAGATAGCAGCTCTAGAGAAGAAGAAATGGCTGGAGCAGAGAGTACGTGGCTCAGTATATTAGACCTTATTTGCCACATAGATGACTTCCAAAAAT ACTCTTTTGG |
| rs11 2589 03 | AAAATTTTAAATTAAATCCAAAAATACATAAAATTAACAACGTTCAAATTTATTGCTTNACCCTGATATTNATATTTTTTGGCTGAAATGAAATNGCAGTTT TTAGCATGAATATGTTTCTGACTCCTCTTCTGTGGGTTCTTTTGAGATTGGCATGTCTATATTGCCCCGTGCTGGGTTAGGAGTCAATTCTCCTATNG[C /T]TTTTTTCCTACCCAACCTTCTGCAAACCTTGAACAATACAGCGAGAGGCCCTTAGCTTACATTTGGCAGAACCACATCATTTGTTACTAAGTAAATCT CTAGTCTTTCTTTGTTGGTCTGAAAGAAATATGGCAGGCAAAACAATCCTGGGCCAGTACTCAACTATGAAGTGGTCATCAGGTGGTCCCCAATCCTC TTAT |
| rs76 4528 9 | AAGTCACTTGCTTTTTGTTTTTTTTCAGTCTCCAGATTGAATAGGCAGACTGCCCAAATCTCCCTTTCTTTCATTTTTTCACTCTCTCTGTCAGAAGAAG GGGGCATTATAGGACAGTATTATTAGGCTAGATCTTTGCCTTGTTTTAGTGTCTTATCCACTGGCCCCTTCATAGGGTTGGGAAATGGTTTCATAAGAT[ A/G]GTAGAAAGGGTGGCATAGGTACTTGCAGTATGGCTAACAGTTGTTCACCATGTAGCTTTAGATATCTTAGACCCAGTTCCCAGCTGTGTCTGCCT TAGTCACTCTGGTCCTCGTCTTCCTCAGTATCTCCTCTATGAGGTNGGCATCCATGCAATTCACTCCAATTGCTTGTACATTCTTACATTAAGTTTTGGG GGATC |
| rs60 3571 2 | TGCCCGGCCTGTTCATCTGTTTTCACACAACTAAAGTTGCTCAGTTGTTGTGCTGGAAAGGACTTCCCTGCCCAAAATATACCAGCAAGACCATCATG CAACCGGAGCAGGCTGTGCCACAGACCCTGGCATGCAGAGGTGTTGTGTAAACACAGAAGGGATCTGGGCAAATGCATGCCTTCATTTATAACAGAT AAAAA[G/T]AAAGAAGTGTGCCAGACAGTGAGGCACTGACGCTTCACAGGAGGGTTCCGAGAGCTTTCCTCCCTGCCTGAAGTTCCTTCTCAACCCAA GTAGCCACCTTGTCCTCGTGTTTGTAGTTCCCCTCTCAATCCAAGCACTATCCTTGGCCTGGGGGGGTCATCATCACCACCAGCAAGGGTCAGCACT GCAGNGTGACAATC |
| rs73 0706 4 | TTGAGAAGAAAAAGAGCAGTTCTTTTGGGGGAATTTAGTTTTGGTCATGCTAATTTAGATACACATTTCTGATAAACAATTAGGAGTATAANTTTAANGT TTAGCNGTTAGCTAATTAGATATGGGAATAATGTTTTACACTTTGGCAGCAGATCCACAGAGACAACATAAGTCATGGTAATACTTAAGCAAGGGTGAT A[A/C]TCATAAGGGATCAAACCATTTAGGTTGAAAATAGGGCAAGAGTCACAAATCAGGTAACTTCAGTGGCCACCAGGTAACAACTATGTGGCGCTAT CTGGTTAAGAAAAGAGGGGCCCTTAATAGAAAGGCGTATACATCCCGTTTAAGGCATTTAAATCNATTTAAAATATATAAATAAATAAAAACAATGCCCA TAATAA |
| rs22 7010 1 | ACNAAGGGGCAGACACTGAGACTTCAGCAAGTTAGGAGTTTGTCAGTAAATTGGAACCAGAGTTGTTTCTTTCTGGGCTATGCAGTTGCTAGATTGCT CTCTGCTTTAGCCTTGCTGTCTAATTGTCATATGTAGGAATCATGCAACTGCAGAACTGTGTACCCTGTCTCTTAAAGCAGCTCTGTAGTGGTCATTCT GAG[C/T]GAATTAATTTAGGATTAGCTCAGAAATAACTAATGCCTCCNGGGTAGCCAGAATTGCAGTCTGCCTATTAACATTTGCTTTGTTCCATTGCTT TAGAGTCATATTCAATTTATCAATTTCCCTCTTCCAGTTTTTGGCATGGTATCCTTTTGTCCCTTTCTCCTGTATCATAACTCTCAGGGAGCCTAGCTTG CTATGTG |
| rs77 8866 8 | CTTTTTTTTTTTTTTTAAGAGATAAGATCTCGTTATATTGCCCAGGCTGGTCTCAAATTCCTGGGCTCGAGTGAACCTCCTGAGTAGTTGGGACTACTAC TCACCACACATGGCAATAAAGATTCTAAAGTTAGAAGGACAAAGTGATGTTACCTAAATTTGCCTGTGCTTCTGATATGACAAATAATCCGCCACTGGT G[A/C]ATGGCCCATAGAGCTCATCTTCATCTTTGGATGAATACGAGTGGACCTGTCATACTCCTCAGCCCTATGACATGTAAAGCACAAAGCCAGGAAT GAGGTCATTGTCTTATCACTTACCCCCAAAAGCTCTCAATGGCTCAACTTACCTAAGTCTTCCTCCTTTATTCTCTTCCTTCATATCAAAACTTCTGCAA ATTTTT |
| rs18 9187 7 | ACCCAGAGCCAAGGGTAAAGGTCTGGGCTTGTTATAATTTATCACCCATCTCAAATAATTGGCTATCATCAAGAATTAGATTCATGCAAGGAATCACT TAAAGATTCATTTGTCTGTCACATTAAAAACACTAACCTATCTTTTTCTTCTTCCTTAGCAGATTTTTCTTCGAGGAGCACAAGGGTGGGTCTGGTGGAA C[C/T]CTTCAGGAGCTAGGGTGTCGGAGGTGGGGGACTAGCCCTGGTCCCACTGTCCTTCCTCCCCTCCTCACTCCCCAAGCTGCCCCTCACCCCCT CTCTTCTCCTTGGGAGCAATCCCCCATCTCCATGCTTGTGACACACACTGAGCTTACTTCTGAGCACTCAGTTAATAAAGGCCTGTACATTGAAAAATA TTTTTAAAA |
| rs73 5073 1 | TCAGCCTTTCTAACCTCACAACTTACTTCCAGACCACAGCATTAATCCATGCCCTTTATGCTCCAGTAAAATATTTTAAAGTCCCAAAAAGTCTTCTCTG ACCCCTGTNTCCCCCAGGATTTGATATGTATCCTTTGCACCTCTTGTGATATTTACCTGAACATAGCACTTATTGAAATTGTTTAAAGTTTTTCTACTCA[ A/G]TGAACTGTAAACTCATTAAGAGAAGGAACGATGTGCTTTTGACTGTTGTTTCCCAAGCACCTCTCAAGGCAGTTGGCACATTAACGTGATCAACCA ATATTAGTTGAGTAAATGAAATGAGTCCCGGGGGACTCACACTAGCTTGAGGGTCATGGGCCAATTCCAAACTCACTGTCTGTAGGGGGATAATGAGT CTGATG |
| rs47 9506 9 | CTGGGAGCTATTTAAAAATGTAGAATCTCGGGCCCCATCCTAGACCTTCTGAATCAGAATTTTAGCAATATTTCCATGTGCTTCATATGCACATTATATG TTACAGTTTGAGAGGCATTGTTCTAGAAGGAGCTCAAAGACTCAGGGATGTGAGAATATTGAAATGGAGCTACTATGTGCAACACGCTTCGACACTAC CC[A/C]CCTAGCCAGAATCCCCGAGCAAGCCCAGGGGATGCACCTATCACCAAGGAATTAAGACATGCATTGGAGAGGAAGGTACTAGCAGTGCTGG AGTGAGGGGGGAGGCAAGAAGACACNCAGGATACAACATTTAACCNGGCACCCACCCTCCGGTACTGATCCTGAATGCATGAGCCTGAAAGTGAAT GCCTCCTTTAATA |
| rs45 4421 4 | TAAAAATGCATTAACGTCTTTTTATATCCATCAAGGGAAGGATGAAATGTTGAATTTGAAGACTAATTCAGTAAGAAGTCCTAGGGGTTTAACTGTACAT ACTACCTGAACTGGCTTTTCTGAGAGATGAATCAATAATGAAACATGTCTGTTTTAAAAACTACCACATGTGACTCCTATTTTTGTTAGCTGAAAGCTGC[ A/T]ATACGGAGTATTACAGNAATGTGAAGGTGACTAGCTTGAAGGTAGGGTAACTAGAGAGCCAGAAAAGTTTTGTTTTAAACTTGATTTAATGCGTTT TTATTTTTTCTTATACAAAATAGAGATAATGTTGCTAACTTCATGGAATATTTGAGGAAATGATATGAAAGTGTCTGGACGTGCAGTAACCTCATGGNTT CTTC |
| rs58 4762 | GATGTTATTGAAAAAGATTGGGAAGATTATGGGTAGTAATAGAGATAATTGCAAAGGAGAAACAAGAAAAAAAATTAATGAATCTTCCATCTCAGGGAG AAATAAATGGCTTTATTATGGGGTCAGGAGGGATGATCCTAAAATTCCCTGTCCAAGAGAAAATGCATAGTGGTAGTTTGGGAGTTGGAGAATTTGGC ANC[A/C]ACAACATCCATGAATCAAAACAGCTTTTAAGGGACAACCTCAGGGGATTGCATTTCTATAAAAGGAATGAGATGTTTTTCTTTATGCTGCTGA AAATGCAGAATCAGAGCCGACTAGAAGCACAGCTAGCTCCTTTGTCATTATATGCAAAGGACAAGAAGAAACATGATGTTCCACTATAATAGGGCCAT AGGCAAAAA |
| rs76 1453 | CAAGTAAGTAAGGATGTGTATAAAGACCCTAGCCCATAAATGGTCATCACTAAATAGGTAGTCATTATTTATCATTGGNATCTTTATTTGTGAGCTGCAA TGGATATGTAAATAAATATAGGTATGTATGCATGATGCACAGGTATCCACCTGTGTATAGCAGTTGCACTATTTCCCTTGCCTAAGGAGAAACTGAAAT G[A/G]TAGTTTAGGCCAAGAGATGATTCAGACCCATGTGTATGACTAATTACAGTACTTAACAAGAAATTGTAATAATATCCATTGGTATAGCTAGAGGT ATGAGTTTCTGCTAACTCAGGATCTAGTGTCACCTTCAACAAACTCCCCTTATTCTGAATGTATCTAGAAGCTCTAAATGCTTTGCTGGAAACTGAAAG GGTAGA |
| rs12 5833 95 | AAAAAATAAGATGATTGGAGGACAATCAGTAGTGTCTGTGACATCTACAACGGAGCAGTTGGAGAGGAGTAGGCAAATCCCTATCTACTGGCAAGAAA GATGTCCAAGAACATTTCCAAGTGAGGACGCAGGTTGTGAGGTCATATGTCCATTATCACCCATCTAAGGTTCTGAAAACANACATTTATCAAGGCATG GAG[A/G]AAGTTCTGGAAGTTACACTCTTTTTCTCTCAGAGAAAGCTGGAATTGGGGTTTGGAAAGAAGAGGAAATATGTCTATTTACTCAACATACTTC TGAATTCTTTAATTGTTGAAATCCTCTAAGAACTTGTATTCTGCGGTCATTTTTAAATACAAGGGAAAAACAAACAAACAACCCAGGCTGTAAGGAAGAA AGTGATA |
| rs20 7043 9 | TCAGCTGCTTGGGAGGCTGACATGGAGGATCCCTTGAGCCCAGGAGTTTGAGGTTAGAGTGAGCTGTGGTCGTGCCTTTGCATTCCAGCCTGGGC AACAACGAGAGACCCTGTTTCAAAACCAAAAGGGAGAATGGATTACCACAGGGGCTCGACATGATCAAGTCAGTTTAGTAGGAACGACATGTGAGGC ATGGCCC[G/T]GTCACAGGCATGAAGGTTCAGAATATAGCCTGGAGTACACATGTATCCCTCTGGGCCAGGCAGAGGCTTCACATCTTGGTGAAAGGC CTGGTCGCACTTTCTCTGGGTTTCAGATGGACTGTTAATGCAAAGTTGTTTTTGTGTTTTTGCAAATCCCAGGAGCTGGTGGATGGGGATGGTGTGAC CCTGTTGGAACCTGC |
| rs80 9518 6 | ACTTAATACTGGGGGCAAGANGGTTCTTGGCTGGGCACACAATGCTATGGAACTTAAAAACAAATGGACTAGCTGCAGGTTTAAATAATGCTACAGT CCTTAGACACTGTATATTATTCATAGCTCGTTGAACTCTATTATCTCATGTGTTCCTCTCAGTGGCTCTGTGAATTACCCAGGACAGGAAAGAAATGATA GA[A/G]TGTGAAGATAGAAGAGTCAGTAGAGGTCAGAACCCTTCCCCAGCCCCTCAGCTTTTTTTTTTTTTTTTTTTTTTTTTTGANATGAAGTCTGGCTAT GTCACTCTGAAGTGTCGTGGTGCGATCTGGGCTCACTGCAACCTCCGCCCCCTGGGTTACAGCGATTCTCCTGCCTCAGCCTCCCGANTAGCTGGG ACTACAGGT |
| rs58 3338 | TACTTTCCTATCATCATCTACCTTTGGGATATTATGTCTTTCTTAGTAAAGTGTCGCTACAAGACTTTTGTCCATTTTTTATTGAAATGTCTGTCTTTTAAA CATTGATTTTTCTTTAAATATTTTGGATACAATTCCTATGTCAGGATTATACATTGCANATATACTCTCTAACTTCTACCCTGTGCCTTGCCTTTTTT[A/G]T TCTCAAGTCATCTGTAATTTGCATTCTTCCGTCTTTTTTCCCGTCAATGTTATCAGGAATTGTATACCTGGAGAGTATAAACCATATACTCTCTAAGCAC ATTTAGAGCTACTTTCTGTAGATCTTGATATGACATGTTATCATTATCATTCAGTTCACAATATTTTCCTCTAATTTCTTTTTTTAAATTTTATTATTA |
| rs22 2824 6 | TTTGCCGAGTGTCCCTTCCTGAGTTCCAGCAGTTCCTTCTTGACTACCAGGGGGTATGGNTGGGCTGACATTGGCCCAGGCTGGTAGGTTGTGGGG GGCTGGGCTCATCCCTGACTGGAGGCTTCTCTCATCCCCTGCCNTCCCTACCCCATCAGGAGCTGTGGGCTGTTGATCGCCTCCAGGTGCAGGAGT TCATGCTC[A/G]GCTTCCTCCGAGACCCCTTACGAGAGATCGAGGAGCCATACTTCTTCCTGGATGAGGTGAGCCCGATGTTTCACCCATTTTTTGTCA AGAGAATGAGTAGGGGTGACCAGGACCCCACCCGGGCTCCAGGAGCTAGACGCTCCTTAGGGATGCCACCTTGTTTCTACCTACTNTGCACCTTGC CCACCCCCAGTTGGGAC |
| rs26 6453 7 | CTGTGTCTATGAATATGACTATGAACTCTGAACTATTTATCCATTGGAAGGTAAAGGAAAGGTCCTACATTGTGGGAGGAAGAACTGATGAGAACCCC ATCTTGCTTGCTGCTTGCTTGGTGGTGGGCAGGCCGAGGGTAGCGGCAGGCTCTGGGCTGTCTGCGAGGATTCTGGAAGCTCTCCCAGGTGCCCA GCAGCC[A/G]GGCANGGGAACGGCATGTGGCAGCAGAGAGTCGGGTTTGGCTCTTCCACGTGGCAGGCGGTTTCCCAGACTGGCCAGTCCTTCACA TTAATCAGATCAAATTCAGTCTGTTTCTGAGAAGAAAACACATGCCTGTCACTCTACGGCAGCTGCCACCACCTGCCCCCCAGGCAGCCTGGTCCTTG CTATACCAGGGTGGCA |
| rs60 7231 7 | AAAGTAATCTTCAGTTCTTCCTCACCCATTACTTATATATACAATATATTATTATAAAGGAGACAACACAGAATATAGAAGGCAGATTTCTAAGGTCCCCT ATTAGAGGTTTATAATTCTTTTATAAAATGTTATCTTTTCTCTTCCTCAACACAACAAACGAACCCATTAAAGTCAAAACTGANAACTCTGGCCAGGA[G/ T]TCTGTTATATGTAAGATGCCTCCTTGTTGATGTTGCTATAGTCCTAATTAACAGTCGACAGGACATTTCAAGACTTCTCACTCTTATCACCAGTCCTG GAAAAGTTGTCTTTTTCTATAACTGAGGACATGGAATCCCATCTCTAGGGCCACACAGGATAAACCAAAGCCAACCTTGACCTTAAGGAAATTTGTAAG GAC |
| rs60 7234 3 | CAACCAACTGGAGAGGTTTTTAAACTCCCAGTATCCGGGGCATCAGTATTCGTCTAGGGCACAGTGATTCTAACACACAGTCAANGTTGAGAACCACT TATTTAATTCATCCTGTAAATCCTCACAATAGAATCCTGCAAGGTAGCTTTTATCACCACCTTTGCCGATGAAATTGANGCTCAAAGAGGTAAATTGATT TC[A/G]GCATCTGTAGCCCCAACCTGTGACCTCTCCTTCCTTCCTTCTCGGGCTCCCTTCAGGGATTTCCTGATGTTACAGAAAATGGTATGTATGACG CTGCCCTCCTGGCCACCTTGACATAGCTCTGCTCCCAACAGGGCTCTTCTGAGATAGCAGCCAGACACTACCTGGGATCAGGACTGATACTAAATGA CTTCTGCCAA |
| rs45 1802 3 | TGTGAGGCTAAAGGCTCCCACGCAGCATGGGGCATGGGGAGCCTGAATGTAAGCCCCAGGGCAGGAAGTTTTGCCTATTTGCTCACCGTGTAATCT CCAGCATCTAGAATAGCATANTAGGCCTTTGGTAAATATCTGTTGAATGGAAGGACTAAAATTAGTTCTGGAAGTGTCTGGGGATGGGGCTTAAGTTA CCTCTG[A/C]AGGGTCTCCAAGGCTAAATCCTTTTCCTGAGTTAGAAAAACAGGGCTGTTCTCACTGAAGTTTTGGCCTTCAGAGTGTGATGAGGAGTG CANAGGGGCACGGGGCAGCTTCAAGGACAGGGCTGGGACCACAGAGGGTGAGAAAAGGGCACAAGGGAGGAGGGGTGCTCCCAAGATGGCAGCC CCTAGAGATCTGGGCTG |
| rs71 4633 2 | TTGGTTCTTTCCCTACCCAGCAGCCCTATACCTTGCCTTNATGCTATGTATAGCTGGAGTTTATCTCATACCATGAATAGCCAAGCATCACATGGAATA TATCCTAAGAAATATTCCATAAAAATGAACTTGATGGGATCATGACAGAGATGGCAGATCTACAAATTTTAAGAATTGGAGACTATCTCATAGACTTTGT T[A/G]AGATCACAATCAGTTCTCAACTTCAATGTGGTACATTTACAAGCATTGCAAACAGTGTGAGATCCCAGCACAGATGTCAAACCAGAAATTATGTG ACTTGAATGGCTTGGAATAATGGCCCATTTGGGGCATCCAGCACTTCCACTGTTGGGTAAGTACTGTTGTCAACAGTCACACTGTCCAAGTGGATGAT GCACCCT |
| rs20 8813 8 | GTGTCACAGACAGCCCCCACCCCAAGCTCCCTTCCAGGTATCCCCATAAATCATTTGGCACACTCCCTCTTGGAATTGCAATTTCCATCTTCCACTTA TCCCTAAGGAGCTGGCCCTGTGGAGAGGTGTGTTGGTTGTTTTGTTTTTGCCAAAGGCCCTCGCTCTAGGTGCTCCTGTAAAGGTACCTTGGCCCCT GATA[C/T]GGGATGAATGGATGAACGAAGCCCCAGATGCTCCAGTGCCAAGGAGGTCAGACCGGGAACAGGCCCCCACTCCCACACCCTGGGATCG GAGCTTGCTTGGTTCTTGCCATCTTGCAGGATGACTTCGAGTCTGGAGGGAGATACACAGTGCCTCTCCATCCCTGGGGTGGGGGGAAGGGATTCT GCTGGGACTCTTGAGT |
| rs48 1062 2 | TAAAAACAAATCAAGAGTTGGGCCAGCTGCATGGAAGGACCCAAGAGGATTATTTAAATGGCATGCCAAATTTCACCCATGCCACCCAATGTCCTCAC CAAACAGGCAACCCAACGGAGCTGTTGTCCTCTGGATAGCCCCAGATGGGTCCCTAACTCTAGCTGCTTTCTCACCTGAAGATGCAGAGCACCCAC TGACA[C/T]TCAGAGGAGCTTGCTCTCCAGAGGCCCAGCCATCCCCCACTTCTTTACAAATGCCCTCCTAGCTTTACTTACTTATTCAACAAACATTCCA GAGCATCTACCANGTGCACAGACCAAGTACCATAGAGAAGGCAAATCCTCATAAAACGGCCTCCCTGCCCTCAAGGAGCTTCCAGTCTAGATTGGGA GGGACACCCCCA |
| rs28 3917 8 | CACCCTATAAATATCAGTTGACATACAGAGTAGCCACCACGTCCCAATCTAACTCCTGACTTTTCCCCTTATATTGTATGTGTGTGTGTGTGTGTGTGT AATCAAGTATTTCTGAAATGCAGTATAATCCAATTAGTCTATTTTAGGTATTCATTGCTATAACATTTTCCCTCATCCTTTTACCACTGAATACAGAAACT[ A/G]ATAGCTCATATATTTTGAATTTGTTAAAAGACTCTTCCCCGTGTGTGTGGATTTTGCCTGTAGGAACACAGGACTCACCGCTGTAGATACTTGCAG AAGCACTGAAGCTCCTGGAGGGTCTCCTTTNCAGTCTGGAAGATTTCCTCCACGAGAAACAAGTCCACTAAGTGGGCACAGACATCCTCACAGCAAC GGGCCAC |
| rs12 5896 23 | Seq ID: AATCGTAGCAACAAAACAAAGGCAATTCCAAATTCAAAAAAATCATTTTTAAATTCTGCGTCTCATCATGCATTCTGATTATGTATTAGTAAAAATATGAA ATCCCACCTGGGCTTAATGCTAACTAAAATTTTAAATATTATCACTACTTTACATTTTTTAAAAAGAATAAAACATTTTAAAAAGCATTATGTAAGAAA[A/C] GCATGGAGTCAGATTTGAGTTGGGAACTGTTATTATGCTGGTTCTTTAGGCTGTTGTTTTCTAAGGGAACTAAANAACTCAATTTTCTTATCCTCTTTGG GTGAGTGGTGCTGTAAGTTTGAAGACTAATGTAAGGCTCTAGAGTCAGAAAGTATTTTATTCCATACCTAATTTTTCCACTATGTTTTGTTATTCATTTT |
| rs24 1466 0 | [?] TAGTTTTATTTTCAAAACAGGAACATCCGGCAGAATCTCAGCTTTCATTTTGAATTCAGNGTTCCCGCTTCATCCATTATTCATACACACTGCACTTTCC TGGTTTGTGATTTTACTAAATTTCTTCATTTGAATTATGGCCTTGTGGTCAACAGCGGCAATATATTATTCTTATATAATTTTTAAATACAAAGGCAAA[C /T]GGCACGGAACAATTAGAATCGATGGCTTGGGAGTCTGGCACTCAGATGTCATAGGCAAATGCTGTTTTCTTGACTTTAACCTATGGGTTCCACTTCA TGTGGGTTGTATTTTTCATACATCAGGAGTTTTTATAAATTTCCTATTCAATCCAGCAGCCCTACTTTGTTCGCAAAATATAAGCAAAACAAATATGTCAG GG [?] |
| rs16 3053 5 | TGACACTGAAAAACCCACTCAGTCACAGGGTTAAGAAACTAATAAAATTACTATTTTTCAAGTCCTTAGTACATATTTAGCATGATTCTAGGCATGGCAC NGGCAGAAGAAAAGTATAAAATATNGCTTCTACATATAACTTACNGTTTTACTAAGGAGATGATTGGGTAAAGACAGTCCATTCAAATGCCATAATCAA C[A/G]GTGCATAGTCAAGGGCCAGCCACCAAGCAGCTGGGAGAGAGAACATTGAGCTAATATTTGGAATAAACCAGGTTTGCCAGTCAATTATCAGAA GTACCTGGTTGGTAAGGAGTCCTGCAGAGAAGAGAAGTTGTTTTCTGACAGCCTTAAGCAGTTGATCATGCCTAAAGACAACATTATCAATGTCCACT AGGCTCTTT |
| rs68 6884 6 | AAAGAAATATGGATTGGCTATCAAGGGACTTCCACCACTAATCAATATTGGACAAAGATCATCTCACTTCTCTGTGTGTCTATGTCTTCACTTTTAAAGT GAGAGAGACAGGCTGAGTGTTTATACTAGAAAAAGCTTTAATTTCAAGGAAATCTGACTAGAATCCCAACATGGAAAAACAGATGAAAACGGGTTCATT C[A/G]TTGAAGCAGAGATGGGGGCCGGAAGGAACGTAAGGATGAAATGGAGGGATGGGGGAAATGGAGAGGTCAGTGAAGGTNTGACAAGTAGAAG CAGCAGTGGCAGTGACATAGTTAAACTGGGAAACAAACAGGATGTGGCAACTGGAAGGCAGGCTGTCTGAACTAGGGATTTTGGAAGCAGACCGGT GTTGCATCTTAAG |
| rs79 0146 3 | TTAATTTATCTCTCATTTGTGAACACTGTAATCATTTCTCTATCCCACATTTATTTAGCTCTCAAAGCTATGCTATTTTATTGTACAATGACTAAACTACTC AACATGAGAGATTTGGGAGGATCTTGTGGTCAGCTTATTATTCTTTTCATTACCACAACAACAGCACTACAAACTCTTTTTCTAAGTGTTTCTCCAT[C/T ]TATGATCTCACTTTTGTGGCTAGACCTCACCTCTAGCCCCTGGGAATTTCTTCTGTGAACGCAGCAGGATGACATCCCTCAAGTCAAATTCCAAAGCC TTAAAGTAGATCAGCAGATCAGGGCCCCCTAATGACTGATTACACACAGCTCTGACCCCACACTTAGCCCCGAGCTTGTTGTTTTCTGCAGCCTTTTAT CCC |
| rs35 6999 | AGGCCTAANGCCTGACTGACAAGCAGCTGTTTGAAGGACACTGTCCATCCAAACATGGATTGTACNGTGGACATACTGTCTTCTCTGGAGTTACAGAG GGAGTTGGGTTCATTGCCACCCTCATTAGGTCACAGAAATCCCAATAAGATAAAATGGAAGATGCTACCCTGCCTGAAAGTGCCCAGCATGGTGCAG GATGC[C/T]GATGGGTACTCAATAAATGGACTGCAGGTTGACTTAGTCCCCCTTCCCAAGTGGGCAGTTTAGATTCTAAGTACTTTCCAGTTGGCTGAG CAGTTTTTCCCAAGAAGTAGGCTCCTTACTGTTCCTGATTTCTTTTGTCTATCCCATCAGAAGCAGGACAATCAAGGCAGGTCTAAGAGAGAACTCATG GCAGGAGCAGA |
| rs17 1750 96 | TTGTAACAGAAATAAAATGAATGTCATTGTTACATATTTTTTTGGTCCTTGTTAGTTCAGCAAACTTCTCGTTTCCTCCTCCTGCTATGCTGTGAGATGCA TATGATGTCTATGGAAGAGCGGGAATAAAATGGTTAAATTACACCAACCCCACTCACCTCTGCTCTTTCCACTGACAGTGAACGAAACCATAGTAACCC [C/T]ATAAGAAGGCATCTGTGAAGAAAGCTTTCCTGTAAATACACCCAGTAAGTTAAGAATCTGTGTTACAAATGCAGAGACACAGAATTCTCTGAAATC ACTAAAAAGAAACCTGACCAAAAATGAATGAATGAATGAATGAGCCAACAACAAACAAAGTGAAGGGAGCTGACAGTCCTAGTACCTTGCAGTTTGTG ACAACT |
| rs49 3951 7 | TGCTCTCCAGCCTGGGGGACAGAGTGAGACTCTGTCTCAAAAAAAAAAAAAAAAAGAAAAGAAGGTCCAAGGGCTGCNGGGAAAAAGCTTCCATGA AGCTGGCAGGAGAAAATCATGCCACCAGCCTCCTAGAAGATTTGTGACTCAACTACTTAACTCAGAGATTTGCTACCCAGCCTCAGAACTGCCTCCTC AATTC[C/T]TCCAAGGGCAGAGGACGTTTCAGATCACCAGATATTCACCTAAAACACTCTCCATCCCTCAGTGAAATGTAAGAGATCTTGATCAGCAGG CTCACTCATCTGACAATATCTACTTCCACAACTGTTTTTTTTTTCTTTGTAAGAGGAAAAAGTAAATGCAGTCCGAAAGGATCTGTAGCCACACCCTAGA AAAGGTCCTG |
| rs10 3744 8 | GAGCCCTGGCATGTGAGAGTTTCATAAGGTACTCTTGACCAACCTTATGTGTTGAAAAACAGCCTTGTCCAAGAGTCAGGGGACTCTTCCAGAACAT TCAATTCACCTTTGCCCCAAACAGGCCTACCAGGGCTAAAACAGGTAGTTGAATGTGTCTCATCATTAGCTATTTGGGCAGGTGGATGTTGATACAGG TTAT[C/T]CTTAGGCATCAGTGCCCCATGGCTAATGCTGTAGGCAGTGGATGTTTTTGGAAGTAGCCAAGCAGGATTGCTGCTTGCCCTTGAGTGCTCC ATGCCGTCTGCCNAGCTCCCATCACTTTAACTAGGAGAACCGGCCTCCAGGATCCCTCCCTTTGGTTCTAACATAGGCCTCAGATCAGATCCAGTGTC TGTGGTTTCAG |
| rs22 4405 7 | TTGCCCCCCATAGTTTCTGAGAGGGCTCTTCCTGGCCCACTGCTTTAGTTACCACTCATATTCACATCTCTCNGACTTCTCTCCCACGCTTTTTCTCAA GATATGTGAGATAAAACTCAACATTTTCCTTTGTGAACATGCCCCTCTGCCTGTATGCCTCATCTTATTCGATGCTAACTTCTCTACCCAGTCACCGAA GA[C/T]AAAAATGCAGGCATCATAATAATCTTTCTTCTTTAATTATTCATACCCTCTCCTAGTCTTTATCATAGTGAATTGATTAAATAGTCATGCTCATTT CCTACTAAATATTTTTTGGCATCTCTCCATCCTCTCTCCTCTCACTCATTAGTTTACACTTTTTCACCTTCTGCTAAAACCATTGCAAGGCCTTCTTAACT GAC |
| rs10 9970 44 | TGTACCAAAAGTACCCTGTCTTTTGCCCTCTCTTCTGAGAGAAACATTGCATTTTTCTGCAATATCTCTTCCTCGTGTCCTCTATCCTACATTTATAGAG TCTCAAATATGCTCAGCCTCAAAATTAAGATGCTTTGAGCTTCCAGAAAACACTACACAGATAAAGGTGGCTTTCAAAAGAGATGTTTCTTATTGTCTAC [A/G]TAATCATGCAGAGTTAAGCAGTCACTGCAACCTTTCATCATTTTCTACAATAAGGCCAACTTTCCAGATAACCACTTAGTGAAACAAGAATCCTGT ATTTCTATGGTGGAGCTACTGCGGGTCTCATCCAACCTCCTGCCATGTTTGGGCCACTGACTGTTTGGGCACTTACGTCCAGCTTGCGAGGATGGAG CCATGGG |
| rs99 4932 4 | GTGGAGTTTCTCTGTTTTCCGAGACGGCTTACTCGGCGCCTAGTGAGNCGGTGCTTCTGTTTTTGTCTCTACTCCGTGTTCTGAGTGCACCCATTTAT NCCCAGATAGCGTTTATACTTACAGAGGAGACAGGCGAACACTAAAACACAGCAGAAATATGACGAAACACCCTGCAACCTTGTTTCTTTTGAGTTCC TATC[A/G]CTCACTTCAATCTATTCTAAAAATAAAGGACTTGTCAGGCCTAATGATAAGGGAGAGTTTTAGGGAAATAAAGAGAGCTGAACTGACACAGA CATAATAATGTCAAGATATAAATCACAGAGTCCTTCTGCCATTTTCATTATCTGCCTCGGAGGATTACGGTGCTTGCTATAAATGAGACGCTATGGGGA CTCTGCCCN |
| rs61 8670 | ATTAAATACCAGTTTATGAATATTGCACCNTTAGGGCATTATCTTTTGCCAGCTTTCCTGCTCACCACTNCCTTGTCCTTTATTCCGAATGTGGACACAT GGGTAGGAGGCCTGCAAACCCGTGATGACATTTCTCAGTGTGTGGGGCCATCTTGGATCCTGGCAGTGGATGAGAAAGGCGTGAAATGGCAAGGAA CGGC[A/G]ATGGTCAACTGAACAGTGCATGGTTGTCCGTGGGTTGAGGAAACGTGATGTCCGTGGATGTTGTTTGCTCTTTTCCAAAGATGCAAATGC NGCTGTGAATACGGAACCACAATTTGACCTAAAGACATGTCTGCACTCACATGCCAAAATAGTTTGTCATTTGCTGTCCTTTGATAATTCCATGTAAAA GACTATCTCAC |
| rs47 9915 9 | TTTATTCAAACCAGAGGACAAGGAAAACTACTGGATGAACACGGTTGTCTTGGGAAGAGACCTGCAGGAAGCTGCCCCAGCAGAGGTAAGCCCGGTA CTAACAGCTTGATTCTCATAAAAAGGTGGCAATAATCGTGAAAGTGTGAGAAGGAATGTGGCGTTATCCATTTAATCCCAGAACTGAGGCTGGGAGTT GTCTC[A/G]CAGTGACGTGGGCCAGGCCTCTCCCCAGCAAGGGCGATTAAAGTGAAACCACTTAGGACATTTGGTCTATTTCAGATCCCTGCCGGTAA CCTTTTCCTTCTTTTCACCAACTCTTTTCAAACTTAGGTTTTTTTTTCCCTTCTGTCTTTGTATTTTCCCGTAACATAGCCCTCGGGGATGTTTACTTAGA TTTCCCTGTT |
| rs10 4908 32 | AAAAATTTCGGCCAAAGACAGACTTCAAATAAGAGGTCATTGTTAAGGGTTTTAAATTCAAGGAGACAAAGTGGCACGGTAACACACATACACAATTTA ATCGGAAAAGAAATTACCTCATAGCCCAGTAAATGTCCATTGCTCAACTTCCAAGGAATGGTGTTCCATGAAACCTCAATTTCTGAGGAAGATAGGCTA TT[G/T]GCAGAGACTTGAGATGGGGCCACTGTAGGCTCTGTTCGGAAACAGAAATTGAAATATGATGATAATTTTGGCAGTTAGTTTAAAACACAGATTA ATGTTCTTATTTTTATAAAAGCATTTTAACGTATTTGAAAATTGTATGAAATATTCTGGCACCATATGTTGAAAATAGAGACACTGGTCCATTTCCGTTTC TTAGG |
| rs15 4538 4 | TTTAGGGAGCAAACCAAGGTGGTATAAGAGAGTAGATTGCTACAATGGAATGTATGGAAATATAAACAAGTTANAAACCATACATGACTGCAGTCAAAG ATTTCTGTAGCCAAATAAACAACAAGCTTCCTCCTGGGCATTTATGGTTTCAAAAATAAATAAGGGCAGAGATTACAGTCTCAGAGTCTATTTACTTGCA C[A/C]AGGGTGACACTTCTTTTTATATCCACTGCTCAGCACCAGCTCAACTCTTTTCTCTGGTTCCAACCCCTTTGCAATTATCTGCCTGAGAAGAAGAG GGACACATGTGGGTATCTAGTGGGAGATAAATGCAGCATGACAAGCGGATTACCATGGTTGAAACACTTTTCTGAACACACTGGAGCTTAAAAAGCCT AAAGCTN |
| rs64 9867 | TGTGGGGTGTCCCTGTGTCTCCGGGGTGACTTGGCCTCCAGAGAACCCCTGGCACAACCTCCAGCTGAAAACAGGAACAAAGAAAGTGAGCTTAA CATTCCGGCTGTTTCAGACAATGTGTTTCTCTTTGTGGGGGCAAGGAGGGCAGGGAGACACCTGAAACCATTTTTATTCATCAGCTCAAGTTGGTGGG TGGCAG[A/G]GTCATGTTGAGGGAAAAAAGCCAAACTCTGTAAAATATTTAGAGAGGTTTCTTCTGAGCCAAATATGAGTGGTCAAGGCCCGAGGCAC AGTCTCAAGAGGTCCTGAGAACATGCGCCCAGGTTGGCTTCAGACATTTAGGGGGACAAAAGTTACAGGCAGACGCCAATCAGTACATGTGAGGTGT GCGTGGGGTTGGTCT |
| rs22 7910 3 | ACTTTTCATTATTATTTTTTGTAAATTATGCATTTTCACTTGTAGAAATCTCTTTCCTTGTGGAGACTCAATGGTTTGCATTATTGATGATCGAGAAGATGT CTGGAAGTTTGCCCCCAATCTGATAACTGTGAAGAAATATGTATACTTCCAGGGCACNGGTGATATGAATGCGCCCCCTGGGTCCCGAGAATCTCAG A[C/T]GAGAAAGAAAGGTGGGTAACCTCCTTCCTGATTCTCTAGAAGAATTCACATTTGCTTATTGNTTAGCTCTTCTTATTTCTTATCTCTGTTTTGACT GCTATAAATTCAAGATACACTTTTTTTATTTGTGTTTCAGTAGAGATTATTGGATTTATTTATAGAGTACTGAAAAACAGGATATTAGGTTGTTTCAATTTG GG |
| rs52 3386 | GCTGAGGCCAAAACAGGCAATTTCCCTTACTCATGTCCTTGGCGTGAAGNGCCTCGTCCTTGTTCAGTCTTGCACCGAGAGGAGAGCTCTCTGAGCT CGTGGCCAGCAGGTTTCCTGCTGGAAGCCTTACCTGGATGCTGGGCAAAGCTGTCAGAATAAAGTTCAAGGTGGTCAGGGCTCTGCAGGGTCCCTC AAGGGAG[C/T]TTCCCTCATCTAAAAACATGATTCAACACAGTGAGCGATTTTCAGTTGTGGGGTTCCTAGCAGTGTCTAACAAGCCATTCTGGAGTCC ATCAACATTTTTTTTTTTTTTAATAAAAAAGGTGAGATGGAATTAAGGCTGGAAGTGGCGGACTTTGGGAGAAACATNCCAGGAGTCTGGCCTCCTGNG ATGAGCAGAGCTC |
| rs77 0493 9 | GAGGATGCAGAAGGAACATCTGGGCTCAGCTATTTCATGCTGTCGGCCATACACTTCCAAGTGGGAGGTGATGGCAGTTTGGTCAGCTGTTTGCCAA CCCAGGAGTTGGGTAAAGGTTGGTAAGAGATGTTATTAGTTTTAGCACCATTTCCTTCCAATAGTCTCATTTTAATACAATACAGGTTAGTAGTGACCA GAAA[A/C]TATTGCTACTGAATTGAAGGATGACTATACAAGGACTTATATCAGATTATTTGTTGCTTTTAGTTTCTAGTGTCCCATAAAGTATAGAAATGG AAATTGCTGACAAGAGAGTACCTTTGGTAGGCACAGTTCCTTTTCTCCCCCCACTGGAGAGGACGCTACAACCTTGCTATAGAGTGTCCTGTGACTGC CCAGTGATG |
| rs19 5843 8 | TATCACTCCAGATGTTCTGGACAGAACAAAGATAGGCTAGAATATGTGATGTAATGAATTAATGAATTTTTTTATACTTCATCAGCCAACATCAAGATAA ACTGTATTGAACTAGCATGTGTCCAGCTGAAGAGTGTTTAAACTGTTAGTGCAAAGCTGAAACTTCAATAAACGCTAGGAAAGGATCCAGGTTTTATTA C[C/T]GGTGATACAGTTTGAGTGGATAAATTCTAACCATTTTAGCTAAACCCTCCAGCTTCATATTAATCATGAAGTAGAATAAAGTGGGGAAAAAAAAC AACCATGGGGAATGTGAAAATAAGAGTTTAAAACTGAAGTATTACAAAGGTCAGCTTCTTTATGAGAAGACAAACATAGCTGCATTTTAATTAAATTATG AGATT |
| rs19 5842 1 | TTATCTTTCAGTAAATTCCTAATGAAATAACAAAGTGTATTGGGTTATCTCCAACTGCCGTACCATACTACTCTCTAAANATAGCAAGTCAAAAATGACT GACTTTGCCAATATCCTGAGATTCTACAGACATCTCAGTGAAGGCAGAGGATGGTGGGGAGAGTGGCATTTGGCTCAGCTTCAGAACTGGCACTGTC TCC[A/G]TGATAAAGGGATCCTGTTAACTTCTGTTTTCCACCTCCTGACAGTTGACATTTTAATGTGTGTCAGGAAAATCCATCAACATGCTACCTCTCT CAAACCCAGGATTTCGCNTTGGGATCAGTAGGAATCTGATCAGAGCTAACACAAGCCCTCCTTCTCATTAAAGCATACCCCATTCCCATTGTTGACTGT CCTAATTT |
| rs93 2373 7 | TATTCCTTCTCATCCAAAATTTGGTTATTTAAAATCTCACTATTTGAACTCATGAATCAACATGTTTGGATATGTTTCCACATAAAATAACTCACTACTTGC TATTTCAAGTTCAGGGTAAATTTTTTAAAAGTGTAAACGATATCCTACACCTCTTTATTCAAATTTACTATCTAAACTTGTGCTGTCCAAATTCAGGA[A/G] GTAGGTAGTTGTGGGTAGCAAGGAATACTTAAAGTGGATTGTAGCTCAAGTCATACAATAGCAGCATTTGTTTTCACGATCCATTAATATCATCTCTAG TTTACAACCATCAGGAGCCTTTTCATAGCAATGAACTTTCCTGTGATAACTTTGAAAAACAAAATCTAAATTTGTCATGGCCTACAATGCAATATATGAA A |
| rs12 1422 66 | TCTCTCCACCAAAAGGAACCAGGCCTCCTTGGAGAAATGGCTGATTCCAGGATTGGGGCAGGGAAATTACAAGATGAGTCTAGACTATCTATCTTTTT ATGGCCAAAAATAAGGAGGTGTTCAAGCAAGATGAGGACATGTTAAAAGGACACAAAAGGCAGCTTGAAGGGGCTCCCACTGGTCAAATCTGCAACA ATTCA[C/T]ACATCAAAAAAATGCCAGTAGTAATAAAGCATAATCTATTGAACTAAATAAGAAACCCATGAGTCCACATTGGTATAAATAAATGAATTATTN CATAAAGGGGGAGGAGAGAAACTTCCTTTGTGTATTAGAAAGCCTACTAATAAATGTAAAAGGAGTTATAGAGTTAGAAAATCACCATTTGGCATCAGT AATAATTAT |
| rs65 0049 7 | AGGGCTCCCGGAGGACAGGACCTTCCAGGGCCTCCCTGCCCCGAACCCATAGGTGCCAGGCTTCTCACTGCTCACGACCTCCGTGCCGGGCTCAT ACCTGCCGCGCCAGCCCCTCTTCAGCAGAGCACAAGTCTGGCAGCAGGAGGGCACCAGCCAGGGGCCCCGCGCCCCCGCCTCTGTACAGCAGGG TCACCGTGGGG[A/T]GACATGAGAACACCGACCCAGGGAATGGGCTCCTGCCACACCTGCCTGGGAACCTGGNCTGTGAGTCCTGCTCGCTGCACC CTCTAGCCTGACGTTGAAGTAACCTCGCCAGCGGGGCCCTCTGTGGCACTGGGCACTCAGGGAGAGCACGTGGCATTGGAGAAGGTATTCTGAGTC CTACAGAGAGGGACCTGCGGGGC |
| rs65 0049 8 | GGCTTCTCACTGCTCACGACCTCCGTGCCGGGCTCATACCTGCCGCGCCAGCCCCTCTTCAGCAGAGCACAAGTCTGGCAGCAGGAGGGCACCAG CCAGGGGCCCCGCGCCCCCGCCTCTGTACAGCAGGGTCACCGTGGGGNGACATGAGAACACCGACCCAGGGAATGGGCTCCTGCCACACCTGCCT GGGAACCTGG[C/G]CTGTGAGTCCTGCTCGCTGCACCCTCTAGCCTGACGTTGAAGTAACCTCGCCAGCGGGGCCCTCTGTGGCACTGGGCACTCA GGGAGAGCACGTGGCATTGGAGAAGGTATTCTGAGTCCTACAGAGAGGGACCTGCGGGGCCTGCCCCACAGGCACGTGGGGCTCCTGCTGGGGCT GTCAAGGACCCCCGGGCACTGAG |
| rs14 5433 3 | TACCGATTTGCAACAAAATTTACTTTTCATGTACTTATGATTCTAATCTATCTCAAAAGTAGATAATCAAACTTTTGTAAATAACCTTTCAATGATGGACAA TGTTATTGCTCCTTTACCTATTACTACCTCTCCCTGTTCTTTTCTTCATCCCAATCAATATGTCTAGATAAATACATTTTTTTCTCATTTGAAATTTA[A/G]T TTGGAAAATGCAGCCACATTTTTACTTATCTTTTTCAAATAATTAAAAACTTCTACCTCACTCATGTAACATCTTCCTGCATGATCTAATCTGCATCTAATT TTTCTCTGACTAGCAGCACTAGATGTTTTGTGATTAGGGAAGTCAGAAGTGATGAGACTTTAATAGAACATAAAATATTTCTTTTCTATATTTCAGT |
| rs95 8969 8 | ATATATATAACTTAGAAAATGTTTGAATGGTATGATTAGAATGGAGTCATAGTGTTTTGCAAAACATGGATTGTTGAAATACAAAAATCCAGGTTTTGAA GATTATTAAATAATTCATGTAAGATATCAGAGTGGAGCTCCAACAATTTGTTCTACTCTTATTGGATATAATGTCTGTGAGTGATTCCGTAAATGAGGT[ C/T]ATAAATATTCATCTATTTTATCACTGGCAATAGCTGAGAATCTCTGAACAGACAGTGGACCACAGAATTCAGACCCTCTATGCCTGTGCCTATCCAT TGACTTATCCAACTTTATTTCCATGTTAGATACCAGCAGTTCAGAAACATTTGTATTTCCCAGCTGCGGAAACAGAATTATGCTTCTTTCTATTAGGATTT GA |
| rs20 2594 9 | GACATCTCAATTTCCACCCTCTTCATAAAATAAACAAACACACAAACTTTGAAATACAATAAAGCTATTAGTTAACCACTGGCAAGTTTGCTACAAAANG GCTTATCAATGATGAATTTGCCTTTTGATTTATTTATCATTGTGAAATACTGTCAACCCTCATTTACAAATTAGAATTCCTAAACCAAACTGGTAAAACT[A/ G]AGATTCTTAAATTGAACAAAACTATTAAATGCTATACTGTAGCATTAAATATTTACAGCATTTTAACTTTTATATTTTTGTATGTAACACTGAAAATTTTT ATAATGGCTTTTGTTCTAATTTGCATATTGAATATACAAACGCTGAACAGGAGAGAAATTAAAAGAAGTGATGTCTTTTCCAAGTTAAAATCTCTCTGG |
| rs90 2923 | AATGAAATGCANATGTAGTAGCCAGAATTTGAGCAGTCGTTTTGATCATGAGGTGGAAGCAATGTGCAGAAGATGATGGGGANAAAAGGAATTTCAA CAGATTTTTGGGAGAGAAAAATCAGATGATACAACTGGTTTAGAAGGGGGAAGAAATTTACAATCCATGGTGCCTGTAGAGATTGAGGCTAATGGGAA GAGA[A/G]TGTTCTGCCTTGCAGAACTTGTGAAATGCTCAGGAATTTGAGGCAGTACATACAGTGGTCAGCTGGATTAATGAGGGCTGGCTGGAGACT GTGGAACAGTCAGACTCTAGAACACCTTCACCCTCCACACTCAGGCAAGTCACTCCTCCAGGTCCTCCAACCACTGCCCCTANAGATGAGATGAGTA TTATTCACTGGAG |
| rs19 8415 1 | GACAACATTCTGATCAGNTTTCTTTCTTTTTATACTCCTTTAATTTTTGCAGTTTTCTTTAGATTGTGCCACCTCCTGGTCTAGCTGTTTCTTTGTGAA TTTNGTAATAGGCTCTCTGTTTGAATTCTATGGTGTTATTCCAAACATTGCTGCCTTCTTTTTGAAGTATGTGGCTCACATTTCACACAGATCCTTTA[C/T ]GATGATTTCCTTGCATAGTGGGAAGTTTTTGACGTGAAGGTTTACTGGAGTCCCTTGGCTTTCAAGNTGGCTCAGAGCAGATGGTACTGCTAGAGAG AGCAACAACACAGCTTTGGGCTTGAAAATGTTAGTGTACACACGATGCTTTTTCCCCTCAGGACTGGTATGATTGATGGGACTTTGGAACTAAAAACTG AGAA |
| rs14 5984 1 | TTTCCTTTTATGGATTGTATTTCATGCATGGAGTCTAAGAACTCTTTGCCTAGCCCTAATTCTCAAATTTTCCTTGTGTTTTCCTAAAAGTTTTGTAATTTT ACATTTTACATTTATGTTTACAATCCATTTTTGGTTAATTTTTCTTTGAGGCATGAAGTTTTCTTGCCTATGTTCATCTTTTTGGATATGGATGTCCA[A/G]T TGCTCCAGGATTTGTCAAAAAGACTGCTATCCTCCCACCATTAAATTGTTTTTGTACCTGTGCACCATACGAAACAGCACATTCCTATGGGTTTATTTCT GGGTTCTCTATTATGCTTCATTGTTACATGTGTCTATCTCTCTGCCCATGCCATTCCAAATAAACCATAGAATNGGTTTGTATATTTAATTAATTAATT |
| rs49 1763 9 | TTATTTCACTTAGCCTAATGTTATGTTCTCAGGGTTCATTCATGTTTTTGCATGTGTCAGATTTTTCTTCCTTTCTAAGGGTGAATAATATTCCGTTGTAT GTATAAACCAAGTTTGCTTACCCATTTATCTGTTGATGGGCACTTGGATTACTTTCACCTTTTGACTTTTTGAGTAATGCTATTATGAATATGGTAAA[A/ C]AAATATCTCTTTGATGTGCTGCTTTCAATTCTTTTGGGTATAAATAACCCAAAAGAATAACCCACCACGCCTGGTTAATTTTTTGTATCTTTAGTAGAA ACTGAGTTTCACCATGTTGGCCAGGCTGGTCTCAAACTCCTGACCCTGTGATCCACCCACCTCAGCCTCCCAAAGTGCTGGAATTATAGGTGTGAGC CACG |
| rs93 3217 2 | ATTTTAGCAAAAAAAAATATGCTGTGTGACTCAGCTAGCTGCAAAGAGCCTGATGAATGGAATTTTTAGGNAAGCATGGAATAAGGGAGTAGGAAATAA AGTTTGGCAAGTTGGTCTACAGCCTCTGCTATACAAGCAGTATTTTTTTCTAGTACTGTACTTTCCAGTTTCTATGTTGGTAACTATATAACTATGTG A[A/G]TAATTTTGAATTCACTGTAATCAAATATGCTGGTAAATAATTTGTCAGATAATTGCATCAAATCATTCCTAGGAAAAGCACAACCAACCATCNGAA TTTACTATTGAAAGCTTGGAAAACACTGCAGTTGACTTGTTTGGAGCTGGGACAGAGACGACAAGCACAACCCTGAGATATGCTCTCCTTCTNCTGCT GAAGCA |
| rs94 8068 4 | CCCCAGTTTTACAGACAAAACAACAACAACAACAACAACAAAACCCCGGAAGNTCAAAGGGGTTGGCCAGTTTGGCCACAATCTCACAGCTGTAACTG CTGAACCAGAATCTGGCCATAGGCTGACTTCAAACATTTATTTATTATAATAGTGTTAGGCTAAAAATCACAAGCTGAAAGGCCGTGATTGCACTTAG TTC[A/G]GTAGAGTTGATGATTCTCATGTATACACGTGACCTTTCATTGAAAAGATCTAANATCTCAAAGGAATTAACACAGCCTACAGGATTTCAATGC AACATGTGTTCAATGAAGCTAATAGGGAGTGAACTGATGGTGCTTGGGGGAGAATAAATTTAGNGTGGTTGTCCTCAAACTTTATTTGCATCAGAATCA CCTGGAGG |
| rs23 4842 7 | CAATCAGCAGAGGACATAGTACTTGGAGTTATTTTCTAATAGTGACAGTGAGCTTGAATTTCCAATATGACTCTATCACCAGGTCCTCTGACCAAGGAC ATTTACTTGTAATATTATACTAACTAGGTAAATCACTAGAGCCTGTACCAGTTAGTTAGCTGGGTTCTTAGGATGGATTTTATATAAAGGATTTGAAGTG A[C/T]ATAATGAAAAAATACAGTTTTGCAAAAGATCCCAAAATGTCCTGTAGGTGGTAATTATTTCTTAAATGGTTCTTCTATGAAAACTAAGAGCATAAC ATTTTGTTTTAAATAAGTGATGCATTCCATCATTTGGGAGGGAAACAAACATAATATTGTTTTAATACATTACTGTCCAATCATCTATTTTATAAGAGATC AAC |
| rs27 7408 9 | CTTTCCCTGGGTGTGGGNACTGCCGGTGTGGGAACTGGCTGCTGGCGTGGGAACTGGCTGCTGGTGTGGGAACGGCTGCTTGTCCCAGTGGTGCC CACCTGTGTCCACACTTCAAGGCCACCTGGGCCTGGAGATGTCACTAAGGGTTGACCCGGAGACGCCGACCTCAGGGACCTGGGAGGCTGCACACT GGCCGTGTT[G/T]GAAGTGACCCTCATGTCACCCAGGTCAAGTGCCACGGGCCCCACACATCCTCTTCTTCCCTGTCTCCCAGCTGCAGGCCCGGAC TGGATGGCTTTGGCTAAGGAACCAACGCAGGTGGAGAAGGAGGCTGACGATGGAGCCCAGGCACTCCAGCCTTGAAGGAGGAGAACCTGACCCTG AGCCTGCATGGCAGCCGTGCG |
| rs49 0767 4 | GTGAACTAGTACACTTGTTTTACCAGAAGTCACACAGGATCCTTAAAGCATCCTCATTCTTTCTCNGTACAGGTTCTGGATGGAGGTCTCATGAGGGAT TCGGGCAGAGGCTGATGAAAACATAAAACAGGCCAGTCTCCAGATTCAAGGGAGAGCTGGAGACAGCATTCATAGCTTGACCAGGCAGCTTTGACCA GGTG[G/T]GACAAAGGAAGCCCAGATGTCCCAGTCTATTCATGTTTCTATCACAAAATGCCTTTGAATTAGTAATTTATAAACAATAGAAATTTATTTCTT ATCGTTCTGGAGGCCGGAAAGTCCACGATGAAGACACCAGCTGGTTCCCAGCCGGTGGGTGAGGACCCAGCCTCTGCTTCCAAGATGGCACCTCTG GGCTGCATCCT |
| rs98 7785 9 | CAACAAGAGATAGATATGCATGTTTGCTGCATGCCAGATATCTTTTATTTAATTCTACAGCTTTTTCCAGAATGGCTGCCAGGTAGAGCACCTGCATCA TTTTGGCAAAGTCCTCTTTGGAGTGGGGTCTTCAAGCCTGATCTTGGAATTGCGTTTTATTATTGTCTGCCTAGGACTCAGGTCTGCACTGTGGGACA CAA[C/T]GCTAAGTGGAAGGCCCCTTATCCATGGCACCATGGAAGCAGGCGCAGGATGCATGGGAAACTGCAATGGCATTAAAGGAGGAAGCTCCAA GTGGATTTGGAGCCATTCTATAACTCCTTGAAAACAAGACTACCTCGGGGATATTTAGGATGACTCGTGTGTCACCAAGTGAGAAATACCTAGTGTTG GAACATTTCCAG |
| rs12 0479 8 | CTAACTACAGATTTAAAATCATTAATTATTGTCATACAAAAGTTACCACATTATAAGACTACACTGATTGTCAAATAAAGTGTCTTCTTCACATTTGAAGC ACTCTCTGAAAGATGACCATGACTTGCTATAATGCTGCTTTCTGTGGGACCCATGTAACNCACAAAATTGTTAACTCTTCAAGCTAACAAAGCCAGCCA[ A/G]TTTCAGGCCCCCAAATTTGAAGATAGATTTTCCAAATATTTCCAGCTGTTAGAAACTATTGAGTTCACTACCAAAGCCAGCTTGGTACNTAGAGTCT NTGTGCTACTCTCTTTTTTTTCCTGATTATGTTTATACTTCAAAGAGTTGTCAGGAAGACTAGTACTCACCCACCTTCTATTTCCAGTGGAATGTTCACC TTA |
| rs77 1932 5 | GGGATTCTCAATCCAAGTGCTGACATTCTGGCTAAAAGAGCAGTAAGAGATGGATGATATAGGCACAGTCTTGGCATGTCTGAGCCCTGTGCCAGAA GGTTATCAGCCCTTTTGGGGGAGATAATATCTGCAGCTTTAATTTTCTCTCTGACTACCAGGCATAATGTAAAATTAATGTGCATCATGTCAAGGTTATG GCA[A/G]GGCATTGTTGTCTGGAAGGGAAAACTGTTTGCTCCCCCGCCCTCCAAACACTTGCACATACCGTGATAATACATTATTAAAAGCACATTTC TTTGTCATTGTCAATAGTGGTGAGCCAGTAAAGGATATTTTCCAGCACTCATTATGCAGGAGGAAGTTAAGAGACTTCTAAAACTCAGTCTTAAAACAA AGAACATAC |
| rs68 7364 0 | TCCCCTCTCCAAAACTATGAAAGCTCAGGTAACAACAGGCAACAGCAAAATCACCCGATGCTTGTTGGAGTCCTGACAACGTTGACAAAGTCCTTGGC AGGCCCTTCTATTTTCATGATCAATGGTACTANAGCCAGAAACAAGCCTCGCTGGCTGTGAGCTGATCTCTACTGTGAAAATGCACGCACGGTGTACA CATT[C/T]GTTTATATTCTAAGCACTGAGGATGAAATAATTCTGCACCGTGTCCATTTTTGTGCTGTTAAGGCATACGGTTGCTTTGCAAGCAAAACAGT AGATCTTTCATTTGCCAGTGACATTCCACAAAGTGCTTATAATTCAGTGTTAGCTTATCAGCCGTCCAATCTCCCACTCCAGGGAATGTGTCAACCATC AAAAGGAGC |
| rs29 8087 2 | GGGGGACACGCAAATGTGTCTCTAAGTCAGGGCACCCTCTGTGTGCACAGAGCCATCGAGGGCACTCGCCTGGCTGGACCCCAGGGAAGGAAATG GGATGGCTTAGTGGAGCAGCCCAGTGTGACTTCCCAGAGCTGACTTCCCAAAACATGCCACGTCCTCATCAAATAAAATCAAACCCAAACCCCTCTAG GCTGCTG[C/T]GCCNGACCTTGCTTACTCTAACCTCATCTCACCATCATGCTCCAGCCATGCCTTCTTCTCTATGTAATAGTGCTGGGCACTTTCTGCC ACAGGACCTTTGCACACGCAGAGTCCTCTTCCAGGGGCCTCTTCCTCACCACCCACCCCTCTGCTTCTCCCCACCAACTCCTACTTCCCCTTCAGGTT TCAGCTACAGTCCC |
| rs55 4710 | CACAGAGAGAGGACTTCTGTCCTGATAGACGTCGGTGAGGACTGAATCCCCACTTACAGGCGTGCACATCAGGGACTGATGGACATCGCTGAGGAC CGATCCCAGCTTACAGGCGTGCACACTGGAGGCTCNGAAGAGCTGACTGTGACTCACTTCCGGCTTCCCCCAGGACAAAACCTGCCTCTCCTTCCA GACTCGC[C/T]GACTTCCCTTCATGTCCCGCTGTGATGTGCAGTCCAGCATCCTTGGGTCATGACACCAGCTGCACTGGCACAGGGAATGAGAGAATA TTCCTGAAAATGAAGACTACCGCGGAAGGCAGGAGCTTCTAAGCTAGAGTGACTGGCAGCTTCAACAGGCCCTGCTGCACCTGAACCAGCCCGGGG CCCCCCAGTGCCNCGGA |
| rs72 0733 | CAACTTTATAGTCACAAAAATTATCTAGAATTGCATCTATGATGCCCATGTTCCAGGCAGCAGAAAAGAGGGCAATGAAAATGATAAATGTCCCCCATT TAAGGACCTTCCCAAGAGTCTCAGGTATCAACATTATGGACATTANATTACTAATTTTAGCTGCAAGGGAAGCAGGGAACTGGAGTACTTTAGCTGGG CTG[A/G]TCTGAATACAATTGTTTTTCTGCTATTCTGAATATTTATTAAAGTCTTAACTCCTTACTAGGTCTATTTCTCTTTTAACAATTGGGTCCACAATT GATTCTTCAGGATGTTTATTGTGTTTGAGGAATAGCTAAAGAGTCATCATGGTGGTTGAAGAGAAGCAGTCAGTCCATATGCTCATGCTTTTACTAAAC TTGAGG |
| rs43 8830 1 | AAGACATGCCTGCTTCCCCTTCACCTCCACCATGATTGTAAATTTCCTGAGGCCTCCCTAGCCATGCTTCCTGTAGAGGCTGTGGAATTGTGAGCCAA TTAAACCTTTTTTCTTTATANATTACCCAGTCTCAGGTATTTCTTTATAGCAGTACAAGAACAGGCTAATATATCCTCTATTTATCTTCTCTCATTTCAAAC [C/T]AAACTTGTTCTCCTTCATCAAAGAAAGAGAGAACCCTGAAATTCAAAGAGACACAAAAACATTAGTTTTTGCTGTTGTGCTTTGATAGGCCCAGCT ATATTGCTAACGGTAGTTCTTGTAAGCAACTACTGTTACTTGAAGGGTCATTTTGACTATCTACTATTTTTGCCTTTTTAGTGTATTTAGAACAGGCCATT CTT |
| rs11 0908 9 | TTCTGTTCATTAGACGCACGTCACTAGGTCCATGCAGGGAGATGACACACAGTGTGGCCACTAGGAGAGCAGGAGATCTGGGAGCCGGCTCCAAGC TGCCTGCCACACAGTCCTAGTTCTTTCAGCTGTTTCTCACGTGACAGGGTACTGTATTTTTGCCCAAGTGGCGACACTTTTAAAGATAGGCAAATACAG CATGC[A/G]GAGGAAACCGTGGTTCTGTAATGCAACCGCAGGTCCTTAGGACACCCATATCCCATTTGTTGACCCACAGCGAGCTTGTAGGGGGGTTA AATTTCATTTTTGTTTTTATCAAATGACAGCACACATCGTTTAAAATAGCACAGTGTGTGTAATTCATTCAACAAGCTAACTTATTCTGAAGCTTGTGATGCA ATCATGAACA |
| rs20 7499 7 | TTTCCTCAGACATACTCCATAGATATTAATGGCTATATTCTTGTGTATTCTGTTACATCAATCAAAAGGTAAGACTCCTGCTGCCTGCTTGAGTTGATTT GCTACTCATAGATAACATTGATCCTTTTAAGGGACTGTGCTGGAATTATTAATAGTGACAATTAGAATTATTAGGCTACATGTTTTTAGTGATTTTCTC[ G/T]TGGAGCAACTGGATTGCTGGGNAACATGGCCCACTAGATGTTCTGTTAGTTACACCAATAGGATAAAAGGCTCTTTCTTACCTCAAGCTACCTAGA AAAACAGTGGTTCAGCATGATCTAAGCTTTTCTGCTAATTGAAATGGGAAATGCCTAAATTAATTTTACTTTGTGTGTGTTTTTACCATATTAAGGTGAAA TAG |
| rs96 5118 | TCCAAGAGCTGCTTTATAAATTGGATGCATACGGGGGCTGGTTCTAGGATTAAGACTGGTAGTAGTAGGGTAAAATGATTAAGGGGCAAATTCTTTTAA AAGTAATCTATAGTAAATTTGTTCACTGGCTTCCACATTTCAGGAGTGAAATTGCACGCAGTGAATTGCTGCATAGCGGTATTTGTTAGCAAGTTCTATT T[A/T]CTACTTAGTAGTTTATGTCGGTACATCCATACCACTATGGTTTAATAGTGGTTTAATTGCCCACCTAGGAATGATGATGAACATGGTTAAATACAG ATTATTTTTAGATTGCATGAAATATTTTGGTTTTGCCCAATTTTAGGGTGAGAACATATACCCTAAATGAATTTTGGGGTAAGAATGTGAAGTATCAAAAA AAA |
| rs37 5315 1 | TAATTCTACTGTCTCTCATTTTAACCTCATGTCAAAGTGGAAAGCTGGCTTTCTTCTGACATCACTCTCCTTGAGCCCCTTTCTCCCTGCNTCTTTGAAT CACCAGAGAATCCATTTCCAACCTAAACCCCTGAAATTCTCCTAAGTAGACTAATAGCTATTTACTAGTAAATGCTACTAACCACGGTCATAGAGGTTTC [C/T]AAAATTTGGTGTTCACAAAGCAAGATTTGTGTTACTAAAGTGTGGTGAGGGCTCTTCATTCATANCCTACTGAGAAGTCCACACCCAGGACGCAG TGCCTTGAGAATGCTGCAACACCTAAGTGCAACCTCCCAACTTTCTGGAAGGCGCTTTGTCATTCACTAACAGTGCTACCTTGAGATGGCAGAAGTCG GAAATTG |
| rs22 9996 5 | TACTCTTCCTCCAAACCAAAACCCACCTTAACTTTTAAAAATAAAAATTATTTTAAAAAATTCTTCTGAGCTTTTATCAGGGAACAGCATTGTATTATTTTT TCCAATGATAAAATGGCACAGTGAGTTTCTCAGCTGGTATTTTCACTACAGAAGGTGTACTTTCAGGGGTGTTGACTCAATCTTATACCTCATGATTA[C/ T]TGCAAAATAGGCACTGTTTACAGAATTATTTTAAGTTTTCAGATTAATATTGACTTTGTTATCATGCTTATTCTCCTAAAAAATAACTTATTAGCCCGGT GTGGTGGCTCATGCCTGTAATCCCAGCACTCTGGGAGGCTGAGGCCGGTGGATTGCTTGACCCCAGGAGTTTGAGACCAGCCTGGACAACGTGATA AAAC |
| rs22 9996 7 | TAAAGTGCTACCCAGAAAGGCCATAAAACTGTTTCCCTGGAGATTTTCAAAACAGGATATTCATCTCTACAGGATGGTTTAAAGACAAAGCTGAGGTGG AAGGCAACAATAATAGCCCTGATAAACTGCAGGAAATTAAATGTTTAGCAACAATGAAGAATGAGTTTTCCTAGAACGGTGGCCAATCTGGAGAGAAG ACC[C/T]GGGATACAGGTGAGGGTGGGAAAGAGCTAGAGAAAAGCAAAGACAAAAATTCTCTATTTCTTAACATATTAAAGAACATGAAATTTAAGAATG ATCAAAACTAGTAATAACACAGTATGAGGAAAATGAGATATGAAATAGAGTATACTCATCCGTCAGCCTGAATGGTTAAAACCATTCATATTTTATAAAG ANAATTT |
| rs69 4819 6 | TGACAACCAGCTTTTAATTATGGTCTAATGGTAAACTAGGTTACCAAAGCTCTCTGTACAGGTAAAATCCCAGTGAAAGATGTACCAGGCTGAAGACAG TTTACGTAACACTACCATTTTAAGTTGAGTTACTTCCTATCGCGGGACTACTCCGTTGTGGTAGAAATAACGGAATGTCAGGGAGCTGGTACTTTTGGC CA[C/T]CACGGCAACAGTTAATACAAAGCACTCCCTGAAAGCCAGACTCCCAGAGTTATAGGTGACGTGTCCTTAAAAGCTTTNACATTGTAAATACCA GGAAAAGTACAGGGGACTGGCAGGGAGAATTGTATAGACTACCTGAGAATTATATTTTACCTATTGGTAGTTCTGACAACACTCAAAAAAAAAGAAAAA AAGAAAAC |
| rs12 6175 66 | ACAACTAAATGTCAGTAAGCCTGGAAAACAACAGAGCTCTAGGGAGTTCAGCTGCAGGAGTTCATGGACTTTTACTCTAATGATCTTCTATTACCTCAG CTAGCAACTTATTAAGTTTCTGCATATCTTAGTTCAAATTCTCAAAAGGGAATCTGATTAATTCAGATTAGTTTATGGATTAGTTCCTCTGGGGTTGGATA [A/G]CTTCTCTTGGCTCAATCAGCCATGTCAGGGGAATGACATTGCTAATGAAGGATAAACAACAGGAAACAAGATATTTCTCTAGTAGAGATATTTATG ATGCTGGTCCATATTTATTTACATGTAAATGTATAATATAAAACAAACTACATGGATATTAATAAAATGTAATTACCTTATTCTAAGACAGTATATTTATAA GC |
| rs14 2178 0 | CTATATTCTTCCCCTATTCTACCCATCTACCTTCCTTTTCTTACCCCAGCTACTCCAAAGAAAGAAAAATGGCCAGTTTGGTCAGATATCAAGACCTTTA CAGGAANATGGTTNGCCAATGGAGGGATCAGGGCCACCAAGTGTCAGAAAAGCTATTCTGCTTTCTACCTCCCTCTGTCAAAGTCACCTGTGCAAGA CTT[A/G]CCTCTTGGGGCTAGGACAGCTGGCATCTCTCCAGTGGGGCACAGGTATATTAGGGGCTCAGTACCCATTCAGTCCCAGCCAGCCCTCTTCC CTCTGACCCGGCCTAAAGCCCACTTCCCTCCTACTTGCAAGAGTGGCTTATTAAGGGTCCCAAGATTTGAAAACAGGAAGAATGCAGGAGCCCTTTAT ACCTCAGTCTT |
| rs30 1191 | TCTGTGATTGAGGGCTGCAAAACAAAGTATGTTTAAAAATATGTTTATGTTATTTTGATAATTTTGAAAATGTACTCTCACCTGAGGATGATCTACAAAG CCAGTTCAGCTGGCTCTTCAGCACTGTATTAAAAATACTCTTAGTTCTGGGGAAAAAAAAAAAAACACTTTCANAAAACAGTTTTTGAAGAGTAACTGTG [A/G]TAGGTTCCGAAGTTGGTNTCTTTCCTGANAGGACTCTAAGTGCCCTANATTTTATATGTAAAATCATGTAGTTTGCAAGACATTGGAAAGGTGGCA CTTCAATTAAACCCTTCACTNCCACCCCCAAAAGTTAAAAAATAAGTAATATCTAGAGAAAAACTGGTTATGGATCCACTGTGACTGGATAGTATTTGAT CACC |
| rs30 1193 | CTAATTCATGGAGTCTGGGTCGGACNTGGCATTCTCTATTTTTAGCAAGCTTCTCAAGGATACCATGTTGATATTCTTCCCATCTTTGAATAGCAACCC ATTGCTTTAAACATATTTAACATCTCTGGGCTACTATTTTCTATTCAATTGTTGGTCTACTCCACTAGGATATATGTGAGGTCCTTTAATTCAGAATAATA[ C/T]CTTATTCAGCTGTGCAATAAGAAATGGCTGAAAAAATGATCATTCCTTCTCCTTTTTCATTATTACTTTTCTTTGACCAAAGAATGCTATTGCTCTAA CAAGAAATGTACCAATTCTGATGAAGTCTGTAGCTTGGTAAAAATCACAGGGGAGGAATAAGACCACTANCTAACCAATGTATTTGCTAACATACCATA GAA |
| rs13 4035 84 | TTTTCACTAGATTCAATAAAAGCTCACGCAAGTGTAAGTATAAGAGTGTTGGGCTTAATTATGGTTTGGCCTACTATTTCCTTTTACTTCATGTTATATCT GAAAGGAATTTACTCATCATGGAGAAAATAAGCTACATTAACACTAAAACACAGTGAGAAACAAATATTGCTCCAATGCTAGAAAAAAAGAAAATTTCC[ A/T]GGGCTCAAAAGAGNTGAGTTTCAAGGACAGTTGACACCTATACCCTACTTACTAAGATAGAGTGAATCAGATATAGCTATAATTTGGCCTCTGTAG GTGTATTTCAGCTGTTTCATTTCTTTTCAGGGAAGAAAGATGGTGTATGAAACTTTTTCCAAGATGGTCTCTGAAATGAGTTAAACTTCGGTGTTCAAAT TGTG |
| rs76 7610 6 | CAGAATTTCTANTGCTATACTTTCTTGAGGTAATTTTAAGAAAGGTTTCCTTANCATTTACCCAAATTTCATACACAGACACACACCAAAACAAAAACAAA ACAAAAACTGAATGAATATTTTTGTGCTGATACAAACCGTCACAAAGGAAGTAATGATGGGATTTGTCTACATTAACCTGACATACTGGTGAAAATAAC[ C/T]CCATTGTTCATTATTTGAATTAGGCAATAATTTGGAGAAATTCTTGAAGAGGNCATAAAGGAGACCGCAGATACGTGTGGGCTGCCTGTGAAAGG CATGCTGCTTTGTAAATATTCACACTAAACTTTCAAACACAAGAGTGATACTACCCTAGCAAAACCTAAGGTTCTCCNGGGAGGCAGGTGACTAGTTCC ACTTTT |
| rs76 8531 4 | NTTACTCTAGAAAGAAGATAAGAATTGTTTCATATATTTGAAGAATTTTTACAAGGTAGATATCCCAAACCAGAAATGTTATATAAACTAATAGCTGGGT AACTTCACACAAAGTTGAAATAACATGGATATAGCACAGTTTAAAGGGTGATCTTTCACATTAGTCAAGAATTTTTATCAGACAGATTCATGAATTTAT[A/ G]GCTTTTATAATAAAAATTATTTATTAAATGGGGTTGTTTATAGTTTGGACTAATTGAGTTCATAGATCAAGGGAAAAAGCAGAAAAAGAGGTTTACACT GTGTTCTTAAAATCTTGAGTTTTTGTGTTCCTAAAGGACACATTTTAGTGAAAAAAATGTTTAAAAATTTATCTGGAAATTAGAATGTTTAAGGCCTAAAT |
| rs12 1436 47 | CTCGAAGCAGGGCATGAGGCCCTTTTTGTTCTTGCCTGGTAGCTCTCCACTGTAGAAACTTTCCCTCCATAGCCCTGGATGCTCCAGCCACACAGTG CTAGCTGCCTTTCCCCAGCTCCCTTGTACTCACGTGCTTCTGCGTTTTTCCTCTGCCTGCCCTCCCCTTCCTGTGCCCTTTGTTTTCCTGTCAAACTGC CTCT[C/T]CACATGAAGCTCCAGCTCAGATACCACCTCCTTCAAGAGACCATCCTTGACCGGGCGTAGTGGCTCACACCTGTGATCCCAGCACTTTGG GAGGCCAAGGCAGGTGAATCGCTTGAGGCCAGGAGTTCAAGACCAGCCTGGGCAACATGACAAAACCCCATCTTAAAAAAAAAAAAAGNCCATTTTT TCTCCTAATGTTC |
| rs75 5027 7 | AACTTGTCATTTACAAACACCTGTAAATNCGAACTCTACTTTTTAATATGGCATTACAACTTACAAGATTTGTATGTATGTATGCAATTAACCATGATACA ATGGCAGCATAAAATGGCACCGGTAAAGTGTCAGGCACACAGCCTATTGCAGAGTGAACACTCACATTTTGTTTCCACGTGCCCCACCTCTTTTCTTG A[C/T]TTAAAGGATTTCTACTGGGCAAATGATAAGAGAAACAAGACACTTTTCTGTCCTTAAGGAACTTAGACTGGTAAAGAGGATAAAATAAACACAGA CAAAGCCAGGCAGGATATACATTTTGAGAGCAGAATGAATCCAGGTGATATAAGAACACAAAGGAGGAAGACCATGTGCCAAGTTCCCTGAGGACAT CAGGATCA |

### Further references

1. Kessler RC, Berglund P, Demler O, Jin R, Merikangas KR, Walters EE. Lifetime prevalence and age-of-onset distributions of DSM-IV disorders in the National Comorbidity Survey Replication. Arch Gen Psychiatry. Jun 2005;62(6):593-602.
2. Nierenberg AA, Gray SM, Grandin LD. Mood disorders and suicide. J Clin Psychiatry. 2001;62 Suppl 25:27-30.
3. Teicher MH, Glod C, Cole JO. Emergence of intense suicidal preoccupation during fluoxetine treatment. Am J Psychiatry. Feb 1990;147(2):207-210.
4. Masand P, Gupta S, Dewan M. Suicidal ideation related to fluoxetine treatment. N Engl J Med. Feb 7 1991;324(6):420.
5. Wirshing WC, Van Putten T, Rosenberg J, Marder S, Ames D, Hicks-Gray T. Fluoxetine, akathisia, and suicidality: is there a causal connection? Arch Gen Psychiatry. Jul 1992;49(7):580-581.
6. Licinio J, Wong ML. Depression, antidepressants and suicidality: a critical appraisal. Nat Rev Drug Discov. Feb 2005;4(2):165-171.
7. Hall WD, Mant A, Mitchell PB, Rendle VA, Hickie IB, McManus P. Association between antidepressant prescribing and suicide in Australia, 1991-2000: trend analysis. Bmj. May 10 2003;326(7397):1008.
8. Morgan OW, Griffiths C, Majeed A. Association between mortality from suicide in England and antidepressant prescribing: an ecological study. BMC Public Health. Dec 21 2004;4:63.
9. Nakagawa A, Grunebaum MF, Ellis SP, et al. Association of suicide and antidepressant prescription rates in Japan, 1999-2003. J Clin Psychiatry. Jun 2007;68(6):908-916.
10. Angst J, Angst F, Gerber-Werder R, Gamma A. Suicide in 406 mood-disorder patients with and without long-term medication: a 40 to 44 years' follow-up. Arch Suicide Res. 2005;9(3):279-300.
11. Perlis RH, Beasley CM, Jr., Wines JD, Jr., et al. Treatment-associated suicidal ideation and adverse effects in an open, multicenter trial of fluoxetine for major depressive episodes. Psychother Psychosom. 2007;76(1):40-46.
12. Jick H, Kaye JA, Jick SS. Antidepressants and the risk of suicidal behaviors. Jama. Jul 21 2004;292(3):338-343.
13. Hammad TA, Laughren T, Racoosin J. Suicidality in Pediatric Patients Treated With Antidepressant Drugs 10.1001/archpsyc.63.3.332. Arch Gen Psychiatry. March 1, 2006 2006;63(3):332-339.
14. Vitiello B, Swedo S. Antidepressant Medications in Children 10.1056/NEJMp038248. N Engl J Med. April 8, 2004 2004;350(15):1489-1491.
15. Marshall E. ANTIDEPRESSANTS AND CHILDREN: Buried Data Can Be Hazardous to a Company's Health 10.1126/science.304.5677.1576. Science. June 11, 2004 2004;304(5677):1576-1577.
16. Libby AM, Brent DA, Morrato EH, Orton HD, Allen R, Valuck RJ. Decline in Treatment of Pediatric Depression After FDA Advisory on Risk of Suicidality With SSRIs 10.1176/appi.ajp.164.6.884. Am J Psychiatry. June 1, 2007 2007;164(6):884-891.
17. Valuck RJ, Libby AM, Orton HD, Morrato EH, Allen R, Baldessarini RJ. Spillover Effects on Treatment of Adult Depression in Primary Care After FDA Advisory on Risk of Pediatric Suicidality With SSRIs. Am J Psychiatry. August 1, 2007 2007;164(8):1198-1205.
18. Gibbons RD, Brown CH, Hur K, et al. Early evidence on the effects of regulators' suicidality warnings on SSRI prescriptions and suicide in children and adolescents. Am J Psychiatry. Sep 2007;164(9):1356-1363.
19. Brent DA, Mann JJ. Family genetic studies, suicide, and suicidal behavior. Am J Med Genet C Semin Med Genet. Feb 15 2005;133(1):13-24.
20. Statham DJ, Heath AC, Madden PA, et al. Suicidal behaviour: an epidemiological and genetic study. Psychol Med. Jul 1998;28(4):839-855.
21. Sherry ST, Ward MH, Kholodov M, et al. dbSNP: the NCBI database of genetic variation. Nucleic Acids Res. Jan 1 2001;29(1):308-311.
22. Sambrook J, Fritsch EF, Maniatis T. Molecular Cloning: A Laboratory Manual. New
23. Kim S, Misra A. SNP genotyping: technologies and biomedical applications. Annu Rev Biomed Eng. 2007;9:289-320.
24. Wang L, Luhm R, Lei M. SNP and mutation analysis. Adv Exp Med Biol. 2007;593:105-116.
25. Hill WG, Robertson A. Linkage disequilibrium in finite populations. Theoretical and Applied Genetics. 1968;38:226-231.
26. Hamilton M. A rating scale for depression. J Neurol Neurosurg Psychiatry. Feb 1960;23:56-62.
27. Hamilton M. Development of a rating scale for primary depressive illness. Br J Soc Clin Psychol. Dec 1967;6(4):278-296.
28. Trivedi MH, Rush AJ, Ibrahim HM, et al. The Inventory of Depressive Symptomatology, Clinician Rating (IDS-C) and Self-Report (IDS-SR), and the Quick Inventory of Depressive Symptomatology, Clinician Rating (QIDS-C) and Self-Report (QIDS-SR) in public sector patients with mood disorders: a psychometric evaluation. Psychol Med. Jan 2004;34(1):73-82.
29. Rush AJ, Trivedi MH, Ibrahim HM, et al. The 16-Item Quick Inventory of Depressive Symptomatology (QIDS), clinician rating (QIDS-C), and self-report (QIDS-SR): a psychometric evaluation in patients with chronic major depression. Biol Psychiatry. Sep 1 2003;54(5):573-583.
30. Rush AJ, Trivedi MH, Carmody TJ, et al. Self-reported depressive symptom measures: sensitivity to detecting change in a randomized, controlled trial of chronically depressed, nonpsychotic outpatients. Neuropsychopharmacology. Feb 2005;30(2):405-416.
31. Rush AJ, Bernstein IH, Trivedi MH, et al. An evaluation of the quick inventory of depressive symptomatology and the hamilton rating scale for depression: a sequenced treatment alternatives to relieve depression trial report. Biol Psychiatry. Mar 15 2006;59(6):493-501.
32. Laje G, Paddock S, Manji H, et al. Genetic Markers of Suicidal Ideation Emerging During Citalopram Treatment of Major Depression. Am J Psychiatry. October 1, 2007 2007;164(10):1530-1538.
33. Wigginton JE, Cutler DJ, Abecasis GR. A note on exact tests of Hardy-Weinberg equilibrium. Am J Hum Genet. May 2005;76(5):887-893.

## Claims

1. A method of diagnosing a predisposition for or the occurrence of treatment emergent suicidal ideation in an individual, the method comprising determining the presence or absence of the SNPs defined by SEQ ID NOs: 8, 18, 19, 47, 50, 52, 56, 64, 73, 86, 87, 92, 94, 95, 97 and 99, wherein the polymorphic position defining the respective SNP is position 201 of the respective SEQ ID NO.

2. The method of claim 1, wherein, in addition to the SNPs defined by SEQ ID NOs: 8, 18, 19, 47, 50, 52, 56, 64, 73, 86, 87, 92, 94, 95, 97 and 99, one, more or all SNPs selected from the SNPs defined by SEQ ID NOs: 1 to 7, 9 to 17, 20 to 46, 48, 49, 51, 53 to 55, 57 to 63, 65 to 72, 74 to 85, 88 to 91, 93, 96, 98 and 100 are used.

3. The method of claim 1 or 2, wherein instead of or in addition to a SNP as defined in claim 1 or 2, a corresponding tagging SNP is used, wherein said tagging SNP is provided in Table 4.

4. The method of any one of the preceding claims, wherein said determining is effected by allele specific hybridization, allele specific oligonucleotide ligation, primer extension, minisequencing, mass spectroscopy, heteroduplex analysis, single strand conformational polymorphism, denaturing gradient gel electrophoresis, oligonucleotide microarray analysis, temperature gradient gel electrophoresis or combinations thereof.

5. The method of any one of the preceding claims, wherein said treatment emergent suicidal ideation occurs or may occur in a patient being administered selective serotonin re-uptake inhibitors, selective noradrenalin re-uptake inhibitors, dual serotonin and noradrenalin re-uptake inhibitors and/or tricyclic antidepressants, and optionally being administered neuroleptics, mood stabilizers and/or benzodiazepines.

6. The method of any one of the preceding claims, wherein said individual is a child or adolescent.

7. The method of any one of the preceding claims, wherein said determining comprises isolating a nucleic acid from said sample.

## Patentansprüche

1. Verfahren zum Diagnostizieren einer Veranlagung für das Auftreten behandlungsinduzierter Selbstmordgedanken in einem Individuum, wobei das Verfahren das Feststellen des Vorliegens oder Fehlens der SNPs umfasst, die durch die SEQ ID Nrn: 8, 18, 19, 47, 50, 52, 56, 64, 73, 86, 87, 92, 94, 95, 97 und 99 definiert sind, wobei die den jeweiligen SNP definierende polymorphe Position die Position 201 der jeweiligen SEQ ID Nr. ist.

2. Verfahren nach Anspruch 1, wobei, zusätzlich zu den durch die SEQ ID Nrn: 8, 18, 19, 47, 50, 52, 56, 64, 73, 86, 87, 92, 94, 95, 97 und 99 definierten SNPs, einer, mehrere oder alle SNPs ausgewählt aus den SNPs, die durch die SEQ ID Nrn: 1 bis 7, 9 bis 17, 20 bis 46, 48, 49, 51, 53 bis 55, 57 bis 63, 65 bis 72, 74 bis 85, 88 bis 91, 93, 96, 98 und 100 definiert sind, verwendet werden.

3. Verfahren nach Anspruch 1 oder 2, wobei anstelle von oder zusätzlich zu einem in Anspruch 1 oder 2 definierten SNP ein entsprechender stellvertretender ("tagging") SNP verwendet wird, wobei der stellvertretende ("tagging") SNP in Tabelle 4 dargestellt ist.

4. Verfahren nach einem der vorangegangenen Ansprüche, wobei das Feststellen mittels Allel-spezifischer Hybridisierung, Allel-spezifischer Oligonukleotid-Ligation, Primer-Verlängerung, Minisequenzierung, Massenspektroskopie, Heteroduplexanalyse, Einzelstrang-Konformations-Polymorphismus, denaturierender Gradienten-Gelelektro-phorese, Oligonukleotid-Microarray-Analyse, Temperaturgradienten-Gelelektro-phorese oder Kombinationen davon durchgeführt wird.

5. Verfahren nach einem der vorangegangenen Ansprüche, wobei die behandlungsinduzierten Selbstmordgedanken bei einem Patienten auftreten oder auftreten können, dem selektive Serotonin-Wiederaufnahmehemmer, selektive Noradrenalin-Wiederaufnahmehemmer, duale Serotonin- und Noradrenalin-Wiederaufnahmehemmer und/oder trizyklische Antidepressiva und gegebenenfalls Neuroleptika, Stimmungsstabilisierer und/oder Benzodiazepine verabreicht werden.

6. Verfahren nach einem der vorangegangenen Ansprüche, wobei das Individuum ein Kind oder Jugendlicher ist.

7. Verfahren nach einem der vorangegangenen Ansprüche, wobei das Feststellen das Isolieren einer Nukleinsäure aus der Probe umfasst.

## Revendications

1. Méthode de diagnostic d'une prédisposition à ou de la survenue d'idéation suicidaire consécutive à un traitement chez un individu, la méthode comprenant la détermination de la présence ou de l'absence des SNP définis par les SEQ ID NO : 8, 18, 19, 47, 50, 52, 56, 64, 73, 86, 87, 92, 94, 95, 97 et 99, où la position polymorphique définissant le SNP respectif est la position 201 de la SEQ ID NO respective.

2. Méthode selon la revendication 1, dans laquelle, en plus des SNP définis par les SEQ ID NO : 8, 18, 19, 47, 50, 52, 56, 64, 73, 86, 87, 92, 94, 95, 97 et 99, un, plusieurs ou la totalité des SNP choisis parmi les SNP définis par les SEQ ID NO : 1 à 7, 9 à 17, 20 à 46, 48, 49, 51, 53 à 55, 57 à 63, 65 à 72, 74 à 85, 88 à 91, 93, 96, 98 et 100 sont utilisés.

3. Méthode selon la revendication 1 ou 2, dans laquelle à la place ou en plus d'un SNP tel que défini dans la revendication 1 ou 2, un SNP de marquage correspondant est utilisé, où ledit SNP de marquage est fourni dans le tableau 4.

4. Méthode selon l'une quelconque des revendications précédentes, dans laquelle ladite détermination est effectuée par une hybridation spécifique de l'allèle, une ligature d'oligonucléotide spécifique de l'allèle, une extension d'amorce, un miniséquençage, une spectrométrie de masse, une analyse d'hétéroduplex, un polymorphisme conformationnel simple brin, une électrophorèse sur gel en gradient dénaturant, une analyse de micropuces d'oligonucléotides, une électrophorèse sur gel en gradient de température ou leurs combinaisons.

5. Méthode selon l'une quelconque des revendications précédentes, dans laquelle ladite idéation suicidaire consécutive à un traitement survient ou peut survenir chez un patient auquel il a été administré des inhibiteurs sélectifs de la recapture de sérotonine, des inhibiteurs sélectifs de la recapture de noradrénaline, des inhibiteurs doubles de la recapture de sérotonine et de noradrénaline et/ou des antidépresseurs tricycliques, et auquel il a été éventuellement administré des neuroleptiques, des stabilisateurs de l'humeur et/ou des benzodiazépines.

6. Méthode selon l'une quelconque des revendications précédentes, dans laquelle ledit individu est un enfant ou un adolescent.

7. Méthode selon l'une quelconque des revendications précédentes, dans laquelle ladite détermination comprend l'isolement d'un acide nucléique à partir dudit échantillon.
